# EUROPEAN PATENT SPECIFICATION

(11) **EP 2 843 048 B1**
(45) Date of publication and mention of the grant of the patent: **15.11.2017**
(21) Application number: 13760999.6
(22) Date of filing: 14.03.2013
(51) Int. Cl.: C12N 15/09, A61K 38/00, A61K 45/00, A61P 19/00, A61P 29/00, A61P 31/04, A61P 35/00, A61P 35/02, A61P 37/02, A61P 43/00, C07K 7/06, C07K 16/28, C12N 5/071, C12N 5/10, C12P 21/02, G01N 33/15, G01N 33/50

(54) **NOVEL INTEGRIN alpha9beta1 LIGAND AND USES THEREOF**
NEUARTIGER INTEGRIN-ALPHA9BETA1-LIGAND UND VERWENDUNGEN DAVON
NOUVEAU LIGAND DE L'INTÉGRINE alpha9beta1 ET SES UTILISATIONS

(30) Priority: 15.03.2012 JP 2012058795
(43) Date of publication of application: 04.03.2015
(73) Proprietor: Osaka University, Suita-shi, Osaka 565-0871 (JP)
(72) Inventor: SEKIGUCHI, Kiyotoshi, Suita-shi Osaka 565-0871 (JP); SATO, Ryoko, Suita-shi Osaka 565-0871 (JP); EZOE, Sachiko, Suita-shi Osaka 565-0871 (JP)
(74) Representative: Kalhammer, Georg
(86) International application number: PCT/JP2013/057204
(87) International publication number: WO 2013/137396

(56) References cited:
- WO-A1-2005/080569
- R.-I. MANABE ET AL: "Transcriptome-based systematic identification of extracellular matrix proteins", PROCEEDINGS OF THE NATIONAL ACADEMY OF SCIENCES, vol. 105, no. 35, 2 September 2008 (2008-09-02), pages 12849-12854, XP055261274, US ISSN: 0027-8424, DOI: 10.1073/pnas.0803640105
- YOKOSAKI Y ET AL: "The integrin alpha9beta1 mediates cell attachment to a non-RGD site in the third fibronectin type III repeat of tenascin", JOURNAL OF BIOLOGICAL CHEMISTRY, AMERICAN SOCIETY FOR BIOCHEMISTRY AND MOLECULAR BIOLOGY, US, vol. 269, no. 43, 28 October 1994 (1994-10-28), pages 26691-26696, XP002146348, ISSN: 0021-9258
- SMITH LAURA L ET AL: "Osteopontin N-terminal domain contains a cryptic adhesive sequence recognized by alpha-9-beta-1 integrin", JOURNAL OF BIOLOGICAL CHEMISTRY, AMERICAN SOCIETY FOR BIOCHEMISTRY AND MOLECULAR BIOLOGY, US, vol. 271, no. 45, 1 January 1996 (1996-01-01), pages 28485-28491, XP002201290, ISSN: 0021-9258, DOI: 10.1074/JBC.271.45.28485
- R. SATO-NISHIUCHI ET AL: "Polydom/SVEP1 Is a Ligand for Integrin 9 1", JOURNAL OF BIOLOGICAL CHEMISTRY, vol. 287, no. 30, 31 May 2012 (2012-05-31) , pages 25615-25630, XP55261550, US ISSN: 0021-9258, DOI: 10.1074/jbc.M112.355016
- D GILGE'S ET AL.: 'Polydom: a secreted protein with pentraxin, complement control protein, epidermal growth factor and von Willebrand factor A domains' BIOCHEM. J. vol. 352, 2000, pages 49 - 59, XP002201830
- 'Gabi Shefer and Dafna Benayahu, SVEP1 is a Novel Marker of Activated Pre-determined Skeletal Muscle Satellite Cells' STEM CELL REVIEWS AND REPORTS vol. 6, no. 1, 2010, pages 42 - 49, XP055164386
- I. SHUR ET AL.: 'SVEP1 expression is regulated in estrogen-dependent manner' JOURNAL OF CELLULAR PHYSIOLOGY vol. 210, no. 3, 2007, pages 732 - 739, XP055164402
- RYAN S. SCHWARZ ET AL.: 'Evolution of polydom- like molecules: Identification and characterization of cnidarian polydom (Cnpolydom) in the basal metazoan Hydractinia' DEVELOPMENTAL & COMPARATIVE IMMUNOLOGY vol. 32, no. 10, 2008, pages 1192 - 1210, XP022732279
- I. SHUR ET AL.: 'Molecular and cellular characterization of SEL-OB/SVEP1 in osteogenic cells in vivo and in vitro' JOURNAL OF CELLULAR PHYSIOLOGY vol. 206, no. 2, 2006, pages 420 - 427, XP055164422

### SEQUENCE LISTING

<110> Osaka University
<120> Novel ligand for integrin alfa9 beta1 and utilization thereof
<130> 011F4525
<150> JP 2012-058795
   <151> 2012-03-15
<160> 5
<170> PatentIn version 3.1
<210> 1
   <211> 9
   <212> PRT
   <213> Artificial
<220>
   <223> Integrin alfa9 beta1 recognition sequence
<400> 1
   Glu Asp Asp Met Met Glu Val Pro Tyr
   1               5
<210> 2
   <211> 3567
   <212> PRT
   <213> Mus musculus
<400> 2
   Met Trp Ser Arg Leu Ala Phe Cys Cys Trp Ala Leu Ala Leu Val Ser
   1               5                   10                  15
   Gly Trp Thr Asn Phe Gln Pro Val Ala Pro Ser Leu Asn Phe Ser Phe
               20                  25                  30
   Arg Leu Phe Pro Glu Ala Ser Pro Gly Ala Leu Gly Arg Leu Ala Val
           35                  40                  45
   Pro Pro Ala Ser Ser Glu Glu Glu Ala Ala Gly Ser Lys Val Glu Arg
       50                  55                  60
   Leu Gly Arg Ala Phe Arg Ser Arg Val Arg Arg Leu Arg Glu Leu Ser
   65                  70                  75                  80
   Gly Ser Leu Glu Leu Val Phe Leu Val Asp Glu Ser Ser Ser Val Gly
                   85                  90                  95
   Gln Thr Asn Phe Leu Asn Glu Leu Lys Phe Val Arg Lys Leu Leu Ser
               100                 105                 110
   Asp Phe Pro Val Val Ser Thr Ala Thr Arg Val Ala Ile Val Thr Phe
           115                 120                 125
   Ser Ser Lys Asn Asn Val Val Ala Arg Val Asp Tyr Ile Ser Thr Ser
       130                 135                 140
   Arg Ala His Gln His Lys Cys Ala Leu Leu Ser Arg Glu Ile Pro Ala
   145                 150                 155                 160
   Ile Thr Tyr Arg Gly Gly Gly Thr Tyr Thr Lys Gly Ala Phe Gln Gln
                   165                 170                 175
   Ala Ala Gln Ile Leu Arg His Ser Arg Glu Asn Ser Thr Lys Val Ile
               180                 185                 190
   Phe Leu Ile Thr Asp Gly Tyr Ser Asn Gly Gly Asp Pro Arg Pro Ile
           195                 200                 205
   Ala Ala Ser Leu Arg Asp Phe Gly Val Glu Ile Phe Thr Phe Gly Ile
       210                 215                 220
   Trp Gln Gly Asn Ile Arg Glu Leu Asn Asp Met Ala Ser Thr Pro Lys
   225                 230                 235                 240
   Glu Glu His Cys Tyr Leu Leu His Ser Phe Glu Glu Phe Glu Ala Leu
                   245                 250                 255
   Ala Arg Arg Ala Leu His Glu Asp Leu Pro Ser Gly Ser Phe Ile Gln
               260                 265                 270
   Glu Asp Met Ala Arg Cys Ser Tyr Leu Cys Glu Ala Gly Lys Asp Cys
           275                 280                 285
   Cys Asp Arg Met Ala Ser Cys Lys Cys Gly Thr His Thr Gly Gln Phe
       290                 295                 300
   Glu Cys Ile Cys Glu Lys Gly Tyr Tyr Gly Lys Gly Leu Gln His Glu
   305                 310                 315                 320
   Cys Thr Ala Cys Pro Ser Gly Thr Tyr Lys Pro Glu Ala Ser Pro Gly
                   325                 330                 335
   Gly Ile Ser Thr Cys Ile Pro Cys Pro Asp Val Ser His Thr Ser Pro
               340                 345                 350
   Pro Gly Ser Thr Ser Pro Glu Asp Cys Val Cys Arg Glu Gly Tyr Gln
           355                 360                 365
   Arg Ser Gly Gln Thr Cys Glu Val Val His Cys Pro Ala Leu Lys Pro
       370                 375                 380
   Pro Glu Asn Gly Phe Phe Ile Gln Asn Thr Cys Lys Asn His Phe Asn
   385                 390                 395                 400
   Ala Ala Cys Gly Val Arg Cys Arg Pro Gly Phe Asp Leu Val Gly Ser
                   405                 410                 415
   Ser Ile His Leu Cys Gln Pro Asn Gly Leu Trp Ser Gly Thr Glu Ser
               420                 425                 430
   Phe Cys Arg Val Arg Thr Cys Pro His Leu Arg Gln Pro Lys His Gly
           435                 440                 445
   His Ile Ser Cys Ser Thr Ala Glu Met Ser Tyr Asn Thr Leu Cys Leu
       450                 455                 460
   Val Thr Cys Asn Glu Gly Tyr Arg Leu Glu Gly Ser Thr Arg Leu Thr
   465                 470                 475                 480
   Cys Gln Gly Asn Ala Gln Trp Asp Gly Pro Glu Pro Arg Cys Val Glu
                   485                 490                 495
   Arg His Cys Ala Thr Phe Gln Lys Pro Lys Gly Val Ile Ile Ser Pro
               500                 505                 510
   Pro Ser Cys Gly Lys Gln Pro Ala Arg Pro Gly Met Thr Cys Gln Leu
           515                 520                 525
   Ser Cys Arg Gln Gly Tyr Ile Leu Ser Gly Val Arg Glu Val Arg Cys
       530                 535                 540
   Ala Thr Ser Gly Lys Trp Ser Ala Lys Val Gln Thr Ala Val Cys Lys
   545                 550                 555                 560
   Asp Val Glu Ala Pro Gln Ile Ser Cys Pro Asn Asp Ile Glu Ala Lys
                   565                 570                 575
   Thr Gly Glu Gln Gln Asp Ser Ala Asn Val Thr Trp Gln Val Pro Thr
               580                 585                 590
   Ala Lys Asp Asn Ser Gly Glu Lys Val Ser Val His Val His Pro Ala
           595                 600                 605
   Phe Thr Pro Pro Tyr Leu Phe Pro Ile Gly Asp Val Ala Ile Thr Tyr
   610 615 620
   Thr Ala Thr Asp Ser Ser Gly Asn Gln Ala Ser Cys Thr Phe Tyr Ile
   625 630 635 640
   Lys Val Ile Asp Val Glu Pro Pro Val Ile Asp Trp Cys Arg Ser Pro
   645 650 655
   Pro Pro Ile Gln Val Val Glu Lys Glu His Pro Ala Ser Trp Asp Glu
   660 665 670
   Pro Gln Phe Ser Asp Asn Ser Gly Ala Glu Leu Val Ile Thr Ser Ser
   675 680 685
   His Thr Gln Gly Asp Met Phe Pro His Gly Glu Thr Val Val Trp Tyr
   690 695 700
   Thr Ala Thr Asp Pro Ser Gly Asn Asn Arg Thr Cys Asp Ile His Ile
   705 710 715 720
   Val Ile Lys Gly Ser Pro Cys Glu Val Pro Phe Thr Pro Val Asn Gly
   725 730 735
   Asp Phe Ile Cys Ala Gln Asp Ser Ala Gly Val Asn Cys Ser Leu Ser
   740 745 750
   Cys Lys Glu Gly Tyr Asp Phe Thr Glu Gly Ser Thr Glu Lys Tyr Tyr
   755 760 765
   Cys Ala Phe Glu Asp Gly Ile Trp Arg Pro Pro Tyr Ser Thr Glu Trp
   770 775 780
   Pro Asp Cys Ala Ile Lys Arg Phe Ala Asn His Gly Phe Lys Ser Phe
   785 790 795 800
   Glu Met Leu Tyr Lys Thr Thr Arg Cys Asp Asp Met Asp Leu Phe Lys
   805 810 815
   Lys Phe Ser Ala Ala Phe Glu Thr Thr Leu Gly Asn Met Val Pro Ser
   820 825 830
   Phe Cys Asn Asp Ala Asp Asp Ile Asp Cys Arg Leu Glu Asp Leu Thr
   835 840 845
   Lys Lys Tyr Cys Ile Glu Tyr Asn Tyr Asn Tyr Glu Asn Gly Phe Ala
   850 855 860
   Ile Gly Pro Gly Gly Trp Gly Ala Gly Asn Arg Leu Asp Tyr Ser Tyr
   865                 870                 875                 880
   Asp His Phe Leu Asp Val Val Gln Glu Thr Pro Thr Asp Val Gly Lys
                   885                 890                 895
   Ala Arg Ser Ser Arg Ile Lys Arg Thr Val Pro Leu Ser Asp Pro Lys
               900                 905                 910
   Ile Gln Leu Ile Phe Asn Ile Thr Ala Ser Val Pro Leu Pro Glu Glu
           915                 920                 925
   Arg Asn Asp Thr Leu Glu Leu Glu Asn Gln Gln Arg Leu Ile Lys Thr
       930                 935                 940
   Leu Glu Thr Ile Thr Asn Arg Leu Lys Ser Thr Leu Asn Lys Glu Pro
   945                 950                 955                 960
   Met Tyr Ser Phe Gln Leu Ala Ser Glu Thr Val Val Ala Asp Ser Asn
                   965                 970                 975
   Ser Leu Glu Thr Glu Lys Ala Phe Leu Phe Cys Arg Pro Gly Ser Val
               980                 985                 990
   Leu Arg Gly Arg Met Cys Val Asn  Cys Pro Leu Gly Thr  Ser Tyr Ser
           995                 1000                 1005
   Leu Glu  His Ser Thr Cys Glu  Ser Cys Leu Met Gly  Ser Tyr Gln
       1010                 1015                 1020
   Asp Glu  Glu Gly Gln Leu Glu  Cys Lys Leu Cys Pro  Pro Arg Thr
       1025                 1030                 1035
   His Thr  Glu Tyr Leu His Ser  Arg Ser Val Ser Glu  Cys Lys Ala
       1040                 1045                 1050
   Gln Cys  Lys Gln Gly Thr Tyr  Ser Ser Ser Gly Leu  Glu Thr Cys
       1055                 1060                 1065
   Glu Ser  Cys Pro Leu Gly Thr  Tyr Gln Pro Glu Phe  Gly Ser Arg
       1070                 1075                 1080
   Ser Cys  Leu Leu Cys Pro Glu  Thr Thr Thr Thr Val  Lys Arg Gly
       1085                 1090                 1095
   Ala Val  Asp Ile Ser Ala Cys  Gly Val Pro Cys Pro  Val Gly Glu
       1100                 1105                 1110
   Phe Ser  Arg Ser Gly Leu Thr  Pro Cys Tyr Pro Cys  Pro Arg Asp
       1115                 1120                 1125
   Tyr Tyr  Gln Pro Asn Ala Gly  Lys Ser Phe Cys Leu  Ala Cys Pro
       1130                 1135                 1140
   Phe Tyr  Gly Thr Thr Thr Ile  Thr Gly Ala Thr Ser  Ile Thr Asp
       1145                 1150                 1155
   Cys Ser  Ser Phe Ser Ser Thr  Phe Ser Ala Ala Glu  Glu Ser Ile
       1160                 1165                 1170
   Val Pro  Leu Val Ala Pro Gly  His Ser Gln Asn Lys  Tyr Glu Val
       1175                 1180                 1185
   Ser Ser  Gln Val Phe His Glu  Cys Phe Leu Asn Pro  Cys His Asn
       1190                 1195                 1200
   Ser Gly  Thr Cys Gln Gln Leu  Gly Arg Gly Tyr Val  Cys Leu Cys
       1205                 1210                 1215
   Pro Pro  Gly Tyr Thr Gly Leu  Lys Cys Glu Thr Asp  Ile Asp Glu
       1220                 1225                 1230
   Cys Ser  Ser Leu Pro Cys Leu  Asn Gly Gly Ile Cys  Arg Asp Gln
       1235                 1240                 1245
   Val Gly  Gly Phe Thr Cys Glu  Cys Ser Leu Gly Tyr  Ser Gly Gln
       1250                 1255                 1260
   Ile Cys  Glu Glu Asn Ile Asn  Glu Cys Ile Ser Ser  Pro Cys Leu
       1265                 1270                 1275
   Asn Lys  Gly Thr Cys Thr Asp  Gly Leu Ala Ser Tyr  Arg Cys Thr
       1280                 1285                 1290
   Cys Val  Lys Gly Tyr Met Gly  Val His Cys Glu Thr  Asp Val Asn
       1295                 1300                 1305
   Glu Cys  Gln Ser Ser Pro Cys  Leu Asn Asn Ala Val  Cys Lys Asp
       1310                 1315                 1320
   Gln Val  Gly Gly Phe Ser Cys  Lys Cys Pro Pro Gly  Phe Leu Gly
       1325                 1330                 1335
   Thr Arg  Cys Glu Lys Asn Val  Asp Glu Cys Leu Ser  Gln Pro Cys
       1340                 1345                 1350
   Gln Asn Gly Ala Thr Cys Lys Asp Gly Ala Asn Ser Phe Arg Cys
   1355 1360 1365
   Gln Cys Pro Ala Gly Phe Thr Gly Thr His Cys Glu Leu Asn Ile
   1370 1375 1380
   Asn Glu Cys Gln Ser Asn Pro Cys Arg Asn Gln Ala Thr Cys Val
   1385 1390 1395
   Asp Glu Leu Asn Ser Tyr Ser Cys Lys Cys Gln Pro Gly Phe Ser
   1400 1405 1410
   Gly His Arg Cys Glu Thr Glu Gln Pro Ser Gly Phe Asn Leu Asp
   1415 1420 1425
   Phe Glu Val Ser Gly Ile Tyr Gly Tyr Val Leu Leu Asp Gly Val
   1430 1435 1440
   Leu Pro Thr Leu His Ala Ile Thr Cys Ala Phe Trp Met Lys Ser
   1445 1450 1455
   Ser Asp Val Ile Asn Tyr Gly Thr Pro Ile Ser Tyr Ala Leu Glu
   1460 1465 1470
   Asp Asp Lys Asp Asn Thr Phe Leu Leu Thr Asp Tyr Asn Gly Trp
   1475 1480 1485
   Val Leu Tyr Val Asn Gly Lys Glu Lys Ile Thr Asn Cys Pro Ser
   1490 1495 1500
   Val Asn Asp Gly Ile Trp His His Ile Ala Ile Thr Trp Thr Ser
   1505 1510 1515
   Thr Gly Gly Ala Trp Arg Val Tyr Ile Asp Gly Glu Leu Ser Asp
   1520 1525 1530
   Gly Gly Thr Gly Leu Ser Ile Gly Lys Ala Ile Pro Gly Gly Gly
   1535 1540 1545
   Ala Leu Val Leu Gly Gln Glu Gln Asp Lys Lys Gly Glu Gly Phe
   1550 1555 1560
   Asn Pro Ala Glu Ser Phe Val Gly Ser Ile Ser Gln Leu Asn Leu
   1565 1570 1575
   Trp Asp Tyr Val Leu Ser Pro Gln Gln Val Lys Leu Leu Ala Ser
   1580 1585 1590
   Ser Cys Pro Glu Glu Leu Ser Arg Gly Asn Val Leu Ala Trp Pro
   1595 1600 1605
   Asp Phe Leu Ser Gly Ile Thr Gly Lys Val Lys Val Asp Ser Ser
   1610 1615 1620
   Ser Met Phe Cys Ser Asp Cys Pro Ser Leu Glu Gly Ser Val Pro
   1625 1630 1635
   His Leu Arg Pro Ala Ser Gly Asn Arg Lys Pro Gly Ser Lys Val
   1640 1645 1650
   Ser Leu Phe Cys Asp Pro Gly Phe Gln Met Val Gly Asn Pro Val
   1655 1660 1665
   Gln Tyr Cys Leu Asn Gln Gly Gln Trp Thr Gln Pro Leu Pro His
   1670 1675 1680
   Cys Glu Arg Ile Arg Cys Gly Leu Pro Pro Ala Leu Glu Asn Gly
   1685 1690 1695
   Phe Tyr Ser Ala Glu Asp Phe His Ala Gly Ser Thr Val Thr Tyr
   1700 1705 1710
   Gln Cys Thr Ser Gly Tyr Tyr Leu Leu Gly Asp Ser Arg Met Phe
   1715 1720 1725
   Cys Thr Asp Asn Gly Ser Trp Asn Gly Ile Ser Pro Ser Cys Leu
   1730 1735 1740
   Asp Val Asp Glu Cys Ala Val Gly Ser Asp Cys Ser Glu His Ala
   1745 1750 1755
   Ser Cys Leu Asn Thr Asn Gly Ser Tyr Val Cys Ser Cys Asn Pro
   1760 1765 1770
   Pro Tyr Thr Gly Asp Gly Lys Asn Cys Ala Glu Pro Val Lys Cys
   1775 1780 1785
   Lys Ala Pro Glu Asn Pro Glu Asn Gly His Ser Ser Gly Glu Ile
   1790 1795 1800
   Tyr Thr Val Gly Thr Ala Val Thr Phe Ser Cys Asp Glu Gly His
   1805 1810 1815
   Glu Leu  Val Gly Val Ser Thr  Ile Thr Cys Leu Glu  Thr Gly Glu
       1820                 1825                 1830
   Trp Asp  Arg Leu Arg Pro Ser  Cys Glu Ala Ile Ser  Cys Gly Val
       1835                 1840                 1845
   Pro Pro  Val Pro Glu Asn Gly  Gly Val Asp Gly Ser  Ala Phe Thr
       1850                 1855                 1860
   Tyr Gly  Ser Lys Val Val Tyr  Arg Cys Asp Lys Gly  Tyr Thr Leu
       1865                 1870                 1875
   Ser Gly  Asp Glu Glu Ser Ala  Cys Leu Ala Ser Gly  Ser Trp Ser
       1880                 1885                 1890
   His Ser  Ser Pro Val Cys Glu  Leu Val Lys Cys Ser  Gln Pro Glu
       1895                 1900                 1905
   Asp Ile  Asn Asn Gly Lys Tyr  Ile Leu Ser Gly Leu  Thr Tyr Leu
       1910                 1915                 1920
   Ser Ile  Ala Ser Tyr Ser Cys  Glu Asn Gly Tyr Ser  Leu Gln Gly
       1925                 1930                 1935
   Pro Ser  Leu Leu Glu Cys Thr  Ala Ser Gly Ser Trp  Asp Arg Ala
       1940                 1945                 1950
   Pro Pro  Ser Cys Gln Leu Val  Ser Cys Gly Glu Pro  Pro Ile Val
       1955                 1960                 1965
   Lys Asp  Ala Val Ile Thr Gly  Ser Asn Phe Thr Phe  Gly Asn Thr
       1970                 1975                 1980
   Val Ala  Tyr Thr Cys Lys Glu  Gly Tyr Thr Leu Ala  Gly Pro Asp
       1985                 1990                 1995
   Thr Ile  Val Cys Gln Ala Asn  Gly Lys Trp Asn Ser  Ser Asn His
       2000                 2005                 2010
   Gln Cys  Leu Ala Val Ser Cys  Asp Glu Pro Pro Asn  Val Asp His
       2015                 2020                 2025
   Ala Ser  Pro Glu Thr Ala His  Arg Leu Phe Gly Asp  Thr Ala Phe
       2030                 2035                 2040
   Tyr Tyr  Cys Ala Asp Gly Tyr  Ser Leu Ala Asp Asn  Ser Gln Leu
       2045                 2050                 2055
   Ile Cys  Asn Ala Gln Gly Asn  Trp Val Pro Pro Ala  Gly Gln Ala
       2060                 2065                 2070
   Val Pro  Arg Cys Ile Ala His  Phe Cys Glu Lys Pro  Pro Ser Val
       2075                 2080                 2085
   Ser Tyr  Ser Ile Leu Glu Ser  Val Ser Lys Ala Lys  Phe Ala Ala
       2090                 2095                 2100
   Gly Ser  Val Val Ser Phe Lys  Cys Met Glu Gly Phe  Val Leu Asn
       2105                 2110                 2115
   Thr Ser  Ala Lys Ile Glu Cys  Leu Arg Gly Gly Glu  Trp Ser Pro
       2120                 2125                 2130
   Ser Pro  Leu Ser Val Gln Cys  Ile Pro Val Arg Cys  Gly Glu Pro
       2135                 2140                 2145
   Pro Ser  Ile Ala Asn Gly Tyr  Pro Ser Gly Thr Asn  Tyr Ser Phe
       2150                 2155                 2160
   Gly Ala  Val Val Ala Tyr Ser  Cys His Lys Gly Phe  Tyr Ile Lys
       2165                 2170                 2175
   Gly Glu  Lys Lys Ser Thr Cys  Glu Ala Thr Gly Gln  Trp Ser Lys
       2180                 2185                 2190
   Pro Thr  Pro Thr Cys His Pro  Val Ser Cys Asn Glu  Pro Pro Lys
       2195                 2200                 2205
   Val Glu  Asn Gly Phe Leu Glu  His Thr Thr Gly Arg  Thr Phe Glu
       2210                 2215                 2220
   Ser Glu  Ala Arg Phe Gln Cys  Asn Pro Gly Tyr Lys  Ala Ala Gly
       2225                 2230                 2235
   Ser Pro  Val Phe Val Cys Gln  Ala Asn Arg His Trp  His Ser Asp
       2240                 2245                 2250
   Ala Pro  Leu Ser Cys Thr Pro  Leu Asn Cys Gly Lys  Pro Pro Pro
       2255                 2260                 2265
   Ile Gln  Asn Gly Phe Leu Lys  Gly Glu Ser Phe Glu  Val Gly Ser
       2270                 2275                 2280
   Lys Val  Gln Phe Val Cys Asn  Glu Gly Tyr Glu Leu  Val Gly Asp
       2285                 2290                 2295
   Asn Ser  Trp Thr Cys Gln Lys  Ser Gly Lys Trp Ser  Lys Lys Pro
       2300                 2305                 2310
   Ser Pro  Lys Cys Val Pro Thr  Lys Cys Ala Glu Pro  Pro Leu Leu
       2315                 2320                 2325
   Glu Asn  Gln Leu Val Leu Lys  Glu Leu Ala Ser Glu  Val Gly Val
       2330                 2335                 2340
   Met Thr  Ile Ser Cys Lys Glu  Gly His Ala Leu Gln  Gly Pro Ser
       2345                 2350                 2355
   Val Leu  Lys Cys Leu Pro Ser  Gly Gln Trp Asn Gly  Ser Phe Pro
       2360                 2365                 2370
   Ile Cys  Lys Met Val Leu Cys  Pro Ser Pro Pro Leu  Ile Pro Phe
       2375                 2380                 2385
   Gly Val  Pro Ala Ser Ser Gly  Ala Leu His Phe Gly  Ser Thr Val
       2390                 2395                 2400
   Lys Tyr  Leu Cys Val Asp Gly  Phe Phe Leu Arg Gly  Ser Pro Thr
       2405                 2410                 2415
   Ile Leu  Cys Gln Ala Asp Ser  Thr Trp Ser Ser Pro  Leu Pro Glu
       2420                 2425                 2430
   Cys Val  Pro Val Glu Cys Pro  Gln Pro Glu Glu Ile  Leu Asn Gly
       2435                 2440                 2445
   Ile Ile  His Val Gln Gly Leu  Ala Tyr Leu Ser Thr  Thr Leu Tyr
       2450                 2455                 2460
   Thr Cys  Lys Pro Gly Phe Glu  Leu Val Gly Asn Ala  Thr Thr Leu
       2465                 2470                 2475
   Cys Gly  Glu Asn Gly Gln Trp  Leu Gly Gly Lys Pro  Met Cys Lys
       2480                 2485                 2490
   Pro Ile  Glu Cys Pro Glu Pro  Lys Glu Ile Leu Asn  Gly Gln Phe
       2495                 2500                 2505
   Ser Ser  Val Ser Phe Gln Tyr  Gly Gln Thr Ile Thr  Tyr Phe Cys
       2510                 2515                 2520
   Asp Arg  Gly Phe Arg Leu Glu  Gly Pro Lys Ser Leu  Thr Cys Leu
   2525 2530 2535
   Glu Thr Gly Asp Trp Asp Met Asp Pro Pro Ser Cys Asp Ala Ile
   2540 2545 2550
   His Cys Ser Asp Pro Gln Pro Ile Glu Asn Gly Phe Val Glu Gly
   2555 2560 2565
   Ala Asp Tyr Arg Tyr Gly Ala Met Ile Ile Tyr Ser Cys Phe Pro
   2570 2575 2580
   Gly Phe Gln Val Leu Gly His Ala Met Gln Thr Cys Glu Glu Ser
   2585 2590 2595
   Gly Trp Ser Ser Ser Ser Pro Thr Cys Val Pro Ile Asp Cys Gly
   2600 2605 2610
   Leu Pro Pro His Ile Asp Phe Gly Asp Cys Thr Lys Val Arg Asp
   2615 2620 2625
   Gly Gln Gly His Phe Asp Gln Glu Asp Asp Met Met Glu Val Pro
   2630 2635 2640
   Tyr Leu Ala His Pro Gln His Leu Glu Ala Thr Ala Lys Ala Leu
   2645 2650 2655
   Glu Asn Thr Lys Glu Ser Pro Ala Ser His Ala Ser His Phe Leu
   2660 2665 2670
   Tyr Gly Thr Met Val Ser Tyr Ser Cys Glu Pro Gly Tyr Glu Leu
   2675 2680 2685
   Leu Gly Ile Pro Val Leu Ile Cys Gln Glu Asp Gly Thr Trp Asn
   2690 2695 2700
   Gly Thr Ala Pro Ser Cys Ile Ser Ile Glu Cys Asp Leu Pro Val
   2705 2710 2715
   Ala Pro Glu Asn Gly Phe Leu His Phe Thr Gln Thr Thr Met Gly
   2720 2725 2730
   Ser Ala Ala Gln Tyr Ser Cys Lys Pro Gly His Ile Leu Glu Gly
   2735 2740 2745
   Ser His Leu Arg Leu Cys Leu Gln Asn Lys Gln Trp Ser Gly Thr
   2750 2755 2760
   Val Pro Arg Cys Glu Ala Ile Ser Cys Ser Lys Pro Asn Pro Leu
       2765                2770                2775
   Trp Asn Gly Ser Ile Lys Gly Asp Asp Tyr Ser Tyr Leu Gly Val
       2780                2785                2790
   Leu Tyr Tyr Glu Cys Asp Ser Gly Tyr Ile Leu Asn Gly Ser Lys
       2795                2800                2805
   Lys Arg Thr Cys Gln Glu Asn Arg Asp Trp Asp Gly His Glu Pro
       2810                2815                2820
   Met Cys Ile Pro Val Asp Cys Gly Ser Pro Pro Val Pro Thr Asn
       2825                2830                2835
   Gly Arg Val Lys Gly Glu Glu Tyr Thr Phe Gln Lys Glu Ile Thr
       2840                2845                2850
   Tyr Ser Cys Arg Glu Gly Phe Ile Leu Glu Gly Ala Arg Ser Arg
       2855                2860                2865
   Ile Cys Leu Thr Asn Gly Ser Trp Ser Gly Ala Thr Pro Ser Cys
       2870                2875                2880
   Met Pro Val Arg Cys Pro Ala Pro Pro Gln Val Pro Asn Gly Val
       2885                2890                2895
   Ala Asp Gly Leu Asp Tyr Gly Phe Lys Lys Glu Val Ala Phe His
       2900                2905                2910
   Cys Leu Glu Gly Tyr Val Leu Gln Gly Ala Pro Arg Leu Thr Cys
       2915                2920                2925
   Gln Ser Asn Gly Thr Trp Asp Ala Glu Val Pro Val Cys Lys Pro
       2930                2935                2940
   Ala Thr Cys Gly Pro Pro Ala Asp Leu Pro Gln Gly Phe Pro Asn
       2945                2950                2955
   Gly Phe Ser Phe Tyr His Gly Gly His Ile Gln Tyr Gln Cys Phe
       2960                2965                2970
   Thr Gly Tyr Lys Leu His Gly Asn Pro Ser Arg Arg Cys Leu Pro
       2975                2980                2985
   Asn Gly Ser Trp Ser Gly Ser Ser Pro Ser Cys Leu Pro Cys Arg
       2990                2995                3000
   Cys Ser  Thr Pro Ile Ile Gln  Gln Gly Thr Ile Asn  Ala Thr Asp
       3005                 3010                 3015
   Leu Gly  Cys Gly Lys Thr Val  Gln Ile Glu Cys Phe  Lys Gly Phe
       3020                 3025                 3030
   Lys Leu  Leu Gly Leu Ser Glu  Ile Thr Cys Asp Ala  Asn Gly Gln
       3035                 3040                 3045
   Trp Ser  Asp Val Pro Leu Cys  Glu His Ala Gln Cys  Gly Pro Leu
       3050                 3055                 3060
   Pro Thr  Ile Pro Asn Ala Ile  Val Leu Glu Gly Ser  Leu Ser Glu
       3065                 3070                 3075
   Asp Asn  Val Val Thr Tyr Ser  Cys Arg Pro Gly Tyr  Thr Met Gln
       3080                 3085                 3090
   Gly Ser  Ser Asp Leu Ile Cys  Thr Glu Lys Ala Ile  Trp Ser Gln
       3095                 3100                 3105
   Pro Tyr  Pro Thr Cys Glu Pro  Leu Ser Cys Gly Pro  Pro Pro Thr
       3110                 3115                 3120
   Val Ala  Asn Ala Val Ala Thr  Gly Glu Ala His Thr  Tyr Glu Ser
       3125                 3130                 3135
   Lys Val  Lys Leu Arg Cys Leu  Glu Gly Tyr Val Met  Asp Ser Asp
       3140                 3145                 3150
   Thr Asp  Thr Phe Thr Cys Gln  Gln Asp Gly His Trp  Val Pro Glu
       3155                 3160                 3165
   Arg Ile  Thr Cys Ser Pro Lys  Lys Cys Pro Val Pro  Ser Asn Met
       3170                 3175                 3180
   Thr Arg  Ile Arg Phe His Gly  Asp Asp Phe Gln Val  Asn Arg Gln
       3185                 3190                 3195
   Val Ser  Val Ser Cys Ala Glu  Gly Phe Thr His Glu  Gly Val Asn
       3200                 3205                 3210
   Trp Ser  Thr Cys Gln Pro Asp  Gly Thr Trp Glu Pro  Pro Phe Ser
       3215                 3220                 3225
   Asp Glu  Ser Cys Ile Pro Val  Val Cys Gly His Pro  Glu Ser Pro
       3230                 3235                 3240
   Ala His Gly Ser Val Val Gly Asn Lys His Ser Phe Gly Ser Thr
   3245 3250 3255
   Ile Val Tyr Gln Cys Asp Pro Gly Tyr Lys Leu Glu Gly Asn Arg
   3260 3265 3270
   Glu Arg Ile Cys Gln Glu Asn Arg Gln Trp Ser Gly Glu Val Ala
   3275 3280 3285
   Val Cys Arg Glu Asn Arg Cys Glu Thr Pro Ala Glu Phe Pro Asn
   3290 3295 3300
   Gly Lys Ala Val Leu Glu Asn Thr Thr Ser Gly Pro Ser Leu Leu
   3305 3310 3315
   Phe Ser Cys His Arg Gly Tyr Thr Leu Glu Gly Ser Pro Glu Ala
   3320 3325 3330
   His Cys Thr Ala Asn Gly Thr Trp Asn His Leu Thr Pro Leu Cys
   3335 3340 3345
   Lys Pro Asn Pro Cys Pro Val Pro Phe Val Ile Pro Glu Asn Ala
   3350 3355 3360
   Val Leu Ser Glu Lys Glu Phe Tyr Val Asp Gln Asn Val Ser Ile
   3365 3370 3375
   Lys Cys Arg Glu Gly Phe Leu Leu Lys Gly Asn Gly Val Ile Thr
   3380 3385 3390
   Cys Ser Pro Asp Glu Thr Trp Thr His Thr Asn Ala Arg Cys Glu
   3395 3400 3405
   Lys Ile Ser Cys Gly Pro Pro Ser His Val Glu Asn Ala Ile Ala
   3410 3415 3420
   Arg Gly Val Tyr Tyr Gln Tyr Gly Asp Met Ile Thr Tyr Ser Cys
   3425 3430 3435
   Tyr Ser Gly Tyr Met Leu Glu Gly Ser Leu Arg Ser Val Cys Leu
   3440 3445 3450
   Glu Asn Gly Thr Trp Thr Pro Ser Pro Val Cys Arg Ala Val Cys
   3455 3460 3465
   Arg Phe Pro Cys Gln Asn Gly Gly Val Cys Gln Arg Pro Asn Ala
   3470 3475 3480
   Cys Ser Cys Pro Asp Gly Trp Met Gly Arg Leu Cys Glu Glu Pro
   3485 3490 3495
   Ile Cys Ile Leu Pro Cys Leu Asn Gly Gly Arg Cys Val Ala Pro
   3500 3505 3510
   Tyr Gln Cys Asp Cys Pro Thr Gly Trp Thr Gly Ser Arg Cys His
   3515 3520 3525
   Thr Ala Thr Cys Gln Ser Pro Cys Leu Asn Gly Gly Lys Cys Ile
   3530 3535 3540
   Arg Pro Asn Arg Cys His Cys Leu Ser Ala Trp Thr Gly His Asp
   3545 3550 3555
   Cys Ser Arg Lys Arg Arg Ala Gly Leu
   3560 3565
<210> 3
   <211> 11275
   <212> DNA
   <213> Mus musculus
<400> 3
   atagccgaga gtcagaggag cacatccctc cagtccctcc cgagtccccg agctgccaga      60
   ggagtctgga tcgtgtcccc agtgtcacat gcaaggacgc tgaggttcgc ggttgctacc     120
   ccgggtcccc tccgcttagt ccgggaacct tggcgcctct ctgcgcgctc ggggactgtc     180
   gccttgcact ccccggggcc accgctcggt ccccagcggg atgtggtcgc gcctggcctt     240
   ttgttgctgg gctctggcac tggtgtcagg ctggaccaac ttccagcccg tggccccttc     300
   gctcaacttc agcttccgcc tgttccccga ggcctctccg ggggctctgg gcagactggc     360
   ggtacctccc gcgtccagtg aggaggaggc agcagggagc aaagtggagc gcctgggccg     420
   cgcgttccgg agccgcgtgc ggcgactgcg ggagctcagc ggcagcctgg agctcgtctt     480
   cctggtggac gagtcgtcca gcgtgggcca aaccaacttc ctcaacgagc tcaagttcgt     540
   gcgcaagctg ctgtccgact tccccgtggt gtccacggcc acgcgtgtgg ccatcgtcac     600
   cttctcatcc aagaacaacg tggtggcgcg cgtggattac atctccacca gccgcgcgca     660
   ccaacacaag tgcgcgctac tcagccgcga gatcccggcc atcacctacc gcggtggtgg     720
   cacctatacc aagggcgcct tccagcaagc cgcgcaaatc cttcgtcact ctagagaaaa     780
   ctccaccaaa gtcatatttc tcatcaccga cggctattcc aatggcggag accccagacc     840
   tattgcagca tcgcttcggg atttcggagt ggagatcttc acgttcggga tttggcaggg     900
   gaatatccgg gaactgaatg acatggcttc caccccgaag gaagaacatt gttacctgct 960
   ccacagtttt gaagaatttg aggctttagc tcgcagggcg ttgcatgaag atctaccttc 1020
   tgggagtttt atccaagagg atatggcccg ctgctcttat ctctgtgagg ctgggaaaga 1080
   ctgctgtgac agaatggcca gctgcaaatg tgggacacac acgggtcaat ttgaatgcat 1140
   ctgtgagaag ggctattacg ggaaaggtct gcagcatgag tgcacagctt gcccatcagg 1200
   gacatataag ccggaagctt ctccaggagg aatcagcacc tgcatcccat gtcctgacgt 1260
   aagccacacc tccccacctg gaagcacttc ccctgaagac tgcgtgtgcc gagagggata 1320
   ccagagatct ggccagacct gtgaggttgt ccactgtcct gccctgaagc ctcctgaaaa 1380
   tggttttttt atacaaaaca cttgcaaaaa ccacttcaat gccgcctgtg gggtccgatg 1440
   tcgcccgggc tttgaccttg tgggaagcag catccatttg tgtcaaccca atggtttgtg 1500
   gtctgggaca gaaagcttct gcagagtgag aacgtgcccc cacctccgac agcccaaaca 1560
   cggccacatc agctgctcca ctgcggaaat gtcctacaac accctgtgtt tggttacctg 1620
   caatgaagga tacagattag aaggcagcac taggcttacc tgtcaaggaa atgcccagtg 1680
   ggatggccca gagccccggt gtgtagaacg ccattgtgcc accttccaga agcccaaagg 1740
   cgtcatcatt tctccaccca gctgcggcaa gcagcctgcc aggcctggga tgacctgtca 1800
   gctaagctgc cgccagggat acattttatc cggggtcaga gaagtgagat gtgccacatc 1860
   tgggaagtgg agtgccaaag ttcagacagc tgtgtgcaaa gatgtggagg ctccacaaat 1920
   cagctgtcca aatgacattg aggcaaagac tggggagcag caggactctg ctaatgtcac 1980
   ctggcaagtc ccaacagcta aagacaactc tggtgaaaag gtgtcagtcc acgtccaccc 2040
   agcctttacc ccaccttacc tcttcccaat tggagacgtg gccatcacct acacggcaac 2100
   cgactcatcc ggtaaccaag ccagctgcac tttctacatt aaggtcattg atgtggaacc 2160
   gcctgtcata gattggtgcc gatctccacc tccaatccag gtcgtagaga aggagcaccc 2220
   tgcaagctgg gatgagcctc agttctcaga caactccggg gctgaattgg tcattaccag 2280
   cagtcacaca caaggcgaca tgtttcctca tggggaaacg gtggtgtggt acacagccac 2340
   tgacccctca ggcaacaaca ggacctgtga catccacatt gtcataaaag gttctccctg 2400
   tgaggtcccc ttcacccctg taaacgggga ctttatctgt gcccaggata gtgctggagt 2460
   taactgtagc ctgagctgca aggagggcta tgatttcaca gaagggtcaa ctgagaagta 2520
   ctactgtgct tttgaagatg gtatctggag accaccatac tctacagaat ggccagactg 2580
   tgctataaaa cgttttgcaa accatggttt caagtccttt gaaatgctat acaaaaccac 2640
   tcgctgtgat gacatggatc tgtttaagaa gttttctgca gcatttgaga ctaccctggg 2700
   gaacatggtc ccgtcctttt gtaacgatgc tgatgacatt gactgcagac tggaggacct 2760
   gaccaaaaaa tactgcatcg agtataatta caactatgaa aatggctttg caattggacc 2820
   aggaggctgg ggtgcaggca acaggctgga ttattcctac gatcacttcc tggatgttgt 2880
   acaggaaaca cccaccgatg tgggcaaggc cagatcgtca cggattaaaa gaactgtccc 2940
   attgtctgac cccaaaattc agctaatttt taacatcaca gctagcgtgc cactcccaga   3000
   ggaaagaaac gatacccttg aattggagaa tcagcagcga ctcattaaga cattggaaac   3060
   aatcaccaat cgcctgaaaa gcaccttgaa taaagagccc atgtattctt tccagctcgc 3120
   ctcggaaaca gtggtggctg acagcaattc cctcgaaaca gaaaaggctt ttctcttctg 3180
   cagaccaggc tctgtgctga gggggcgcat gtgtgtcaac tgccccctgg gaacctctta 3240
   ctctctggag cattccacct gtgaaagctg cctcatggga tcctaccaag atgaagaagg 3300
   gcagctggaa tgcaagctct gtcccccaag gactcacacg gaatacctcc attcaagaag 3360
   cgtctctgaa tgcaaagctc agtgtaagca aggcacctac tcttccagtg ggctggagac 3420
   ctgcgaatcg tgtccgctgg gtacttatca accggaattt ggatcccgga gctgcctcct 3480
   atgcccagaa accaccacaa cggtgaaaag aggagccgtg gacatctctg cttgtggagt 3540
   gccctgccca gtaggagaat tctcccgttc tgggctaaca ccctgctacc cttgccctcg 3600
   agactattac caacccaatg cagggaagtc cttctgcctc gcttgtccct tttatggaac 3660
   tacaaccatc actggcgcca cgtccatcac agactgctca agttttagct ctactttctc 3720
   agcagcagaa gaaagcatag tgcccctcgt ggcccctgga cattcccaga acaagtacga 3780
   agtcagcagt caggtctttc acgaatgctt cttaaacccc tgccacaaca gtggaacctg 3840
   ccaacagctt gggcgtggtt atgtctgtct ctgcccacct ggatacacag gcttaaagtg 3900
   tgaaacagat attgatgaat gcagctctct gccttgcctc aatggtggaa tttgtagaga 3960
   ccaagttggg ggattcacgt gcgaatgttc attgggctat tcaggtcaaa tatgtgaaga 4020
   aaatataaat gagtgtatct ccagcccttg cttaaataaa ggaacctgca ctgacggctt 4080
   ggcaagctac cgctgtacct gtgtgaaagg atacatgggt gtgcactgtg aaacagacgt 4140
   caatgaatgc cagtcaagcc cctgcttaaa caacgcagtt tgtaaagacc aagttggggg 4200
   gttctcatgc aaatgcccac ccggattttt gggtactcgg tgtgaaaaaa atgtggatga 4260
   gtgtctcagt cagccatgcc aaaatggagc cacttgtaag gatggtgcca acagcttcag 4320
   gtgtcaatgt ccagcaggct tcacagggac acactgtgaa ctgaacatca acgagtgtca 4380
   gtccaaccca tgtaggaacc aggccacctg tgtggatgaa ctaaactcat acagttgtaa 4440
   atgtcagcca ggattttcag gccacaggtg tgagacagaa cagccttccg gttttaacct 4500
   ggattttgaa gtttctggca tctacgggta cgtcctgcta gatggagtgc tgccaaccct 4560
   ccatgccata acctgcgcat tctggatgaa atcctctgat gtcatcaact acgggacgcc 4620
   catctcctat gcacttgagg atgacaaaga caacaccttc ctcctgactg attacaacgg 4680
   ctgggttctt tatgtgaatg gaaaggaaaa gatcaccaac tgcccctccg taaatgatgg 4740
   catttggcat catattgcaa tcacatggac aagtactggt ggagcctgga gggtctatat 4800
   agatggggaa ttatctgacg gtggtactgg cctctccatt ggcaaagcca tacctggtgg 4860
   cggtgcatta gttcttgggc aagagcaaga caaaaaagga gaggggttca acccggctga 4920
   gtcttttgtg ggctccataa gccagctcaa cctctgggac tatgtcctgt ctccacagca 4980
   ggtgaagttg ctggccagct cctgcccaga ggaactgagt cggggaaacg tgttagcatg 5040
   gcccgatttc ctgtcgggaa tcacggggaa ggtgaaggtt gattccagca gcatgttctg 5100
   ctctgattgt ccgtctttag aaggatccgt gcctcacctg agacctgcat caggaaatcg 5160
   aaagccaggc tccaaagtca gtctgttctg tgatccgggc ttccagatgg ttgggaatcc 5220
   tgtgcagtat tgtctgaacc aagggcagtg gacacaacca ctcccccact gtgaacgcat 5280
   tcgctgtggg ctgcctcccg ccttggagaa tggcttctac tcagccgagg acttccatgc 5340
   gggcagcacg gtgacctatc agtgcaccag tggctactac ctgctgggtg attcccgaat 5400
   gttctgcaca gacaacggga gctggaacgg catttcacca tcctgtctcg atgtcgatga 5460
   gtgtgcagtc ggctcggact gtagtgagca cgcctcctgc ctgaacacca acggatccta 5520
   cgtatgttcc tgtaacccac catacacggg agatgggaaa aactgtgcag aacctgtaaa 5580
   atgtaaggct ccagaaaatc cagaaaatgg ccactcttct ggtgagattt acaccgtggg 5640
   tactgcagtc acattttcct gtgacgaagg gcacgagctg gtgggagtga gcaccatcac 5700
   gtgtttggag actggcgagt gggatcgcct caggccgtcc tgtgaagcca tttcctgtgg 5760
   tgtcccacct gttcctgaaa atggtggtgt tgacgggtcg gcattcacat acggcagtaa 5820
   ggtggtgtac aggtgtgata aaggatatac tttgtctggg gatgaagagt cagcatgcct 5880
   tgctagtggt tcctggagtc attcctctcc tgtgtgcgag ctagtgaagt gttcccagcc 5940
   tgaggacata aataacggca aatacatctt aagtgggctc acctaccttt ctattgcatc 6000
   gtactcctgt gagaacggat acagtttaca gggcccatcc ctccttgaat gcacagcttc 6060
   cggcagctgg gacagagcgc cacctagctg tcaacttgtc tcctgcggag agcctccaat 6120
   cgtcaaagat gctgtcatca ctgggagcaa cttcactttt gggaacacag ttgcttacac 6180
   atgcaaagag ggctacaccc ttgctgggcc tgacaccatc gtatgccagg ccaacggcaa 6240
   atggaattca agtaaccacc agtgcctggc tgtctcctgt gacgagcccc ccaatgtgga 6300
   ccacgcctct ccagagactg ctcacaggct ctttggagac accgcgtttt actactgtgc 6360
   ggatggttac agcctggctg ataattccca gctcatctgc aatgcccagg ggaactgggt 6420
   tccccccgcg ggccaggctg tgccgcgctg catagctcac ttctgtgaaa aacccccatc 6480
   tgtttcctac agcatcttgg aatctgtgag caaagcaaag tttgcagctg gctcggtagt 6540
   gagcttcaag tgcatggagg gttttgtgct gaacacctca gcgaagattg aatgcctgag 6600
   aggtggagag tggagccctt ctcccctctc ggtccagtgc atcccggtgc gatgcggaga 6660
   gcctccaagc atcgcaaatg gctacccgag tgggacaaac tacagttttg gggccgtggt 6720
   ggcctacagc tgccacaagg gattctatat caagggggag aagaagagca cgtgtgaggc 6780
   cacaggacag tggagtaaac ccacgcccac ctgccatcct gtgtcctgta acgagccacc 6840
   taaggttgag aacggcttcc tggagcacac cactggcagg acctttgaga gcgaagcaag 6900
   gttccagtgc aacccaggct ataaggcagc cggaagtcct gtgtttgttt gccaagccaa 6960
   tcgccactgg cacagcgacg cccctctgtc ctgcacccct ctcaactgtg ggaaaccccc 7020
   tcccattcag aatggctttt tgaaaggaga aagctttgaa gtagggtcca aggttcagtt 7080
   tgtctgtaat gagggatatg agctcgttgg tgataattct tggacttgcc agaaatctgg 7140
   caaatggagt aagaagccaa gcccgaagtg tgtccccacc aagtgtgcag agcctcctct 7200
   cttagaaaac cagctcgtat tgaaggaatt agcttccgag gtaggagtga tgaccatttc 7260
   ctgtaaagag gggcatgcct tgcaaggccc ctctgtcctg aagtgcttgc catccgggca 7320
   atggaatggt tcctttccta tttgtaagat ggtcctttgt ccctcgcctc ccttgattcc 7380
   cttcggcgtc cctgcgtctt ccggtgctct tcattttggc agtactgtca agtatctgtg 7440
   tgtcgacggg tttttcttaa gaggcagtcc aaccatcctc tgccaggctg atagcacctg 7500
   gagttctcca ttgcccgaat gcgttccggt agaatgtccc caacctgagg agatcctcaa 7560
   cggtatcatc cacgtacaag ggcttgccta tctcagcacc acgctctaca cctgcaagcc 7620
   aggctttgag ttagtgggca atgctaccac cctctgtggg gaaaatggcc agtggctcgg 7680
   aggaaaacca atgtgcaaac ccattgaatg cccagagccc aaggagattt taaatggcca 7740
   attctcttcc gtgagctttc agtatggaca aaccatcaca tacttttgtg accggggctt 7800
   ccggctcgaa ggtcccaaat ccctgacctg tttagagaca ggtgactggg atatggatcc 7860
   cccctcttgt gatgccatcc actgcagtga cccacagccc attgaaaatg gtttcgtaga 7920
   aggtgcggat tacagatacg gtgccatgat catctatagc tgcttccctg ggtttcaggt 7980
   gcttggtcat gccatgcaga cctgtgaaga gtcgggatgg tcaagctcca gcccaacctg 8040
   tgtacccata gactgcggtc tccctcctca catagacttt ggtgactgta ctaaagtcag 8100
   agatggccag ggacattttg atcaagaaga tgacatgatg gaagtcccat atctggctca 8160
   ccctcaacat ttggaagcaa cagctaaggc cttggaaaat acaaaggagt cgcctgcctc 8220
   acatgcatcc cacttcctct atggcacgat ggtttcctac agctgcgagc ctggttatga 8280
   actgctggga atccctgtgc tgatctgcca ggaagatggt acgtggaatg gtaccgcacc 8340
   ctcttgcatt tccattgaat gtgatttgcc tgttgctccc gaaaatggct ttttacattt 8400
   cacacagacg actatgggca gtgctgcaca atatagctgc aagccggggc acattctaga 8460
   aggctcccac ttaagactct gtctgcagaa taagcagtgg agtggcactg ttccacgctg 8520
   tgaagccatc tcatgcagta agccaaaccc actctggaat ggatccatca aaggagatga 8580
   ctactcctac ctgggtgtgt tatactacga gtgtgactct ggctatattc tcaatggctc 8640
   taagaagagg acatgccaag aaaatagaga ttgggatggg catgagccca tgtgtattcc 8700
   tgtagactgt ggctcacccc cagtccccac caatggccga gtgaagggag aagaatacac 8760
   attccaaaag gagattacat actcttgccg tgaagggttc atactggaag gagccaggag 8820
   tcgtatctgt cttaccaatg gaagttggag tggtgccact cccagctgca tgcctgttag 8880
   atgtcctgcc ccaccacagg tgccaaatgg ggtggcagat ggcctagact atgggttcaa 8940
   gaaggaagta gcgttccact gtctagaggg ctatgtgctg cagggggctc caagactcac 9000
   ctgtcagtcc aatgggactt gggatgcaga agtccctgtc tgtaaaccag ctacctgtgg 9060
   tcctcctgcc gaccttcccc agggcttccc taatggcttt tctttttatc atgggggcca 9120
   catacagtat cagtgtttta ctggttataa gcttcatgga aacccatcaa gaagatgcct 9180
   tcccaatggt tcctggagcg gcagctcgcc atcctgccta ccttgcaggt gttccacacc 9240
   catcattcaa cagggaacca tcaacgcaac tgatttggga tgtggaaaga cggtccagat 9300
   tgagtgcttc aaaggcttca agctgcttgg actttctgaa atcacctgtg atgccaatgg 9360
   ccaatggtct gacgtcccac tgtgtgagca cgctcagtgc gggcctctcc caaccatacc 9420
   caacgcaatt gtccttgagg gcagcctttc ggaggacaat gtggtaactt acagctgcag 9480
   acctggctac accatgcaag gtagttcaga tctgatttgt acggaaaaag cgatatggag 9540
   ccagccttac ccaacgtgtg aacccctgtc ctgtggaccc ccaccaactg tagccaatgc 9600
   agtggcaaca ggagaggctc atacctatga aagcaaagtg aaactcaggt gtctggaagg 9660
   gtatgtgatg gattcggata cagatacatt cacctgccag caagatggcc attgggtccc 9720
   tgaaagaatc acctgcagtc ctaaaaaatg ccctgtgcca tccaacatga cacgcatacg 9780
   ttttcacgga gatgacttcc aggtgaacag acaagtttct gtgtcatgtg cagaagggtt 9840
   tacccacgaa ggagtgaact ggtcaacatg ccagcccgac ggtacatggg agccaccatt 9900
   ttctgatgaa tcctgtatcc cagttgtttg tgggcatcct gaaagcccag cgcatggctc 9960
   cgtggttggc aataagcaca gctttggaag caccattgtt taccagtgtg accctggcta 10020
   caaattagag gggaacaggg aacgaatctg ccaggagaac agacagtgga gtggagaggt 10080
   ggcagtgtgc agagagaaca gatgtgagac tccagctgag tttcccaatg ggaaggctgt 10140
   cttggaaaac accacatctg gacccagcct tctgttttcc tgtcacagag gctacaccct 10200
   ggaagggtcc cccgaggcac actgcactgc aaatggaacc tggaatcacc tgactcccct 10260
   ctgcaaacca aatccatgcc ctgtcccttt tgtgattcct gagaacgccg tcctttctga 10320
   aaaagagttt tatgtcgacc agaatgtatc tatcaagtgc agggaaggct tcctgctcaa 10380
   aggcaatggt gtcatcacgt gcagccctga cgagacatgg acgcacacca atgccagatg 10440
   tgaaaaaatc tcctgtggtc ctccaagtca cgtagagaat gcaattgctc gaggagtgta 10500
   ttaccagtat ggggacatga tcacctactc ctgttacagt ggctacatgc tagaaggttc 10560
   cctccggagt gtttgcctag aaaatggaac atggacacca tctcctgttt gcagagctgt 10620
   ctgtcggttc ccatgtcaga atggaggtgt ctgtcaacgt ccaaatgctt gctcatgccc 10680
   agacggctgg atgggacgtc tctgtgaaga gccaatatgc atactcccct gtttgaatgg 10740
   tgggcgctgt gtggcccctt atcagtgtga ctgccccaca ggctggactg ggtcccgctg 10800
   tcatacagct acttgtcagt ccccctgctt aaatggcggg aaatgcataa gaccaaaccg 10860
   atgccattgt ctctcagcct ggacaggaca tgattgttcc aggaaaagga gagccgggct 10920
   ttgatctcat gccccacccc ctctccctaa gcagcatcat ctccttccgg tagctcctgg 10980
   gactcccacc aagaaagacc aacgcggtgc tggggcttgt ttggttttat aagcttgagg 11040
   ttgacttttt aattttgtga tctattttgt taaatttttt tgtgacgtcc tttcttacat 11100
   gtgtgcgttg tttaaatatg cttgcatttt ctatataaaa tttatattaa acggacgcac 11160
   ttcatcctca ccagatgtac atactctgct gtctgctggg aaagcccctg gaatacattt 11220
   ttattcaatt acttaaagat gactttccat taaaatatat tttgctacta aataa 11275
<210> 4
   <211> 3571
   <212> PRT
   <213> Homo sapiens
<400> 4
   Met Trp Pro Arg Leu Ala Phe Cys Cys Trp Gly Leu Ala Leu Val Ser
   1               5                   10                  15
   Gly Trp Ala Thr Phe Gln Gln Met Ser Pro Ser Arg Asn Phe Ser Phe
               20                  25                  30
   Arg Leu Phe Pro Glu Thr Ala Pro Gly Ala Pro Gly Ser Ile Pro Ala
           35                  40                  45
   Pro Pro Ala Pro Gly Asp Glu Ala Ala Gly Ser Arg Val Glu Arg Leu
       50                  55                  60
   Gly Gln Ala Phe Arg Arg Arg Val Arg Leu Leu Arg Glu Leu Ser Glu
   65                  70                  75                  80
   Arg Leu Glu Leu Val Phe Leu Val Asp Asp Ser Ser Ser Val Gly Glu
                   85                  90                  95
   Val Asn Phe Arg Ser Glu Leu Met Phe Val Arg Lys Leu Leu Ser Asp
               100                 105                 110
   Phe Pro Val Val Pro Thr Ala Thr Arg Val Ala Ile Val Thr Phe Ser
           115                 120                 125
   Ser Lys Asn Tyr Val Val Pro Arg Val Asp Tyr Ile Ser Thr Arg Arg
       130                 135                 140
   Ala Arg Gln His Lys Cys Ala Leu Leu Leu Gln Glu Ile Pro Ala Ile
   145                 150                 155                 160
   Ser Tyr Arg Gly Gly Gly Thr Tyr Thr Lys Gly Ala Phe Gln Gln Ala
                   165                 170                 175
   Ala Gln Ile Leu Leu His Ala Arg Glu Asn Ser Thr Lys Val Val Phe
               180                 185                 190
   Leu Ile Thr Asp Gly Tyr Ser Asn Gly Gly Asp Pro Arg Pro Ile Ala
           195                 200                 205
   Ala Ser Leu Arg Asp Ser Gly Val Glu Ile Phe Thr Phe Gly Ile Trp
       210                 215                 220
   Gln Gly Asn Ile Arg Glu Leu Asn Asp Met Ala Ser Thr Pro Lys Glu
   225                 230                 235                 240
   Glu His Cys Tyr Leu Leu His Ser Phe Glu Glu Phe Glu Ala Leu Ala
                   245                 250                 255
   Arg Arg Ala Leu His Glu Asp Leu Pro Ser Gly Ser Phe Ile Gln Asp
               260                 265                 270
   Asp Met Val His Cys Ser Tyr Leu Cys Asp Glu Gly Lys Asp Cys Cys
           275                 280                 285
   Asp Arg Met Gly Ser Cys Lys Cys Gly Thr His Thr Gly His Phe Glu
       290                 295                 300
   Cys Ile Cys Glu Lys Gly Tyr Tyr Gly Lys Gly Leu Gln Tyr Glu Cys
   305                 310                 315                 320
   Thr Ala Cys Pro Ser Gly Thr Tyr Lys Pro Glu Gly Ser Pro Gly Gly
                   325                 330                 335
   Ile Ser Ser Cys Ile Pro Cys Pro Asp Glu Asn His Thr Ser Pro Pro
               340                 345                 350
   Gly Ser Thr Ser Pro Glu Asp Cys Val Cys Arg Glu Gly Tyr Arg Ala
           355                 360                 365
   Ser Gly Gln Thr Cys Glu Leu Val His Cys Pro Ala Leu Lys Pro Pro
       370                 375                 380
   Glu Asn Gly Tyr Phe Ile Gln Asn Thr Cys Asn Asn His Phe Asn Ala
   385                 390                 395                 400
   Ala Cys Gly Val Arg Cys His Pro Gly Phe Asp Leu Val Gly Ser Ser
                   405                 410                 415
   Ile Ile Leu Cys Leu Pro Asn Gly Leu Trp Ser Gly Ser Glu Ser Tyr
               420                 425                 430
   Cys Arg Val Arg Thr Cys Pro His Leu Arg Gln Pro Lys His Gly His
           435                 440                 445
   Ile Ser Cys Ser Thr Arg Glu Met Leu Tyr Lys Thr Thr Cys Leu Val
       450                 455                 460
   Ala Cys Asp Glu Gly Tyr Arg Leu Glu Gly Ser Asp Lys Leu Thr Cys
   465                 470                 475                 480
   Gln Gly Asn Ser Gln Trp Asp Gly Pro Glu Pro Arg Cys Val Glu Arg
                   485                 490                 495
   His Cys Ser Thr Phe Gln Met Pro Lys Asp Val Ile Ile Ser Pro His
               500                 505                 510
   Asn Cys Gly Lys Gln Pro Ala Lys Phe Gly Thr Ile Cys Tyr Val Ser
           515                 520                 525
   Cys Arg Gln Gly Phe Ile Leu Ser Gly Val Lys Glu Met Leu Arg Cys
       530                 535                 540
   Thr Thr Ser Gly Lys Trp Asn Val Gly Val Gln Ala Ala Val Cys Lys
   545                 550                 555                 560
   Asp Val Glu Ala Pro Gln Ile Asn Cys Pro Lys Asp Ile Glu Ala Lys
                   565                 570                 575
   Thr Leu Glu Gln Gln Asp Ser Ala Asn Val Thr Trp Gln Ile Pro Thr
               580                 585                 590
   Ala Lys Asp Asn Ser Gly Glu Lys Val Ser Val His Val His Pro Ala
           595                 600                 605
   Phe Thr Pro Pro Tyr Leu Phe Pro Ile Gly Asp Val Ala Ile Val Tyr
       610                 615                 620
   Thr Ala Thr Asp Leu Ser Gly Asn Gln Ala Ser Cys Ile Phe His Ile
   625                 630                 635                 640
   Lys Val Ile Asp Ala Glu Pro Pro Val Ile Asp Trp Cys Arg Ser Pro
                   645                 650                 655
   Pro Pro Val Gln Val Ser Glu Lys Val His Ala Ala Ser Trp Asp Glu
               660                 665                 670
   Pro Gln Phe Ser Asp Asn Ser Gly Ala Glu Leu Val Ile Thr Arg Ser
           675                 680                 685
   His Thr Gln Gly Asp Leu Phe Pro Gln Gly Glu Thr Ile Val Gln Tyr
       690                 695                 700
   Thr Ala Thr Asp Pro Ser Gly Asn Asn Arg Thr Cys Asp Ile His Ile
   705                 710                 715                 720
   Val Ile Lys Gly Ser Pro Cys Glu Ile Pro Phe Thr Pro Val Asn Gly
                   725                 730                 735
   Asp Phe Ile Cys Thr Pro Asp Asn Thr Gly Val Asn Cys Thr Leu Thr
               740                 745                 750
   Cys Leu Glu Gly Tyr Asp Phe Thr Glu Gly Ser Thr Asp Lys Tyr Tyr
           755                 760                 765
   Cys Ala Tyr Glu Asp Gly Val Trp Lys Pro Thr Tyr Thr Thr Glu Trp
       770                 775                 780
   Pro Asp Cys Ala Lys Lys Arg Phe Ala Asn His Gly Phe Lys Ser Phe
   785                 790                 795                 800
   Glu Met Phe Tyr Lys Ala Ala Arg Cys Asp Asp Thr Asp Leu Met Lys
                   805                 810                 815
   Lys Phe Ser Glu Ala Phe Glu Thr Thr Leu Gly Lys Met Val Pro Ser
               820                 825                 830
   Phe Cys Ser Asp Ala Glu Asp Ile Asp Cys Arg Leu Glu Glu Asn Leu
           835                 840                 845
   Thr Lys Lys Tyr Cys Leu Glu Tyr Asn Tyr Asp Tyr Glu Asn Gly Phe
   850 855 860
   Ala Ile Gly Pro Gly Gly Trp Gly Ala Ala Asn Arg Leu Asp Tyr Ser
   865 870 875 880
   Tyr Asp Asp Phe Leu Asp Thr Val Gln Glu Thr Ala Thr Ser Ile Gly
   885 890 895
   Asn Ala Lys Ser Ser Arg Ile Lys Arg Ser Ala Pro Leu Ser Asp Tyr
   900 905 910
   Lys Ile Lys Leu Ile Phe Asn Ile Thr Ala Ser Val Pro Leu Pro Asp
   915 920 925
   Glu Arg Asn Asp Thr Leu Glu Trp Glu Asn Gln Gln Arg Leu Leu Gln
   930 935 940
   Thr Leu Glu Thr Ile Thr Asn Lys Leu Lys Arg Thr Leu Asn Lys Asp
   945 950 955 960
   Pro Met Tyr Ser Phe Gln Leu Ala Ser Glu Ile Leu Ile Ala Asp Ser
   965 970 975
   Asn Ser Leu Glu Thr Lys Lys Ala Ser Pro Phe Cys Arg Pro Gly Ser
   980 985 990
   Val Leu Arg Gly Arg Met Cys Val Asn Cys Pro Leu Gly Thr Tyr Tyr
   995 1000 1005
   Asn Leu Glu His Phe Thr Cys Glu Ser Cys Arg Ile Gly Ser Tyr
   1010 1015 1020
   Gln Asp Glu Glu Gly Gln Leu Glu Cys Lys Leu Cys Pro Ser Gly
   1025 1030 1035
   Met Tyr Thr Glu Tyr Ile His Ser Arg Asn Ile Ser Asp Cys Lys
   1040 1045 1050
   Ala Gln Cys Lys Gln Gly Thr Tyr Ser Tyr Ser Gly Leu Glu Thr
   1055 1060 1065
   Cys Glu Ser Cys Pro Leu Gly Thr Tyr Gln Pro Lys Phe Gly Ser
   1070 1075 1080
   Arg Ser Cys Leu Ser Cys Pro Glu Asn Thr Ser Thr Val Lys Arg
   1085 1090 1095
   Gly Ala  Val Asn Ile Ser Ala  Cys Gly Val Pro Cys  Pro Glu Gly
       1100                 1105                 1110
   Lys Phe  Ser Arg Ser Gly Leu  Met Pro Cys His Pro  Cys Pro Arg
       1115                 1120                 1125
   Asp Tyr  Tyr Gln Pro Asn Ala  Gly Lys Ala Phe Cys  Leu Ala Cys
       1130                 1135                 1140
   Pro Phe  Tyr Gly Thr Thr Pro  Phe Ala Gly Ser Arg  Ser Ile Thr
       1145                 1150                 1155
   Glu Cys  Ser Ser Phe Ser Ser  Thr Phe Ser Ala Ala  Glu Glu Ser
       1160                 1165                 1170
   Val Val  Pro Pro Ala Ser Leu  Gly His Ile Lys Lys  Arg His Glu
       1175                 1180                 1185
   Ile Ser  Ser Gln Val Phe His  Glu Cys Phe Phe Asn  Pro Cys His
       1190                 1195                 1200
   Asn Ser  Gly Thr Cys Gln Gln  Leu Gly Arg Gly Tyr  Val Cys Leu
       1205                 1210                 1215
   Cys Pro  Leu Gly Tyr Thr Gly  Leu Lys Cys Glu Thr  Asp Ile Asp
       1220                 1225                 1230
   Glu Cys  Ser Pro Leu Pro Cys  Leu Asn Asn Gly Val  Cys Lys Asp
       1235                 1240                 1245
   Leu Val  Gly Glu Phe Ile Cys  Glu Cys Pro Ser Gly  Tyr Thr Gly
       1250                 1255                 1260
   Gln Arg  Cys Glu Glu Asn Ile  Asn Glu Cys Ser Ser  Ser Pro Cys
       1265                 1270                 1275
   Leu Asn  Lys Gly Ile Cys Val  Asp Gly Val Ala Gly  Tyr Arg Cys
       1280                 1285                 1290
   Thr Cys  Val Lys Gly Phe Val  Gly Leu His Cys Glu  Thr Glu Val
       1295                 1300                 1305
   Asn Glu  Cys Gln Ser Asn Pro  Cys Leu Asn Asn Ala  Val Cys Glu
       1310                 1315                 1320
   Asp Gln  Val Gly Gly Phe Leu  Cys Lys Cys Pro Pro  Gly Phe Leu
       1325                 1330                 1335
   Gly Thr Arg Cys Gly Lys Asn Val Asp Glu Cys Leu Ser Gln Pro
       1340                1345                1350
   Cys Lys Asn Gly Ala Thr Cys Lys Asp Gly Ala Asn Ser Phe Arg
       1355                1360                1365
   Cys Leu Cys Ala Ala Gly Phe Thr Gly Ser His Cys Glu Leu Asn
       1370                1375                1380
   Ile Asn Glu Cys Gln Ser Asn Pro Cys Arg Asn Gln Ala Thr Cys
       1385                1390                1395
   Val Asp Glu Leu Asn Ser Tyr Ser Cys Lys Cys Gln Pro Gly Phe
       1400                1405                1410
   Ser Gly Lys Arg Cys Glu Thr Glu Gln Ser Thr Gly Phe Asn Leu
       1415                1420                1425
   Asp Phe Glu Val Ser Gly Ile Tyr Gly Tyr Val Met Leu Asp Gly
       1430                1435                1440
   Met Leu Pro Ser Leu His Ala Leu Thr Cys Thr Phe Trp Met Lys
       1445                1450                1455
   Ser Ser Asp Asp Met Asn Tyr Gly Thr Pro Ile Ser Tyr Ala Val
       1460                1465                1470
   Asp Asn Gly Ser Asp Asn Thr Leu Leu Leu Thr Asp Tyr Asn Gly
       1475                1480                1485
   Trp Val Leu Tyr Val Asn Gly Arg Glu Lys Ile Thr Asn Cys Pro
       1490                1495                1500
   Ser Val Asn Asp Gly Arg Trp His His Ile Ala Ile Thr Trp Thr
       1505                1510                1515
   Ser Ala Asn Gly Ile Trp Lys Val Tyr Ile Asp Gly Lys Leu Ser
       1520                1525                1530
   Asp Gly Gly Ala Gly Leu Ser Val Gly Leu Pro Ile Pro Gly Gly
       1535                1540                1545
   Gly Ala Leu Val Leu Gly Gln Glu Gln Asp Lys Lys Gly Glu Gly
       1550                1555                1560
   Phe Ser Pro Ala Glu Ser Phe Val Gly Ser Ile Ser Gln Leu Asn
       1565                1570                1575
   Leu Trp Asp Tyr Val Leu Ser Pro Gln Gln Val Lys Ser Leu Ala
   1580 1585 1590
   Thr Ser Cys Pro Glu Glu Leu Ser Lys Gly Asn Val Leu Ala Trp
   1595 1600 1605
   Pro Asp Phe Leu Ser Gly Ile Val Gly Lys Val Lys Ile Asp Ser
   1610 1615 1620
   Lys Ser Ile Phe Cys Ser Asp Cys Pro Arg Leu Gly Gly Ser Val
   1625 1630 1635
   Pro His Leu Arg Thr Ala Ser Glu Asp Leu Lys Pro Gly Ser Lys
   1640 1645 1650
   Val Asn Leu Phe Cys Asp Pro Gly Phe Gln Leu Val Gly Asn Pro
   1655 1660 1665
   Val Gln Tyr Cys Leu Asn Gln Gly Gln Trp Thr Gln Pro Leu Pro
   1670 1675 1680
   His Cys Glu Arg Ile Ser Cys Gly Val Pro Pro Pro Leu Glu Asn
   1685 1690 1695
   Gly Phe His Ser Ala Asp Asp Phe Tyr Ala Gly Ser Thr Val Thr
   1700 1705 1710
   Tyr Gln Cys Asn Asn Gly Tyr Tyr Leu Leu Gly Asp Ser Arg Met
   1715 1720 1725
   Phe Cys Thr Asp Asn Gly Ser Trp Asn Gly Val Ser Pro Ser Cys
   1730 1735 1740
   Leu Asp Val Asp Glu Cys Ala Val Gly Ser Asp Cys Ser Glu His
   1745 1750 1755
   Ala Ser Cys Leu Asn Val Asp Gly Ser Tyr Ile Cys Ser Cys Val
   1760 1765 1770
   Pro Pro Tyr Thr Gly Asp Gly Lys Asn Cys Ala Glu Pro Ile Lys
   1775 1780 1785
   Cys Lys Ala Pro Gly Asn Pro Glu Asn Gly His Ser Ser Gly Glu
   1790 1795 1800
   Ile Tyr Thr Val Gly Ala Glu Val Thr Phe Ser Cys Gln Glu Gly
   1805 1810 1815
   Tyr Gln Leu Met Gly Val Thr Lys Ile Thr Cys Leu Glu Ser Gly
   1820 1825 1830
   Glu Trp Asn His Leu Ile Pro Tyr Cys Lys Ala Val Ser Cys Gly
   1835 1840 1845
   Lys Pro Ala Ile Pro Glu Asn Gly Cys Ile Glu Glu Leu Ala Phe
   1850 1855 1860
   Thr Phe Gly Ser Lys Val Thr Tyr Arg Cys Asn Lys Gly Tyr Thr
   1865 1870 1875
   Leu Ala Gly Asp Lys Glu Ser Ser Cys Leu Ala Asn Ser Ser Trp
   1880 1885 1890
   Ser His Ser Pro Pro Val Cys Glu Pro Val Lys Cys Ser Ser Pro
   1895 1900 1905
   Glu Asn Ile Asn Asn Gly Lys Tyr Ile Leu Ser Gly Leu Thr Tyr
   1910 1915 1920
   Leu Ser Thr Ala Ser Tyr Ser Cys Asp Thr Gly Tyr Ser Leu Gln
   1925 1930 1935
   Gly Pro Ser Ile Ile Glu Cys Thr Ala Ser Gly Ile Trp Asp Arg
   1940 1945 1950
   Ala Pro Pro Ala Cys His Leu Val Phe Cys Gly Glu Pro Pro Ala
   1955 1960 1965
   Ile Lys Asp Ala Val Ile Thr Gly Asn Asn Phe Thr Phe Arg Asn
   1970 1975 1980
   Thr Val Thr Tyr Thr Cys Lys Glu Gly Tyr Thr Leu Ala Gly Leu
   1985 1990 1995
   Asp Thr Ile Glu Cys Leu Ala Asp Gly Lys Trp Ser Arg Ser Asp
   2000 2005 2010
   Gln Gln Cys Leu Ala Val Ser Cys Asp Glu Pro Pro Ile Val Asp
   2015 2020 2025
   His Ala Ser Pro Glu Thr Ala His Arg Leu Phe Gly Asp Ile Ala
   2030 2035 2040
   Phe Tyr Tyr Cys Ser Asp Gly Tyr Ser Leu Ala Asp Asn Ser Gln
   2045 2050 2055
   Leu Leu Cys Asn Ala Gln Gly Lys Trp Val Pro Pro Glu Gly Gln
   2060 2065 2070
   Asp Met Pro Arg Cys Ile Ala His Phe Cys Glu Lys Pro Pro Ser
   2075 2080 2085
   Val Ser Tyr Ser Ile Leu Glu Ser Val Ser Lys Ala Lys Phe Ala
   2090 2095 2100
   Ala Gly Ser Val Val Ser Phe Lys Cys Met Glu Gly Phe Val Leu
   2105 2110 2115
   Asn Thr Ser Ala Lys Ile Glu Cys Met Arg Gly Gly Gln Trp Asn
   2120 2125 2130
   Pro Ser Pro Met Ser Ile Gln Cys Ile Pro Val Arg Cys Gly Glu
   2135 2140 2145
   Pro Pro Ser Ile Met Asn Gly Tyr Ala Ser Gly Ser Asn Tyr Ser
   2150 2155 2160
   Phe Gly Ala Met Val Ala Tyr Ser Cys Asn Lys Gly Phe Tyr Ile
   2165 2170 2175
   Lys Gly Glu Lys Lys Ser Thr Cys Glu Ala Thr Gly Gln Trp Ser
   2180 2185 2190
   Ser Pro Ile Pro Thr Cys His Pro Val Ser Cys Gly Glu Pro Pro
   2195 2200 2205
   Lys Val Glu Asn Gly Phe Leu Glu His Thr Thr Gly Arg Ile Phe
   2210 2215 2220
   Glu Ser Glu Val Arg Tyr Gln Cys Asn Pro Gly Tyr Lys Ser Val
   2225 2230 2235
   Gly Ser Pro Val Phe Val Cys Gln Ala Asn Arg His Trp His Ser
   2240 2245 2250
   Glu Ser Pro Leu Met Cys Val Pro Leu Asp Cys Gly Lys Pro Pro
   2255 2260 2265
   Pro Ile Gln Asn Gly Phe Met Lys Gly Glu Asn Phe Glu Val Gly
   2270 2275 2280
   Ser Lys Val Gln Phe Phe Cys Asn Glu Gly Tyr Glu Leu Val Gly
       2285                2290                2295
   Asp Ser Ser Trp Thr Cys Gln Lys Ser Gly Lys Trp Asn Lys Lys
       2300                2305                2310
   Ser Asn Pro Lys Cys Met Pro Ala Lys Cys Pro Glu Pro Pro Leu
       2315                2320                2325
   Leu Glu Asn Gln Leu Val Leu Lys Glu Leu Thr Thr Glu Val Gly
       2330                2335                2340
   Val Val Thr Phe Ser Cys Lys Glu Gly His Val Leu Gln Gly Pro
       2345                2350                2355
   Ser Val Leu Lys Cys Leu Pro Ser Gln Gln Trp Asn Asp Ser Phe
       2360                2365                2370
   Pro Val Cys Lys Ile Val Leu Cys Thr Pro Pro Pro Leu Ile Ser
       2375                2380                2385
   Phe Gly Val Pro Ile Pro Ser Ser Ala Leu His Phe Gly Ser Thr
       2390                2395                2400
   Val Lys Tyr Ser Cys Val Gly Gly Phe Phe Leu Arg Gly Asn Ser
       2405                2410                2415
   Thr Thr Leu Cys Gln Pro Asp Gly Thr Trp Ser Ser Pro Leu Pro
       2420                2425                2430
   Glu Cys Val Pro Val Glu Cys Pro Gln Pro Glu Glu Ile Pro Asn
       2435                2440                2445
   Gly Ile Ile Asp Val Gln Gly Leu Ala Tyr Leu Ser Thr Ala Leu
       2450                2455                2460
   Tyr Thr Cys Lys Pro Gly Phe Glu Leu Val Gly Asn Thr Thr Thr
       2465                2470                2475
   Leu Cys Gly Glu Asn Gly His Trp Leu Gly Gly Lys Pro Thr Cys
       2480                2485                2490
   Lys Ala Ile Glu Cys Leu Lys Pro Lys Glu Ile Leu Asn Gly Lys
       2495                2500                2505
   Phe Ser Tyr Thr Asp Leu His Tyr Gly Gln Thr Val Thr Tyr Ser
       2510                2515                2520
   Cys Asn  Arg Gly Phe Arg Leu  Glu Gly Pro Ser Ala  Leu Thr Cys
       2525                 2530                 2535
   Leu Glu  Thr Gly Asp Trp Asp  Val Asp Ala Pro Ser  Cys Asn Ala
       2540                 2545                 2550
   Ile His  Cys Asp Ser Pro Gln  Pro Ile Glu Asn Gly  Phe Val Glu
       2555                 2560                 2565
   Gly Ala  Asp Tyr Ser Tyr Gly  Ala Ile Ile Ile Tyr  Ser Cys Phe
       2570                 2575                 2580
   Pro Gly  Phe Gln Val Ala Gly  His Ala Met Gln Thr  Cys Glu Glu
       2585                 2590                 2595
   Ser Gly  Trp Ser Ser Ser Ile  Pro Thr Cys Met Pro  Ile Asp Cys
       2600                 2605                 2610
   Gly Leu  Pro Pro His Ile Asp  Phe Gly Asp Cys Thr  Lys Leu Lys
       2615                 2620                 2625
   Asp Asp  Gln Gly Tyr Phe Glu  Gln Glu Asp Asp Met  Met Glu Val
       2630                 2635                 2640
   Pro Tyr  Val Thr Pro His Pro  Pro Tyr His Leu Gly  Ala Val Ala
       2645                 2650                 2655
   Lys Thr  Trp Glu Asn Thr Lys  Glu Ser Pro Ala Thr  His Ser Ser
       2660                 2665                 2670
   Asn Phe  Leu Tyr Gly Thr Met  Val Ser Tyr Thr Cys  Asn Pro Gly
       2675                 2680                 2685
   Tyr Glu  Leu Leu Gly Asn Pro  Val Leu Ile Cys Gln  Glu Asp Gly
       2690                 2695                 2700
   Thr Trp  Asn Gly Ser Ala Pro  Ser Cys Ile Ser Ile  Glu Cys Asp
       2705                 2710                 2715
   Leu Pro  Thr Ala Pro Glu Asn  Gly Phe Leu Arg Phe  Thr Glu Thr
       2720                 2725                 2730
   Ser Met  Gly Ser Ala Val Gln  Tyr Ser Cys Lys Pro  Gly His Ile
       2735                 2740                 2745
   Leu Ala  Gly Ser Asp Leu Arg  Leu Cys Leu Glu Asn  Arg Lys Trp
   2750 2755 2760
   Ser Gly Ala Ser Pro Arg Cys Glu Ala Ile Ser Cys Lys Lys Pro
   2765 2770 2775
   Asn Pro Val Met Asn Gly Ser Ile Lys Gly Ser Asn Tyr Thr Tyr
   2780 2785 2790
   Leu Ser Thr Leu Tyr Tyr Glu Cys Asp Pro Gly Tyr Val Leu Asn
   2795 2800 2805
   Gly Thr Glu Arg Arg Thr Cys Gln Asp Asp Lys Asn Trp Asp Glu
   2810 2815 2820
   Asp Glu Pro Ile Cys Ile Pro Val Asp Cys Ser Ser Pro Pro Val
   2825 2830 2835
   Ser Ala Asn Gly Gln Val Arg Gly Asp Glu Tyr Thr Phe Gln Lys
   2840 2845 2850
   Glu Ile Glu Tyr Thr Cys Asn Glu Gly Phe Leu Leu Glu Gly Ala
   2855 2860 2865
   Arg Ser Arg Val Cys Leu Ala Asn Gly Ser Trp Ser Gly Ala Thr
   2870 2875 2880
   Pro Asp Cys Val Pro Val Arg Cys Ala Thr Pro Pro Gln Leu Ala
   2885 2890 2895
   Asn Gly Val Thr Glu Gly Leu Asp Tyr Gly Phe Met Lys Glu Val
   2900 2905 2910
   Thr Phe His Cys His Glu Gly Tyr Ile Leu His Gly Ala Pro Lys
   2915 2920 2925
   Leu Thr Cys Gln Ser Asp Gly Asn Trp Asp Ala Glu Ile Pro Leu
   2930 2935 2940
   Cys Lys Pro Val Asn Cys Gly Pro Pro Glu Asp Leu Ala His Gly
   2945 2950 2955
   Phe Pro Asn Gly Phe Ser Phe Ile His Gly Gly His Ile Gln Tyr
   2960 2965 2970
   Gln Cys Phe Pro Gly Tyr Lys Leu His Gly Asn Ser Ser Arg Arg
   2975 2980 2985
   Cys Leu Ser Asn Gly Ser Trp Ser Gly Ser Ser Pro Ser Cys Leu
   2990 2995 3000
   Pro Cys Arg Cys Ser Thr Pro Val Ile Glu Tyr Gly Thr Val Asn
   3005 3010 3015
   Gly Thr Asp Phe Asp Cys Gly Lys Ala Ala Arg Ile Gln Cys Phe
   3020 3025 3030
   Lys Gly Phe Lys Leu Leu Gly Leu Ser Glu Ile Thr Cys Glu Ala
   3035 3040 3045
   Asp Gly Gln Trp Ser Ser Gly Phe Pro His Cys Glu His Thr Ser
   3050 3055 3060
   Cys Gly Ser Leu Pro Met Ile Pro Asn Ala Phe Ile Ser Glu Thr
   3065 3070 3075
   Ser Ser Trp Lys Glu Asn Val Ile Thr Tyr Ser Cys Arg Ser Gly
   3080 3085 3090
   Tyr Val Ile Gln Gly Ser Ser Asp Leu Ile Cys Thr Glu Lys Gly
   3095 3100 3105
   Val Trp Ser Gln Pro Tyr Pro Val Cys Glu Pro Leu Ser Cys Gly
   3110 3115 3120
   Ser Pro Pro Ser Val Ala Asn Ala Val Ala Thr Gly Glu Ala His
   3125 3130 3135
   Thr Tyr Glu Ser Glu Val Lys Leu Arg Cys Leu Glu Gly Tyr Thr
   3140 3145 3150
   Met Asp Thr Asp Thr Asp Thr Phe Thr Cys Gln Lys Asp Gly Arg
   3155 3160 3165
   Trp Phe Pro Glu Arg Ile Ser Cys Ser Pro Lys Lys Cys Pro Leu
   3170 3175 3180
   Pro Glu Asn Ile Thr His Ile Leu Val His Gly Asp Asp Phe Ser
   3185 3190 3195
   Val Asn Arg Gln Val Ser Val Ser Cys Ala Glu Gly Tyr Thr Phe
   3200 3205 3210
   Glu Gly Val Asn Ile Ser Val Cys Gln Leu Asp Gly Thr Trp Glu
   3215 3220 3225
   Pro Pro Phe Ser Asp Glu Ser Cys Ser Pro Val Ser Cys Gly Lys
       3230                3235                3240
   Pro Glu Ser Pro Glu His Gly Phe Val Val Gly Ser Lys Tyr Thr
       3245                3250                3255
   Phe Glu Ser Thr Ile Ile Tyr Gln Cys Glu Pro Gly Tyr Glu Leu
       3260                3265                3270
   Glu Gly Asn Arg Glu Arg Val Cys Gln Glu Asn Arg Gln Trp Ser
       3275                3280                3285
   Gly Gly Val Ala Ile Cys Lys Glu Thr Arg Cys Glu Thr Pro Leu
       3290                3295                3300
   Glu Phe Leu Asn Gly Lys Ala Asp Ile Glu Asn Arg Thr Thr Gly
       3305                3310                3315
   Pro Asn Val Val Tyr Ser Cys Asn Arg Gly Tyr Ser Leu Glu Gly
       3320                3325                3330
   Pro Ser Glu Ala His Cys Thr Glu Asn Gly Thr Trp Ser His Pro
       3335                3340                3345
   Val Pro Leu Cys Lys Pro Asn Pro Cys Pro Val Pro Phe Val Ile
       3350                3355                3360
   Pro Glu Asn Ala Leu Leu Ser Glu Lys Glu Phe Tyr Val Asp Gln
       3365                3370                3375
   Asn Val Ser Ile Lys Cys Arg Glu Gly Phe Leu Leu Gln Gly His
       3380                3385                3390
   Gly Ile Ile Thr Cys Asn Pro Asp Glu Thr Trp Thr Gln Thr Ser
       3395                3400                3405
   Ala Lys Cys Glu Lys Ile Ser Cys Gly Pro Pro Ala His Val Glu
       3410                3415                3420
   Asn Ala Ile Ala Arg Gly Val His Tyr Gln Tyr Gly Asp Met Ile
       3425                3430                3435
   Thr Tyr Ser Cys Tyr Ser Gly Tyr Met Leu Glu Gly Phe Leu Arg
       3440                3445                3450
   Ser Val Cys Leu Glu Asn Gly Thr Trp Thr Ser Pro Pro Ile Cys
       3455                3460                3465
   Arg Ala  Val Cys Arg Phe Pro  Cys Gln Asn Gly Gly  Ile Cys Gln
       3470                 3475                 3480
   Arg Pro  Asn Ala Cys Ser Cys  Pro Glu Gly Trp Met  Gly Arg Leu
       3485                 3490                 3495
   Cys Glu  Glu Pro Ile Cys Ile  Leu Pro Cys Leu Asn  Gly Gly Arg
       3500                 3505                 3510
   Cys Val  Ala Pro Tyr Gln Cys  Asp Cys Pro Pro Gly  Trp Thr Gly
       3515                 3520                 3525
   Ser Arg  Cys His Thr Ala Val  Cys Gln Ser Pro Cys  Leu Asn Gly
       3530                 3535                 3540
   Gly Lys  Cys Val Arg Pro Asn  Arg Cys His Cys Leu  Ser Ser Trp
       3545                 3550                 3555
   Thr Gly  His Asn Cys Ser Arg  Lys Arg Arg Thr Gly  Phe
       3560                 3565                 3570
<210> 5
   <211> 12356
   <212> DNA
   <213> Homo sapiens
<400> 5
   agggagagaa ggagggaagc gggagatttt tcttgactgc cccctttcct tcaaacattt      60
   tataggcttc agggagagag aggaggagga gagagggaag aaaaaaagag gagagcgaga     120
   ggggtagaga gcgcgcgccg ttccctccgg agttcccgag ctgctgagga gtctggattg     180
   tgtctgtccc cagtgtcaga tgaaagggcg ctgaggctct tggccgctgc cccgcgccca     240
   gctccgcgca cgcccctctg cgagtccggc cgcccagcgc ctcttcccgc ccgagccgcc     300
   gcctgcgctc cggggcagcc gctctgtctc cagcgcgatg tggcctcgcc tggccttttg     360
   ttgctggggt ctggcgctcg tttcgggctg ggcgaccttt cagcagatgt ccccgtcgcg     420
   caatttcagc ttccgcctct tccccgagac cgcgcccggg gcccccggga gtatccccgc     480
   gccgcccgct cctggcgacg aagcggcggg gagcagagtg gagcggctgg gccaggcgtt     540
   ccggcgacgc gtgcggctgc tgcgggagct cagcgagcgc ctggagcttg tcttcctggt     600
   ggatgattcg tccagcgtgg gcgaagtcaa cttccgcagc gagctcatgt tcgtccgcaa     660
   gctgctgtcc gacttccccg tggtgcccac ggccacgcgc gtggccatcg tgaccttctc     720
   gtccaagaac tacgtggtgc cgcgcgtcga ttacatctcc acccgccgcg cgcgccagca     780
   caagtgcgcg ctgctcctcc aagagatccc tgccatctcc taccgaggtg gcggcaccta     840
   caccaagggc gccttccagc aagccgcgca aattcttctt catgctagag aaaactcaac 900
   aaaagttgta tttctcatca ctgatggata ttccaatggg ggagacccta gaccaattgc 960
   agcgtcactg cgagattcag gagtggagat cttcactttt ggcatatggc aagggaacat 1020
   tcgagagctg aatgacatgg cttccacccc aaaggaggag cactgttacc tgctacacag 1080
   ttttgaagaa tttgaggctt tagctcgccg ggcattgcat gaagatctac cttctgggag 1140
   ttttattcaa gatgatatgg tccactgctc atatctttgt gatgaaggca aggactgctg 1200
   tgaccgaatg ggaagctgca aatgtgggac acacacaggc cattttgagt gcatctgtga 1260
   aaaggggtat tacgggaaag gtctgcagta tgaatgcaca gcttgcccat cggggacata 1320
   caaacctgaa ggctcaccag gaggaatcag cagttgcatt ccatgtcctg atgaaaatca 1380
   cacctctcca cctggaagca catcccctga agactgtgtc tgcagagagg gatacagggc 1440
   atctggccag acctgtgaac ttgtccactg ccctgccctg aagcctcccg aaaatggtta 1500
   ctttatccaa aacacttgca acaaccactt caatgcagcc tgtggggtcc gatgtcaccc 1560
   tggatttgat cttgtgggaa gcagcatcat cttatgtcta cccaatggtt tgtggtccgg 1620
   ttcagagagc tactgcagag taagaacatg tcctcatctc cgccagccga aacatggcca 1680
   catcagctgt tctacaaggg aaatgttata taagacaaca tgtttggttg cctgtgatga 1740
   agggtacaga ctagaaggca gtgataagct tacttgtcaa ggaaacagcc agtgggatgg 1800
   gccagaaccc cggtgtgtgg agcgccactg ttccaccttt cagatgccca aagatgtcat 1860
   catatccccc cacaactgtg gcaagcagcc agccaaattt gggacgatct gctatgtaag 1920
   ttgccgccaa gggttcattt tatctggagt caaagaaatg ctgagatgta ccacttctgg 1980
   aaaatggaat gtcggagttc aggcagctgt gtgtaaagac gtggaggctc ctcaaatcaa 2040
   ctgtcctaag gacatagagg ctaagactct ggaacagcaa gattctgcca atgttacctg 2100
   gcagattcca acagctaaag acaactctgg tgaaaaggtg tcagtccacg ttcatccagc 2160
   tttcacccca ccttaccttt tcccaattgg agatgttgct atcgtataca cggcaactga 2220
   cctatccggc aaccaggcca gctgcatttt ccatatcaag gttattgatg cagaaccacc 2280
   tgtcatagac tggtgcagat ctccacctcc cgtccaggtc tcggagaagg tacatgccgc 2340
   aagctgggat gagcctcagt tctcagacaa ctcaggggct gaattggtca ttaccagaag 2400
   tcatacacaa ggagaccttt tccctcaagg ggagactata gtacagtata cagccactga 2460
   cccctcaggc aataacagga catgtgatat ccatattgtc ataaaaggtt ctccctgtga 2520
   aattccattc acacctgtaa atggggattt tatatgcact ccagataata ctggagtcaa 2580
   ctgtacatta acttgcttgg agggctatga tttcacagaa gggtctactg acaagtatta 2640
   ttgtgcttat gaagatggcg tctggaaacc aacatatacc actgaatggc cagactgtgc 2700
   caaaaaacgt tttgcaaacc acgggttcaa gtcctttgag atgttctaca aagcagctcg 2760
   ttgtgatgac acagatctga tgaagaagtt ttctgaagca tttgagacga ccctgggaaa 2820
   aatggtccca tcattttgta gtgatgcaga ggacattgac tgcagactgg aggagaacct 2880
   gaccaaaaaa tattgcctag aatataatta tgactatgaa aatggctttg caattggacc 2940
   aggtggctgg ggtgcagcta ataggctgga ttactcttac gatgacttcc tggacactgt 3000
   gcaagaaaca gccacaagca tcggcaatgc caagtcctca cggattaaaa gaagtgcccc 3060
   attatctgac tataaaatta agttaatttt taacatcaca gctagtgtgc cattacccga 3120
   tgaaagaaat gatacccttg aatgggaaaa tcagcaacga ctccttcaga cattggaaac 3180
   tatcacaaat aaactgaaaa ggactctcaa caaagacccc atgtattcct ttcagcttgc 3240
   atcagaaata cttatagccg acagcaattc attagaaaca aaaaaggctt cccccttctg 3300
   cagaccaggc tcagtgctga gagggcgtat gtgtgtcaat tgccctttgg gaacctatta 3360
   taatctggaa catttcacct gtgaaagctg ccggatcgga tcctatcaag atgaagaagg 3420
   gcaacttgag tgcaagcttt gcccctctgg gatgtacacg gaatatatcc attcaagaaa 3480
   catctctgat tgtaaagctc agtgtaaaca aggcacctac tcatacagtg gacttgagac 3540
   ttgtgaatcg tgtccactgg gcacttatca gccaaaattt ggttcccgga gctgcctctc 3600
   gtgtccagaa aacacctcaa ctgtgaaaag aggagccgtg aacatttctg catgtggagt 3660
   tccttgtcca gaaggaaaat tctcgcgttc tgggttaatg ccctgtcacc catgtcctcg 3720
   tgactattac caacctaatg cagggaaggc cttctgcctg gcctgtccct tttatggaac 3780
   taccccattc gctggttcca gatccatcac agaatgttca agttttagtt caactttctc 3840
   agcggcagag gaaagtgtgg tgccccctgc ctctcttgga catattaaaa agaggcatga 3900
   aatcagcagt caggttttcc atgaatgctt ctttaaccct tgccacaata gtggaacctg 3960
   ccagcaactt gggcgtggtt atgtttgtct ctgtccactt ggatatacag gcttaaagtg 4020
   tgaaacagac atcgatgagt gcagcccact gccttgcctc aacaatggag tttgtaaaga 4080
   cctagttggg gaattcattt gtgagtgccc atcaggttac acaggtcagc ggtgtgaaga 4140
   aaatataaat gagtgtagct ccagtccttg tttaaataaa ggaatctgtg ttgatggtgt 4200
   ggctggctat cgttgcacat gtgtgaaagg atttgtaggc ctgcattgtg aaacagaagt 4260
   caatgaatgc cagtcaaacc catgcttaaa taatgcagtc tgtgaagacc aggttggggg 4320
   attcttgtgc aaatgcccac ctggattttt gggtacccga tgtggaaaga acgtcgatga 4380
   gtgtctcagt cagccatgca aaaatggagc tacctgtaaa gacggtgcca atagcttcag 4440
   atgcctgtgt gcagctggct tcacaggatc acactgtgaa ttgaacatca atgaatgtca 4500
   gtctaatcca tgtagaaatc aggccacctg tgtggatgaa ttaaattcat acagttgtaa 4560
   atgtcagcca ggattttcag gcaaaaggtg tgaaacagaa cagtctacag gctttaacct 4620
   ggattttgaa gtttctggca tctatggata tgtcatgcta gatggcatgc tcccatctct 4680
   ccatgctcta acctgtacct tctggatgaa atcctctgac gacatgaact atggaacacc 4740
   aatctcctat gcagttgata acggcagcga caataccttg ctcctgactg attataacgg 4800
   ctgggttctt tatgtgaatg gcagggaaaa gataacaaac tgtccctcgg tgaatgatgg 4860
   cagatggcat catattgcaa tcacttggac aagtgccaat ggcatctgga aagtctatat 4920
   cgatgggaaa ttatctgacg gtggtgctgg cctctctgtt ggtttgccca tacctggtgg 4980
   tggtgcgtta gttctggggc aagagcaaga caaaaaagga gagggattca gcccagctga 5040
   gtcttttgtg ggctccataa gccagctcaa cctctgggac tatgtcctgt ctccacagca 5100
   ggtgaagtca ctggctacct cctgcccaga ggaactcagt aaaggaaacg tgttagcatg 5160
   gcctgatttc ttgtcaggaa ttgtggggaa agtgaagatc gattctaaga gcatattttg 5220
   ttctgattgc ccacgcttag gagggtcagt gcctcatctg agaactgcat ctgaagattt 5280
   aaagccaggt tccaaagtca atctgttctg tgatccaggc ttccagctgg tcgggaaccc 5340
   tgtgcagtac tgtctgaatc aaggacagtg gacacaacca cttcctcact gtgaacgcat 5400
   tagctgtggg gtgccacctc ctttggagaa tggcttccat tcagccgatg acttctatgc 5460
   tggcagcaca gtaacctacc agtgcaacaa tggctactat ctattgggtg actcaaggat 5520
   gttctgtaca gataatggga gctggaacgg cgtttcacca tcctgccttg atgtcgatga 5580
   gtgtgcagtt ggatcagatt gtagtgagca tgcttcttgc ctgaacgtag atggatccta 5640
   catatgttca tgtgtcccac cgtacacagg agatgggaaa aactgtgcag aacctataaa 5700
   atgtaaggct ccaggaaatc cggaaaatgg ccactcctca ggtgagattt atacagtagg 5760
   tgccgaagtc acattttcgt gtcaggaagg ataccagttg atgggagtaa ccaaaatcac 5820
   atgtttggag tctggagaat ggaatcatct aataccatat tgtaaagctg tttcatgtgg 5880
   taaaccggct attccagaaa atggttgcat tgaggagtta gcatttactt ttggcagcaa 5940
   agtgacatat aggtgtaata aaggatatac tctggccggt gataaagaat catcctgtct 6000
   tgctaacagt tcttggagtc attcccctcc tgtgtgtgaa ccagtgaagt gttctagtcc 6060
   ggaaaatata aataatggaa aatatatttt gagtgggctt acctaccttt ctactgcatc 6120
   atattcatgc gatacaggat acagcttaca gggcccttcc attattgaat gcacggcttc 6180
   tggcatctgg gacagagcgc cacctgcctg tcacctcgtc ttctgtggag aaccacctgc 6240
   catcaaagat gctgtcatta cggggaataa cttcactttc aggaacaccg tcacttacac 6300
   ttgcaaagaa ggctatactc ttgctggtct tgacaccatt gaatgcctgg ccgacggcaa 6360
   gtggagtaga agtgaccagc agtgcctggc tgtctcctgt gatgagccac ccattgtgga 6420
   ccacgcctct ccagagactg cccatcggct ctttggagac attgcattct actactgctc 6480
   tgatggttac agcctagcag acaattccca gcttctctgc aatgcccagg gcaagtgggt 6540
   acccccagaa ggtcaagaca tgccccgttg tatagctcat ttctgtgaaa aacctccatc 6600
   ggtttcctat agcatcttgg aatctgtgag caaagcaaaa tttgcagctg gctcagttgt 6660
   gagctttaaa tgcatggaag gctttgtact gaacacctca gcaaagattg aatgtatgag 6720
   aggtgggcag tggaaccctt cccccatgtc catccagtgc atccctgtgc ggtgtggaga 6780
   gccaccaagc atcatgaatg gctatgcaag tggatcaaac tacagttttg gagccatggt 6840
   ggcttacagc tgcaacaagg ggttctacat caaaggggaa aagaagagca cctgcgaagc 6900
   cacagggcag tggagtagtc ctataccgac gtgccacccg gtatcttgtg gtgaaccacc 6960
   taaggttgag aatggctttc tggagcatac aactggcagg atctttgaga gtgaagtgag 7020
   gtatcagtgt aacccgggct ataagtcagt cggaagtcct gtatttgtct gccaagccaa 7080
   tcgccactgg cacagtgaat cccctctgat gtgtgttcct ctcgactgtg gaaaacctcc 7140
   cccgatccag aatggcttca tgaaaggaga aaactttgaa gtagggtcca aggttcagtt 7200
   tttctgtaat gagggttatg agcttgttgg tgacagttct tggacatgtc agaaatctgg 7260
   caaatggaat aagaagtcaa atccaaagtg catgcctgcc aagtgcccag agccgcccct 7320
   cttggaaaac cagctagtat taaaggagtt gaccaccgag gtaggagttg tgacattttc 7380
   ctgtaaagaa gggcatgtcc tgcaaggccc ctctgtcctg aaatgcttgc catcccagca 7440
   atggaatgac tctttccctg tttgtaagat tgttctttgt accccacctc ccctaatttc 7500
   ctttggtgtc cccattcctt cttctgctct tcattttgga agtactgtca agtattcttg 7560
   tgtaggtggg tttttcctaa gaggaaattc taccaccctc tgccaacctg atggcacctg 7620
   gagctctcca ctgccagaat gtgttccagt agaatgtccc caacctgagg aaatccccaa 7680
   tggaatcatt gatgtgcaag gccttgccta tctcagcaca gctctctata cctgcaagcc 7740
   aggctttgaa ttggtgggaa atactaccac cctttgtgga gaaaatggtc actggcttgg 7800
   aggaaaacca acatgtaaag ccattgagtg cctgaaaccc aaggagattt tgaatggcaa 7860
   attctcttac acggacctac actatggaca gaccgttacc tactcttgca accgaggctt 7920
   tcggctcgaa ggtcccagtg ccttgacctg tttagagaca ggtgattggg atgtagatgc 7980
   cccatcttgc aatgccatcc actgtgattc cccacaaccc attgaaaatg gttttgtaga 8040
   aggtgcagat tacagctatg gtgccataat catctacagt tgcttccctg ggtttcaggt 8100
   ggctggtcat gccatgcaga cctgtgaaga gtcaggatgg tcaagttcca tcccaacatg 8160
   tatgccaata gactgtggcc tccctcctca tatagatttt ggagactgta ctaaactcaa 8220
   agatgaccag ggatattttg agcaagaaga cgacatgatg gaagttccat atgtgactcc 8280
   tcaccctcct tatcatttgg gagcagtggc taaaacctgg gaaaatacaa aggagtctcc 8340
   tgctacacat tcatcaaact ttctgtatgg taccatggtt tcatacacct gtaatccagg 8400
   atatgaactt ctggggaacc ctgtgctgat ctgccaggaa gatggaactt ggaatggcag   8460
   tgcaccatcc tgcatttcaa ttgaatgtga cttgcctact gctcctgaaa atggcttttt   8520
   gcgttttaca gagactagca tgggaagtgc tgtgcagtat agctgtaaac ctggacacat   8580
   tctagcaggc tctgacttaa ggctttgtct agagaataga aagtggagtg gtgcctcccc   8640
   acgctgtgaa gccatttcat gcaaaaagcc aaatccagtc atgaatggat ccatcaaagg   8700
   aagcaactac acatacctga gcacgttgta ctatgagtgt gaccccggat atgtgctgaa   8760
   tggcactgag aggagaacat gccaggatga caaaaactgg gatgaggatg agcccatttg   8820
   cattcctgtg gactgcagtt cacccccagt ctcagccaat ggccaggtga gaggagacga   8880
   gtacacattc caaaaagaga ttgaatacac ttgcaatgaa gggttcttgc ttgagggagc   8940
   caggagtcgg gtttgtcttg ccaatggaag ttggagtgga gccactcccg actgtgtgcc   9000
   tgtcagatgt gccaccccgc cacaactggc caatggggtg acggaaggcc tggactatgg   9060
   cttcatgaag gaagtaacat tccactgtca cgagggctac atcttgcacg gtgctccaaa   9120
   actcacctgt cagtcagatg gcaactggga tgcagagatt cctctctgta aaccagtcaa   9180
   ctgtggacct cctgaagatc ttgcccatgg tttccctaat ggtttttcct ttattcatgg   9240
   gggccatata cagtatcagt gctttcctgg ttataagctc catggaaatt catcaagaag   9300
   gtgcctctcc aatggctcct ggagtggcag ctcaccttcc tgcctgcctt gcagatgttc   9360
   cacaccagta attgaatatg gaactgtcaa tgggacagat tttgactgtg gaaaggcagc   9420
   ccggattcag tgcttcaaag gcttcaagct cctaggactt tctgaaatca cctgtgaagc   9480
   cgatggccag tggagctctg ggttccccca ctgtgaacac acttcttgtg gttctcttcc   9540
   aatgatacca aatgcgttca tcagtgagac cagctcttgg aaggaaaatg tgataactta   9600
   cagctgcagg tctggatatg tcatacaagg cagttcagat ctgatttgta cagagaaagg   9660
   ggtatggagc cagccttatc cagtctgtga gcccttgtcc tgtgggtccc caccgtctgt   9720
   cgccaatgca gtggcaactg gagaggcaca cacctatgaa agtgaagtga aactcagatg   9780
   tctggaaggt tatacgatgg atacagatac agatacattc acctgtcaga aagatggtcg   9840
   ctggttccct gagagaatct cctgcagtcc taaaaaatgt cctctcccgg aaaacataac   9900
   acatatactt gtacatgggg acgatttcag tgtgaatagg caagtttctg tgtcatgtgc   9960
   agaagggtat acctttgagg gagttaacat atcagtatgt cagcttgatg gaacctggga  10020
   gccaccattc tccgatgaat cttgcagtcc agtttcttgt gggaaacctg aaagtccaga  10080
   acatggattt gtggttggca gtaaatacac ctttgaaagc acaattattt atcagtgtga  10140
   gcctggctat gaactagagg ggaacaggga acgtgtctgc caggagaaca gacagtggag  10200
   tggaggggtg gcaatatgca aagagaccag gtgtgaaact ccacttgaat ttctcaatgg  10260
   gaaagctgac attgaaaaca ggacgactgg acccaacgtg gtatattcct gcaacagagg 10320
   ctacagtctt gaagggccat ctgaggcaca ctgcacagaa aatggaacct ggagccaccc 10380
   agtccctctc tgcaaaccaa atccatgccc tgttcctttt gtgattcccg agaatgctct 10440
   gctgtctgaa aaggagtttt atgttgatca gaatgtgtcc atcaaatgta gggaaggttt 10500
   tctgctgcag ggccacggca tcattacctg caaccccgac gagacgtgga cacagacaag 10560
   cgccaaatgt gaaaaaatct catgtggtcc accagctcac gtagaaaatg caattgctcg 10620
   aggcgtacat tatcaatatg gagacatgat cacctactca tgttacagtg gatacatgtt 10680
   ggagggtttc ctgaggagtg tttgtttaga aaatggaaca tggacatcac ctcctatttg 10740
   cagagctgtc tgtcgatttc catgtcagaa tgggggcatc tgccaacgcc caaatgcttg 10800
   ttcctgtcca gagggctgga tggggcgcct ctgtgaagaa ccaatctgca ttcttccctg 10860
   tctgaacgga ggtcgctgtg tggcccctta ccagtgtgac tgcccgcctg gctggacggg 10920
   gtctcgctgt catacagctg tttgccagtc tccctgctta aatggtggaa aatgtgtaag 10980
   accaaaccga tgtcactgtc tttcttcttg gacgggacat aactgttcca ggaaaaggag 11040
   gactgggttt taaccactgc acgaccatct ggctctccca aaagcaggat catctctcct 11100
   cggtagtgcc tgggcatcct ggaacttatg caaagaaagt ccaacatggt gctgggtctt 11160
   gtttagtaaa cttgttactt ggggttactt tttttatttt gtgatatatt ttgttattcc 11220
   ttgtgacata ctttcttaca tgtttccatt tttaaatatg cctgtatttt ctatataaaa 11280
   attatattaa atagatgctg ctctaccctc acaaaatgta catattctgc tgtctattgg 11340
   gaaagttcct ggtacacatt tttattcagt tacttaaaat gatttttcca ttaaagtata 11400
   ttttgctact aaataatatt ttgctggata gtaccatttt atgaggtggc caagggattc 11460
   atagagagga ctcatgatct ttcatctgtg ccactggcac cacataggag accccttaca 11520
   ataaaggaga cccctttgtt ctggttccat cttctataca atatgtgtaa taataaaagc 11580
   tttttctttc atttaatcaa tcagagagta actgttggac ctggtactct ccaccacata 11640
   attatgtcct ggtacttagg gtagcctggc taattctttt cacaacattt gaaaagccgt 11700
   aggcattaac ttcatggatg tggagaaaca gcataatgac cttggttcac aatgaaagtg 11760
   ctagccttct ttctcgtggc tggaataaga aatgcaagaa ctggaagatt aacaaagagc 11820
   tgatacccgg actggtaatc ccacaactca ctattttggg aggaagggga agacagggaa 11880
   agagaaggaa aaaggcaaat gggaaacatt ccccagagct gtgctcctgg tctcacctgc 11940
   aaaaaagaat tgcagccacc taagagaaag ctgagctgcc cctcagtcct cactgtcacc 12000
   tacctctatc acaacaattg taccaagatg aagccttccc cagacttcag aaaataaagt 12060
   caaaccctag attttgtttt aaaataggaa actcagaatc aacttgcctc catcctctgg 12120
   gaaaactgct cccacacagg ccttggagtg tgttgtagca ctgtggagga atgcagaaag 12180
   gatgaaagag atcttgattc tcctagtggt tctcttcact accgtaggca tccctcagcc  12240
   attgactcct ccttctttct cttgacattc actttcttgg ccagtcttac atgcttatga  12300
   gtctactttc caataaattt actcatagtc cattaacttc caaaaaaaaa aaaaaa      12356

## Description

### TECHNICAL FIELD

The present invention relates to a novel ligand for integrin α9β1 and use thereof.

### BACKGROUND ART

The extracellular matrix (hereinafter referred to as "ECM") is a structure surrounding cells and plays an essential role as cells' immediate microenvironment in maintaining cellular survival and controlling cellular proliferation and differentiation. Integrins are major ECM receptors on the cell surface and have a number of isoforms with different subunit compositions. In humans, 24 kinds of integrins exist and 18 kinds of them function as ECM receptors. Most of these integrins have a β1 chain and, depending on the type of α chain, are roughly classified into a "collagen-binding subgroup (α1β1, α2β1, etc.)," a "laminin-binding subgroup (α3β1, α6β1, etc.)," and an "Arg-Gly-Asp (RGD) sequence-binding subgroup (α5β1, α8β1, etc.)." However, as for some integrins such as α9β1, their true ligands still remain unidentified.

Integrin α9β1 is expressed in epithelial cells, smooth muscle cells, vascular endothelial cells, neutrophils, etc., and reportedly involved in angiogenesis, lymphangiogenesis, wound healing and autoimmune arthritis. In addition, integrin α9β1 is expressed in corneal stem cells and hematopoietic stem cells, and may be involved in maintaining the functions of these tissue stem cells. The so far reported ligands for integrin α9β1 include tenascin-C, osteopontin and cellular fibronectin (these are ECM proteins); VCAM-1 (vascular cell adhesion molecule 1) and ADAM protease (these are membrane proteins); and VEGF (vascular endothelial growth factor) and NGF (nerve growth factor) (these are growth factors) (see Non Patent Literature 1 to 7). However, the binding affinities of these proteins for integrin α9β1 are markedly lower than those between laminin-binding or RGD-binding integrins and their respective ligands, and the measurement is often based on cell adhesion activity, not direct integrin α9β1-binding activity. In addition, regarding tenascin-C and osteopontin as typical ECM ligands for integrin α9β1, the ligand activities can be detected only in their specific fragments, not in their full-length proteins. Therefore, it still remains uncertain whether they function as physiological ligands. For the reasons above, it has been thought that true ECM ligands for integrin α9β1 are other than those proteins.

### CITATION LIST

### Non Patent Literature

Non Patent Literature 1:
   Yokosaki, Y., Palmer, E. L., Prieto, A. L., Crossin, K. L., Bourdon, M. A., Pytela, R., and Sheppard, D. (1994) J. Biol. Chem. 269, 26691-26696
Non Patent Literature 2:
   Smith, L. L., Cheung, H. K., Ling, L. E., Chen, J., Sheppard, D., Pytela, R. , and Giachelli, C. M. (1996) J. Biol. Chem. 271, 28485-28491
Non Patent Literature 3:
   Liao, Y. F., Gotwals, P. J., Koteliansky, V. E., Sheppard, D., and Van De Water, L. (2002) J. Biol. Chem. 277, 14467-14474 Non Patent Literature 4:
   Eto, K., Huet, C., Tarui, T., Kupriyanov, S., Liu, H. Z., Puzon-McLaughlin, W. , Zhang, X. P., Sheppard, D., Engvall, E., and Takada, Y. (2002) J. Biol. Chem. 277, 17804-17810
Non Patent Literature 5:
   Taooka, Y., Chen, J., Yednock, T., and Sheppard, D. (1999) J. Cell Biol. 145, 413-420
Non Patent Literature 6:
   Vlahakis, N. E., Young, B. A., Atakilit, A., and Sheppard, D. (2005) J. Biol. Chem. 280, 4544-4552
Non Patent Literature 7:
   Staniszewska, I., Sariyer, I. K., Lecht, S., Brown, M. C., Walsh, E. M., Tuszynski, G. P., Safak, M., Lazarovici, P., and Marcinkiewicz, C. (2008) J. Cell Sci. 121, 504-513

### SUMMARY OF INVENTION

### TECHNICAL PROBLEM

An object of the present invention is to provide a novel ligand for integrin α9β1 which has a higher binding affinity than those of tenascin-C and osteopontin, which are known ligands for integrin α9β1; and usage thereof.

### SOLUTION TO PROBLEM

The present invention includes the following to achieve the above-mentioned object.
[1] A ligand for integrin α9β1, consisting of a peptide having the following amino acid sequence:
   (A) EDDMMEVPY (SEQ ID NO: 1) or
   (B) an amino acid sequence the same as the amino acid sequence represented by SEQ ID NO: 1 except for having deletion, substitution or addition of 1 to 3 amino acids.
[2] The ligand for integrin α9β1 according to the above [1], wherein the ligand is SVEP1 or an active fragment thereof.
[3] The ligand for integrin α9β1 according to the above [1] or [2], wherein the ligand has 2500 amino acid residues or less.
[4] A polynucleotide encoding a peptide constituting the ligand for integrin α9β1 according to any one of the above [1] to [3].
[5] An expression vector containing the polynucleotide according to the above [4].
[6] An antibody against the ligand for integrin α9β1 according to any one of the above [1] to [3].
[7] A method for screening for substances capable of inhibiting interaction of SVEP1 with integrin α9β1, the method using the ligand for integrin α9β1 according to any one of the above [1] to [3].
[8] A method for culturing integrin α9β1-expressing cells, the method using the ligand for integrin α9β1 according to any one of the above [1] to [3] as a substrate.
[9] A method for culturing tissue stem cells with maintenance of their undifferentiated state and inhibition of their proliferation, the method using the ligand for integrin α9β1 according to any one of the above [1] to [3] as a substrate.
[10] A method for separating integrin α9β1-expressing cells, the method allowing selective separation of cells capable of binding to the ligand for integrin α9β1 according to any one of the above [1] to [3].
[11] A drug delivery system for delivering a drug to integrin α9β1-expressing cells, the system comprising the ligand for integrin α9β1 according to any one of the above [1] to [3], and the drug.
[12] A method for inducing tissue stem cells in a resting phase or an antiproliferative state into a proliferative state, the method comprising bringing the stem cells into contact with the ligand for integrin α9β1 according to any one of the above [1] to [3].

### ADVANTAGEOUS EFFECTS OF INVENTION

According to the present invention, a novel ligand for integrin α9β1 can be provided. The novel ligand for integrin α9β1 has a higher binding affinity than those of known ligands for integrin α9β1. The novel ligand for integrin α9β1 can be preferably used for a method for screening for substances capable of inhibiting the interaction of SVEP1 with integrin α9β1; a method for culturing integrin α9β1-expressing cells; a method for culturing hematopoietic stem cells with maintenance of their undifferentiated state and inhibition of their proliferation; a method for delivering a drug to integrin α9β1-expressing cells; a method for inducing stem cells in the resting phase or an antiproliferative state into a proliferative state; and other purposes.

### BRIEF DESCRIPTION OF DRAWINGS

Fig. 1 shows the domain structure of mouse SVEP1 (referred to as "polydom" in the Drawings and the Brief Description of Drawings).
Fig. 2 shows the results of 6% non-reducing or reducing SDS-PAGE for a recombinant polydom having the signal sequence of the mouse immunoglobulin κ chain.
Fig. 3 shows the results of immunoblotting for a recombinant polydom having an N-terminal FLAG tag and a C-terminal His tag. (A) shows the results of immunoblotting using an anti-His antibody, and (B) shows the results of immunoblotting using an anti-FLAG antibody.
Fig. 4 shows the results of the examination of adhesion of RD cells to polydom.
Fig. 5 shows the results of microscopic observation of adhesion of RD cells onto different substrates.
Fig. 6 shows the results of the examination on integrin dependence of adhesion of RD cells onto polydom.
Fig. 7 shows the results of the examination of binding between recombinant integrin α9β1 and polydom.
Fig. 8 shows the results of the examination of binding between integrin α9β1 and polydom or other known ligands for integrin α9β1.
Fig. 9 shows N-terminal deletion mutants of polydom prepared for identification of the integrin α9β1-binding site in polydom.
Fig. 10 shows the results of the evaluation of the integrin α9β1-binding activities of N-terminal deletion mutants of polydom.
Fig. 11 shows the alignment of the amino acid sequences of the 20th, 21st and 22nd CCP domains by ClustalW.
Fig. 12 shows the results of the examination of the integrin α9β1-binding site in the 21st CCP domain (hereinafter referred to as "CCP21").
Fig. 13 shows the results of the examination of the integrin α9β1-binding site in the extra 37-amino acid segment (D2628-S2664) of CCP21.
Fig. 14 shows the results of the examination using alanine scanning mutants on the contribution of the amino acid residues in the extra 12-amino acid segment (D2634-L2645) of CCP21 to the binding to integrin α9β1.
Fig. 15 shows the results of the integrin α9β1-pol-C binding inhibition assay using synthetic peptides consisting of the EDDMMEVPY sequence or a part of the sequence.
Fig. 16 shows the localization of polydom and integrin α9 as revealed by immunofluorescence staining of mouse embryonic tissues.
Fig. 17 shows the results of the in situ integrin binding assay using cryosections of mouse embryos.
Fig. 18 shows the results of the examination of proliferation of hematopoietic stem cells on polydom or fibronectin.
Fig. 19 shows the results of the examination of differentiation of hematopoietic stem cells on polydom or fibronectin.
Fig. 20 shows the results of the examination of the effects of the EDDMMEVPY peptide on proliferation of hematopoietic stem cells on polydom.
Fig. 21 shows the results of fractionation of cardiac cells based on the fluorescence of green fluorescent protein (GFP) by cell sorter FACSAria. The cardiac cells were prepared 6 weeks after a fusion protein of GFP and histone H2B (H2B-GFP) was expressed in the heart of a mouse for 2 weeks starting 1 week before birth.
Fig. 22 shows the results of integrin α9 expression in the GFP-positive or GFP-non-positive cells fractionated as shown in Fig. 21, as analyzed by cell sorter FACSAria.
Fig. 23 shows the localization of GFP label-retaining cells and integrin α9 expressing cells in the cryosections of a mouse heart containing H2B-GFP-labeled cardiac stem cells, as revealed by double fluorescence staining.
Fig. 24 shows the localization of GFP label-retaining cells and polydom expressing regions in the cryosections of a mouse heart containing H2B-GFP-labeled cardiac stem cells.
Fig. 25 shows the results of the examination of the cell adhesion activities of polydom and fibronectin for H2B-GFP-labeled cardiac stem cells (GFP label-retaining cells).
Fig. 26 shows the results of the examination of the colony forming ability of H2B-GFP-labeled cardiac stem cells (GFP label-retaining cells) on polydom or fibronectin. (A) shows the colony forming activity, and (B) shows typical colony morphologies.

### DESCRIPTION OF EMBODIMENTS

The present inventors previously established a bioinformatics-based protocol for screening for ECM proteins and identified 16 novel ECM proteins from among more than 65000 varieties of mouse cDNAs available in the Riken FANTOM cDNA collection (Manabe, R., Tsutsui, K., Yamada, T., Kimura, M., Nakano, I., Shimono, C., Sanzen, N. , Furutani, Y. , Fukuda, T., Oguri, Y., Shimamoto, K., Kiyozumi, D., Sato, Y., Sado, Y., Senoo, H., Yamashina, S., Fukuda, S., Kawai, J., Sugiura, N., Kimata, K. , Hayashizaki, Y. , and Sekiguchi, K. (2008) Proc. Natl. Acad. Sci. U. S. A. 105, 12849-12854). The present inventors then extended the screening using this protocol to mouse and human genome databases, and focusing on transcripts encoding proteins of 1500 or more amino acid residues, identified more ECM proteins. One of the obtained candidates was SVEP1 (also called polydom). As a result of intensive research, the present inventors found that SVEP1 is a ligand for integrin α9β1. Subsequently, the present inventors found that SVEP1 is cleaved into an N-terminal fragment and a C-terminal fragment after secretion and that the C-terminal fragment has a ligand activity for integrin α9β1. Next, the present inventors found that the integrin α9β1 recognition sequence is present in the 21st complement control protein (CCP) domain from the N-terminus (CCP21) of mouse SVEP1, which contains 34 CCPs. The present inventors further found that an extra segment is present in CCP21, not in the other CCPs of mouse SVEP1 and that the integrin α9β1 recognition sequence is the amino acid sequence EDDMMEVPY (SEQ ID NO: 1) in the extra segment.

### Ligand for Integrin α9β1

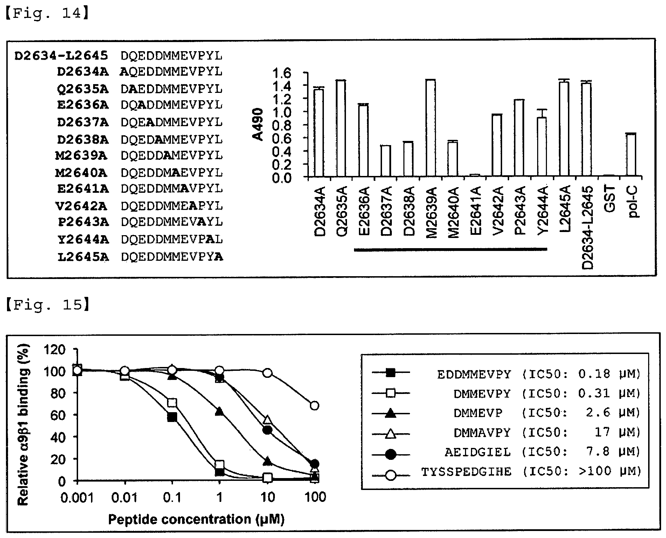

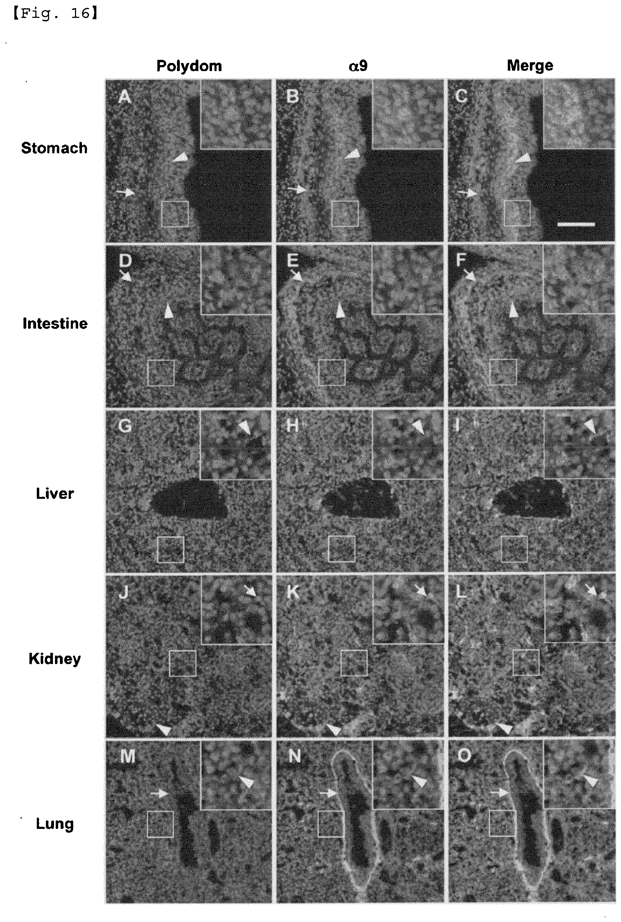

The present invention provides a ligand for integrin α9β1 consisting of a peptide having the amino acid sequence EDDMMEVPY (SEQ ID NO: 1). The present invention also provides a ligand for integrin α9β1 consisting of a peptide having an amino acid sequence the same as the amino acid sequence represented by SEQ ID NO: 1 except for having deletion, substitution or addition of 1 to 3 amino acids (hereinafter also referred to as "mutant of the sequence of SEQ ID NO: 1"). The present inventors examined the integrin α9β1 recognition sequence using deletion mutants, and as a result, found that deletion of E at residue 1 and deletion of D at residue 2 in the amino acid sequence represented by SEQ ID NO: 1 did not compromise the integrin α9β1-binding activity, and that further deletion of Y at residue 9 reduced the integrin α9β1-binding activity but did not abrogate it (see Fig. 16). The present inventors also used alanine scanning mutants to examine the integrin α9β1 recognition sequence. The examination results showed that alanine substitution of M at residue 4 hardly altered the integrin α9β1-binding activity and that alanine substitution of E at residue 1, P at residue 8, or the like had only a small contribution to reduction in the binding activity (see Fig. 14) . Therefore, peptides or proteins having a mutant of the sequence of SEQ ID NO: 1 have a ligand activity for integrin α9β1.

The ligand of the present invention for integrin α9β1 (hereinafter referred to as "ligand of the present invention") is not limited as long as the ligand consists of a peptide having the amino acid sequence represented by SEQ ID NO: 1 or a mutant of the sequence of SEQ ID NO: 1. That is, the peptide constituting the ligand of the present invention may consist of the amino acid sequence represented by SEQ ID NO: 1 or a mutant of the sequence of SEQ ID NO: 1, or comprise the amino acid sequence represented by SEQ ID NO: 1 or a mutant of the sequence of SEQ ID NO: 1, and an additional amino acid sequence other than both sequences. The additional amino acid sequence other than the amino acid sequence represented by SEQ ID NO: 1 and a mutant of the sequence of SEQ ID NO: 1 is not particularly limited and the number of its amino acid residues is also not limited. The "peptide" as used herein means a molecule of two or more amino acids bound together via a peptide bond(s) and the number of the amino acids bound together is not limited. That is, the "peptide" as used herein encompasses polypeptides.

In the amino acid sequence represented by SEQ ID NO: 1, E at residue 1 is considered to be substitutable by any amino acid. D at residue 2 is considered to be substitutable by any amino acid, preferably an acidic amino acid. D at residue 3 is considered to be substitutable by E. M at residue 4 is considered to be substitutable by any amino acid. M at residue 5 is considered to be substitutable by another hydrophobic amino acid. E at residue 6 is considered to be substitutable by D. V at residue 7 is considered to be substitutable by any amino acid, preferably a hydrophobic amino acid. P at residue 8 is considered to be substitutable by any amino acid, but preferably remains as it is. Y at residue 9 is considered to be substitutable by any amino acid, but preferably remains as it is.

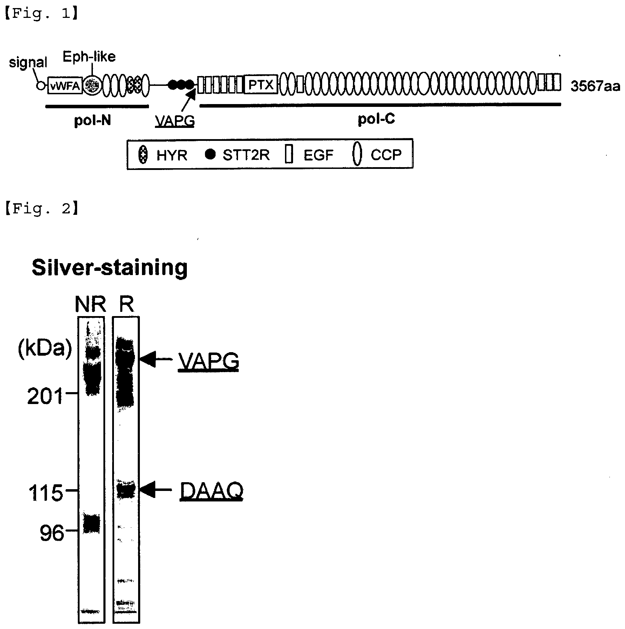

The ligand of the present invention is preferably full-length SVEP1 or an active fragment thereof. SVEP1 is a secretory protein present mainly in vertebrate ECM and has a number of domains. As shown in Fig. 1, mouse SVEP1 (full-length) has a signal sequence, a von Willebrand factor A domain (vWFA), an Eph-like domain, CCP domains, HYR domains, STT2R domains, EGF domains and a PTX domain. The domain structures of SVEP1 proteins of other mammals are the same as that shown in Fig. 1. The amino acid sequence represented by SEQ ID NO: 1 is well conserved in mammals. Therefore, the ligand of the present invention is preferably a mammalian SVEP1 or an active fragment thereof, and more preferably human SVEP1 or an active fragment thereof.

The accession numbers for the SVEP1 amino acid sequences and the nucleotide sequences of the SVEP1-encoding genes in typical mammals are shown in Table 1. These amino acid and nucleotide sequence data can be obtained from known databases (GenBank etc.). Each of the mammalian SVEP1 proteins shown in Table 1 has the amino acid sequence represented by SEQ ID NO: 1, and the amino acid sequence represented by SEQ ID NO: 1 is present in a CCP domain containing an extra segment, specifically present in the extra segment. The SVEP1 as the ligand of the present invention is not limited to these examples, and SVEP1 proteins of other animals are also considered suitable as the ligand for integrin α9β1. The amino acid and nucleotide sequence data of SVEP1 proteins of other animals can be obtained from known databases (GenBank etc.). The amino acid sequence of mouse SVEP1 is shown in SEQ ID NO: 2, the nucleotide sequence of mouse SVEP1 is shown in SEQ ID NO: 3, the amino acid sequence of human SVEP1 is shown in SEQ ID NO: 4, and the nucleotide sequence of human SVEP1 is shown in SEQ ID NO: 5.

**Table 1**

| Sequence data of SVEP1 (accession numbers) | | |
|---|---|---|
| | Amino acid sequence | Nucleotide sequence |
| Mouse | NP_073725 | NM_022814 |
| Human | NP_699197 | NM_153366 |
| Chimpanzee | XP_520182 | XM_520182 |
| Dog | XP_532030 | XM_532030 |
| Cattle | XP_002689967 | XM_002689921 |
| Horse | XP_001916184 | XM_001916149 |
| Pig | XP_003122123 | XM_003122075 |
| Rat | XP_001065678 | XM_001065678 |
| Rabbit | XP_002708118 | XM_002708072 |

The active fragment of SVEP1 as the ligand for integrin α9β1 may be any fragment that consists of a part of SVEP1 and has a ligand activity for integrin α9β1 (binding activity for integrin α9β1). Preferable examples of the active fragment of SVEP1 include a SVEP1 fragment having the amino acid sequence represented by SEQ ID NO: 1 or a mutant of the sequence of SEQ ID NO: 1. The specific examples include a mammalian SVEP1 fragment corresponding to the C-terminal fragment of mouse SVEP1 represented as "pol-C" in Fig. 1; a mammalian SVEP1 fragment having a CCP domain containing an extra segment; a mammalian SVEP1 fragment having the extra segment; a SVEP1 fragment having the amino acid sequence represented by SEQ ID NO: 1; and a SVEP1 fragment having a mutant of the sequence of SEQ ID NO: 1.

The number of amino acid residues of the peptide constituting the ligand of the present invention is not particularly limited, but preferably not more than about 2500 residues. The lower limit is 6 residues, which is the number when 3 amino acids are deleted from the amino acid sequence represented by SEQ ID NO: 1. Therefore, the number of amino acid residues of the peptide constituting the ligand of the present invention is preferably 6 to about 2500 residues, more preferably 6 to about 2000 residues, further preferably 6 to about 1500 residues, further preferably 6 to about 1000 residues, further preferably 6 to about 500 residues, further preferably 6 to about 200 residues, further preferably 6 to about 100 residues, further preferably 6 to about 50 residues, further preferably 6 to about 20 residues, and most preferably 9 residues.

The ligand of the present invention can be prepared, for example, by using known genetic engineering techniques, specifically by constructing a recombinant expression vector having an expressible insert of a gene encoding SVEP1 or an active fragment thereof, transfecting the vector into appropriate host cells for expression of a recombinant protein, and purifying the recombinant protein. Alternatively, for example, in vitro coupled transcription-translation system can also be used for the preparation of the ligand of the present invention. Also, according to a known ordinary peptide synthesis protocol, the ligand of the present invention can be prepared by a solid phase synthesis method (the Fmoc method, the Boc method) or a liquid phase synthesis method.

The C-terminus of the peptide maybe a carboxyl group (-COOH), a carboxylate (-COO⁻), an amide (-CONH₂) or an ester (-COOR). Examples of the R moiety in the ester include C₁₋₆ alkyl groups such as methyl, ethyl, n-propyl, isopropyl and n-butyl; C₃₋₈ cycloalkyl groups such as cyclopentyl and cyclohexyl; C₆₋₁₂ aryl groups such as phenyl and α-naphthyl; C₇₋₁₄ aralkyl groups such as phenyl-C₁₋₂ alkyl groups including benzyl and phenethyl, and α-naphthyl-C₁₋₂ alkyl groups including α-naphthylmethyl; and a pivaloyloxymethyl group, which is widely used as an ester for oral use. When the peptide has a carboxyl group or a carboxylate in a site other than the C-terminus, these groups may be amidated or esterified.

The amino acids constituting the peptide may have a substituent in the side chain. The substituent is not particularly limited and the examples include a fluorine atom, a chlorine atom, a cyano group, a hydroxyl group, a nitro group, an alkyl group, a cycloalkyl group, an alkoxy group and an amino group. Also, in the peptide, the amino group of the N-terminal amino acid residue may be protected by a protecting group (for example, C₁₋₆ acyl groups including a formyl group and a C₂₋₆ alkanoyl group such as acetyl, etc.); a glutamyl group resulting from in vivo N-terminal cleavage of the peptide may be pyroglutamated; and a side chain substituent (s) (for example, -OH, -SH, an amino group, an imidazole group, an indole group, a guanidino group, etc.) of the intramolecular amino acid(s) may be protected by an appropriate protecting group(s) (for example, C₁₋₆ acyl groups including a formyl group and a C₂₋₆ alkanoyl group such as acetyl, etc.).

The peptide may be in the form of a pharmaceutically acceptable salt. Examples of the salt include a salt formed with an acid such as hydrochloric acid, sulfuric acid, phosphoric acid, lactic acid, tartaric acid, maleic acid, fumaric acid, oxalic acid, malic acid, citric acid, oleic acid and palmitic acid; a salt formed with a hydroxide or a carbonate of an alkali or alkaline earth metal such as sodium, potassium and calcium, and a salt formed with a hydroxide or a carbonate of aluminum; and a salt formed with triethylamine, benzylamine, diethanolamine, tert-butylamine, dicyclohexylamine, arginine or the like.

### Polynucleotide

The polynucleotide of the present invention may be any polynucleotide that encodes the peptide constituting the ligand of the present invention. The polynucleotide can be present in the form of RNA (for example, mRNA) or DNA (for example, cDNA or genomic DNA). The polynucleotide may be a double or single strand. The double strand may be a double-stranded DNA, a double-stranded RNA or a DNA-RNA hybrid. The single strand may be a coding strand (sense strand) or a non-coding strand (antisense strand). The polynucleotide of the present invention may be fused with a polynucleotide encoding a tag for labeling (a tag sequence or a marker sequence) at the 5'- or 3'-terminus. The polynucleotide of the present invention may further contain an untranslated region (UTR) sequence, a vector sequence (including an expression vector sequence), etc.

The polynucleotide of the present invention can be obtained by a known DNA synthesis method, PCR, etc. To be more specific, in one example, based on the amino acid sequence of the peptide constituting the ligand of the present invention, the nucleotide sequence is designed by appropriate selection of the codon of each amino acid, and the designed nucleotide sequence is chemically synthesized on a commercial DNA synthesizer to give the polynucleotide of the present invention. In another example, primers are designed for amplifying a region which encodes the peptide constituting the ligand of the present invention in the nucleotide sequence of the SVEP1-encoding gene (see SEQ ID NOS: 3 and 5, and the accession numbers in Table 1), and using these primers and a template such as genomic DNA and cDNA of the same animal origin, PCR is performed to give a DNA fragment containing the polynucleotide of the present invention in a large amount.

### Expression Vector

The present invention provides an expression vector used for the production of the ligand of the present invention. The expression vector of the present invention is not particularly limited as long as it contains a polynucleotide encoding the peptide constituting the ligand of the present invention, but preferred are plasmid vectors carrying a recognition sequence for RNA polymerase (pSP64, pBluescript, etc.). The method for preparing the expression vector is not particularly limited, and the expression vector may be prepared with the use of a plasmid, a phage, a cosmid or the like. The kind of the vector is not particularly limited and any appropriate vector that can be expressed in host cells can be selected. For example, depending on the kind of the host cell, an appropriate promoter sequence to ensure the expression of the polynucleotide of the present invention is selected, and this promoter sequence and the polynucleotide of the present invention are inserted into a plasmid etc. to prepare the desired expression vector. After a host transformed with the expression vector of the present invention is cultured, cultivated or bred, the peptide constituting the ligand of the present invention can be collected and purified from the culture products etc. by conventional methods (for example, filtration, centrifugation, cell disruption, gel filtration chromatography, ion exchange chromatography, etc.).

The expression vector preferably contains at least one selection marker. Examples of the marker include a dihydrofolate reductase gene and a neomycin resistance gene for eukaryote cell culture; and a tetracycline resistance gene and an ampicillin resistance gene for culture of *Escherichia coli* (*E. coli*) and other bacteria. Such a selection marker is useful for checking whether the polynucleotide of the present invention has been successfully transfected into host cells and is reliably expressed therein. Also, the peptide constituting the ligand of the present invention may be expressed as a fusion peptide. For example, green fluorescent protein (GFP) derived from *Aequorea coerulescens* is used as a marker and the peptide constituting the ligand of the present invention is expressed as a GFP fusion peptide.

The host is not particularly limited and various known cells can be preferably used. Specific examples of the host include bacteria such as *E. coli,* yeasts (budding yeast *Saccharomyces cerevisiae* and fission yeast *Schizosaccharomyces pombe*), nematodes (*Caenorhabditis elegans*), *Xenopus laevis* oocytes and animal cells (for example, CHO cells, COS cells and Bowes melanoma cells). The method for transfecting the above-mentioned expression vector into host cells, i.e. the transformation method, is also not particularly limited and known methods such as electroporation, the calcium phosphate method, the liposome method and the DEAE dextran method can be preferably used. The thus-obtained transformant is also described herein.

### Antibody

The present invention provides an antibody against the ligand of the present invention. The antibody against the ligand of the present invention may be a polyclonal or monoclonal antibody. The antibody may also be a complete antibody molecule or an antibody fragment capable of specifically binding to an antigen of interest (for example, Fab, F (ab')₂, Fab', Fv, scFv, etc.). The polyclonal antibody can be obtained in the following manner, for example. An antigen (the peptide constituting the ligand of the present invention) is dissolved in PBS and if needed further mixed with an appropriate amount of a usual adjuvant (for example, Freund's complete adjuvant) to prepare an immunogen, and a mammal (a mouse, a rat, a rabbit, a goat, a horse, etc.) is immunized with the immunogen. The method for the immunization is not particularly limited, but preferred is subcutaneous or intraperitoneal injection given once or repeated several times at appropriate intervals, for example. Then, blood collection from the immunized animal, serum separation and purification of polyclonal antibody fractions are performed in a usual manner. The monoclonal antibody can be obtained by fusing immune cells (for example, splenocytes) obtained from the above-mentioned immunized mammal with myeloma cells to produce a hybridoma, culturing the hybridoma and collecting an antibody from the culture. A recombinant monoclonal antibody can also be produced by using recombinant techniques, specifically by cloning an antibody gene from the hybridoma, inserting the gene into a suitable vector and transfecting the vector into host cells. A phage display method can also be used for production of the monoclonal antibody.

### Screening Method

The present invention provides a method for screening for substances capable of inhibiting the interaction of SVEP1 with integrin α9β1. The screening method of the present invention is not limited as long as the method uses the ligand of the present invention. The test substance to be screened is not particularly limited and the examples include nucleic acids, peptides, proteins, non-peptidic compounds, synthetic compounds, fermentation products, cell extracts, cell culture supernatants, plant extracts, mammalian tissue extracts and plasma.

The details of the screening method are not particularly limited. For example, in the case where the binding level of the ligand of the present invention and integrin α9β1-expressing cells brought into contact with a test substance is reduced as compared with that of the ligand of the present invention and integrin α9β1-expressing cells not brought into contact with the test substance, the test substance is selected as a substance capable of inhibiting the interaction of SVEP1 with integrin α9β1. The degree of reduction of the binding level by a test substance to be selected is not particularly limited, and for example, the binding level is reduced to preferably 50% or less and more preferably 25% or less relative to that of the cells not brought into contact with the test substance.

The substance selected by the screening method of the present invention can inhibit the interaction of SVEP1 with integrin α9β1, and thus is useful as, for example, a proliferation inducer for stem cells in the resting phase or an antiproliferative state, a therapeutic medicine of autoimmune arthritis, etc.

### Culture Method

The present invention provides a method for culturing integrin α9β1-expressing cells. The culture method of the present invention is not limited as long as the method uses the ligand of the present invention as a substrate. The ligand of the present invention is discovered based on the ECM protein SVEP1 and thus is very useful as a substrate for culture of integrin α9β1-expressing cells. In an example of the culture method of the present invention, integrin α9β1-expressing cells are cultured on a culture plate coated with the ligand of the present invention. Examples of the integrin α9β1-expressing cells include tissue stem cells such as hematopoietic stem cells, corneal stem cells and cardiac stem cells, epithelial cells, smooth muscle cells, vascular endothelial cells, hepatocytes and neutrophils. The medium to be used is not particularly limited and any appropriate medium for the cells to be cultured may be selected. As needed, growth factors such as serum can be added depending on the purpose.

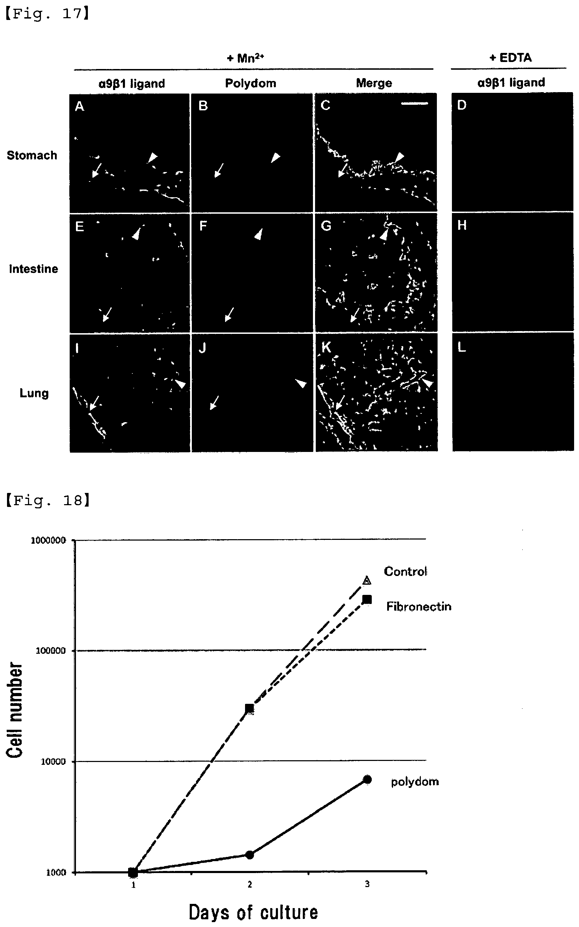

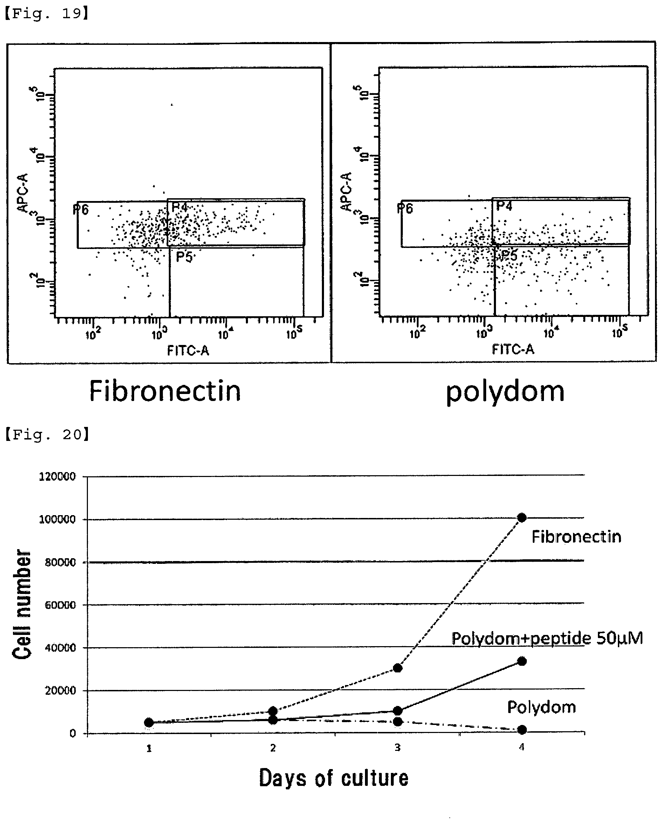

The present invention also provides a method for culturing tissue stem cells with maintenance of their undifferentiated state and inhibition of their proliferation. The method of the present invention for culturing tissue stem cells is not limited as long as the method uses the ligand of the present invention as a substrate. The present inventors found that hematopoietic stem cells cultured on a culture substrate coated with the integrin α9β1 ligand SVEP1 were remarkably inhibited from proliferating and maintained in their undifferentiated state in comparison with those cultured on a culture substrate coated with PBS (control) or fibronectin (see Figs. 18 and 19). Therefore, the ligand of the present invention is very useful as a substrate for culturing tissue stem cells with maintenance of their undifferentiated state and inhibition of their proliferation. The medium used for the method for culturing tissue stem cells with maintenance of their undifferentiated state and inhibition of their proliferation is not particularly limited as long as the medium is suitable for culture of tissue stem cells. However, it is preferable that growth factor-free media such as serum-free media are used.

### Separation Method

The present invention provides a method for separating integrin α9β1-expressing cells. The separation method of the present invention is not limited as long as the method allows selective separation of cells capable of binding to the ligand of the present invention. Since the ligand of the present invention is capable of binding to integrin α9β1-expressing cells, selective separation of cells bound to the ligand of the present invention enables isolation and concentration of integrin α9β1-expressing cells. The means for selective separation of the cells bound to the ligand of the present invention is not particularly limited and known means for cell separation can be preferably used. Examples of the known means include magnetic beads onto which the ligand of the present invention is immobilized, resins onto which the ligand of the present invention is immobilized, and a cell sorter. Examples of the integrin α9β1-expressing cells to be separated include tissue stem cells such as hematopoietic stem cells, corneal stem cells and cardiac stem cells, smooth muscle cells, vascular endothelial cells, hepatocytes and neutrophils.

### Drug Delivery System

The present invention provides a drug delivery system which comprises the ligand of the present invention and a drug and is intended for delivering the drug to integrin α9β1-expressing cells. Since the ligand of the present invention has an integrin α9β1-binding activity, it can be preferably used in a drug delivery system for delivering a drug to integrin α9β1-expressing cells. Examples of the integrin α9β1-expressing cells include tissue stem cells such as hematopoietic stem cells, corneal stem cells and cardiac stem cells, epithelial cells, smooth muscle cells, vascular endothelial cells, hepatocytes and neutrophils.

The drug delivery system of the present invention may be in the form of a composition in which the ligand of the present invention and the drug are mixed but not integrated with each other, but preferably in the form in which the ligand of the present invention and the drug are integrated with each other via some interaction. The means for such integration is not particularly limited, and the drug delivery system may be in the form of, for example, a complex of the ligand of the present invention and a liposome encapsulating the drug, or a complex of the ligand of the present invention and a nano material carrying the drug. Another possible form is the one in which the ligand of the present invention and the drug are directly bound to each other. In the case where the drug is a protein, a fusion protein of the drug and the ligand of the present invention is suitable.

The drug used in the drug delivery system of the present invention is not particularly limited as long as it is preferable to deliver the drug to target integrin α9β1-expressing cells. Examples of the drug include drugs for disease treatment and drugs for visualization of integrin α9β1-expressing cells (a fluorescent substance, a radioactive substance, a chemiluminescent substance, a magnetic substance, etc.).

### Proliferation Inducing Method

The present invention provides a method for inducing stem cells in the resting phase or an antiproliferative state into a proliferative state by bringing the stem cells into contact with the ligand of the present invention. The present inventors found that although hematopoietic stem cells cultured on a culture substrate coated with the integrin α9β1 ligand SVEP1 was remarkably inhibited from proliferating, the antiproliferative state of hematopoietic stem cells on a SVEP1-coated culture substrate was canceled by treatment with a peptide consisting of the amino acid sequence represented by SEQ ID NO: 1, which is an integrin α9β1 ligand (see Fig. 20). Therefore, the ligand of the present invention can be preferably used for the applications requiring cancellation of the undifferentiated state of hematopoietic stem cells and induction of the cells into a proliferative state.

The application of the proliferation inducing method of the present invention to leukemia patients holds promise for improving the effectiveness of leukemia treatment. That is, the application of the proliferation inducing method enables leukemic stem cells in the resting phase or an antiproliferative state present in the bone marrow of leukemia patients to be induced into an antiproliferative state and thereby migrated into a blood flow, and subsequent administration of a medicine having specific effect on leukemia cells (tyrosine kinase inhibitors etc.) can potentially result in efficient killing of the leukemic stem cells.

### Other Applications

The ligand of the present invention is useful for other applications, for example, as a reagent for integrin α9β1 activation signaling studies, a reagent for collection of integrin α9β1-expressing cells, etc.

### EXAMPLES

Hereinafter, the present invention will be illustrated in detail by examples, but is not limited thereto.

### Example 1: Identification of Novel Ligand for Integrin α9β1 Experimental Procedures

### (1) cDNA Cloning and Construction of Expression Vectors

A DNA segment encoding a FLAG tag and a DNA segment encoding a 6xHis tag were PCR-amplified. These amplified segments were separately inserted into the NotI/ApaI site of the pcDNA3.1(+) vector or the pSecTag2B vector (these vectors are available from Invitrogen), yielding pcDNA-FLAG, pSec-FLAG and pSec-His. For construction of expression vectors for N-terminal FLAG-tagged proteins, the DNA segment encoding the FLAG sequence was inserted into the HindIII/BamHI site of the pSec-His vector to yield pSec-NFLAG-His. A cDNA encoding mouse SVEP1 (hereinafter referred to as "polydom" in Examples) was obtained by RT-PCR using RNA extracted from mouse embryos at embryonic day 7 (Clontech) and subcloned into pBluescript KS(+) (Stratagene). After verification of the nucleotide sequence, an error-free cDNA fragment was inserted into pcDNA-FLAG, pSec-FLAG, pSec-His or pSec-NFLAG-His at the BamHI/NotI site. Expression vectors for truncated forms of polydom were also constructed by subcloning of a PCR-amplified cDNA into pSec-His at the BamHI/NotI site or the HindIII/NotI site.

### (2) Expression and Purification of Recombinant Polydom Proteins

Recombinant polydom and its fragments were produced using the Freestyle™ 293 Expression System (Invitrogen). An N-terminal fragment of polydom (hereinafter referred to as "pol-N"; see Fig. 1) is a fragment having the amino acid sequence of residues 18 to 789 of SEQ ID NO: 2 and additionally having a His tag at the C-terminus. A C-terminal fragment of polydom (hereinafter referred to as "pol-C"; see Fig. 1) is a fragment having the amino acid sequence of residues 1192 to 3567 of SEQ ID NO: 2 and additionally having a His tag at the C-terminus. Freestyle™ 293-F cells were transfected with the expression vectors using 293fectin (Invitrogen) and grown in serum-free Freestyle™ 293 expression medium for 72 hours. The conditioned media were collected and clarified by centrifugation. To check the expression, the cells were lysed with TBS containing 1% (w/v) Nonidet P-40, 1 mM phenylmethylsulfonyl fluoride, 5 µg/ml aprotinin, 5 µg/ml leupeptin and 5 µg/ml pepstatin. The conditioned media and the cell lysates were pulled down using Ni-NTA-agarose (Qiagen) or anti-FLAG M2-agarose (Sigma), and subjected to immunoblotting. For purification of FLAG-tagged proteins, the conditioned media were separately applied to an anti-FLAG M2-agarose column, and the bound proteins were eluted with 100 µg/ml FLAG peptide in PBS. For purification of His-tagged proteins, the conditioned media were subjected to affinity chromatography using Ni-NTA-agarose. After column washing with PBS, the bound proteins were eluted with PBS containing 200 mM imidazole. The eluted proteins were dialyzed against PBS. The concentrations of the purified proteins were determined by the Bradford assay using bovine serum albumin (BSA) as a standard.

### (3) Anti-polydom Antibodies

Antibodies against polydom were raised in rabbits using FLAG-tagged full-length polydom as an immunogen. The antibodies were affinity-purified on columns containing either pol-N or pol-C. The columns were washed with 10 mM Tris-HCl buffer (pH 7.5) containing 0.5 M NaCl, and the bound antibodies were then eluted with 0.1 M glycine-HCl (pH 2.8). The eluted fractions were immediately neutralized with 1 M Tris-HCl (pH 9.0) and dialyzed against PBS. An Alexa Fluor™ 555-conjugated anti-polydom (pol-N) antibody was prepared using the APEX™ Alexa Fluor antibody labeling kit (Invitrogen).

### (4) Cell Adhesion Assay

A cell adhesion assay was performed as described in Sato, Y., Uemura, T. , Morimitsu, K. , Sato-Nishiuchi, R. , Manabe, R. , Takagi, J., Yamada, M., and Sekiguchi, K. (2009) J. Biol. Chem. 284, 14524-14536. For an assay involving blocking antibodies, RD cells (human rhabdomyosarcoma cells) were preincubated with monoclonal antibodies of interest at room temperature for 15 minutes and plated on 96-well plates coated with recombinant polydom proteins or fibronectin. The cells were incubated for 30 minutes, washed, fixed with 3.7% formaldehyde, and stained with 0.5% toluidine blue.

### (5) Flow Cytometry

The integrin α9β1 expression levels on A549 cells (human lung adenocarcinoma cells), HT1080 cells (human fibrosarcoma cells) and RD cells were verified by flow cytometry using anti-integrin α9β1 monoclonal antibody Y9A2. Normal mouse IgG was used as a control.

### (6) Expression and Purification of Recombinant Integrins

A cDNA encoding the extracellular region of human integrin α9 was amplified by RT-PCR from total RNA extracted from RD cells and cloned into pBluescript KS(+). After verification of the nucleotide sequence, an error-free cDNA fragment was inserted into pcDNA-ACID-FLAG (Nishiuchi, R., Takagi, J., Hayashi, M., Ido, H., Yagi, Y. , Sanzen, N. , Tsuji, T., Yamada, M., and Sekiguchi, K. (2006) Matrix Biol. 25, 189-197). An expression vector for the extracellular region of integrin β1 was provided by Dr. Junichi Takagi (Takagi, J., Erickson, H. P., and Springer, T. A. (2001) Nat. Struct. Biol. 8, 412-416). The recombinant integrins were produced using the Freestyle™ 293 Expression System (Invitrogen) and purified as described in the above-mentioned publication Matrix Biol. 25, 189-197, (2006).

### (7) SDS-PAGE, Western Blotting and Amino Acid Sequencing

SDS-PAGE was performed according to the Laemmli protocol. The separated proteins were visualized by staining with silver nitrate or Coomassie Brilliant Blue (CBB) or transferred onto a nitrocellulose membrane (for immunoblotting) or a polyvinylidene difluoride membrane (for amino acid sequencing). For immunoblotting, the membrane was probed with antibodies and then visualized with an ECL detection kit (GE Healthcare). For amino acid sequencing, the protein bands on the membrane were visualized by CBB staining and then dissected out. The N-terminal amino acid sequences were analyzed by the method of Edman using a Procise 491 cLC protein sequencer (Applied Biosystems).

### (8) Integrin Binding Assay

An integrin binding assay was performed as described in the above-mentioned publication Matrix Biol. 25, 189-197, (2006). In some experiments, integrins were preincubated with synthetic peptides at various concentrations to evaluate the inhibitory activities of the synthetic peptides. The apparent dissociation constants were determined as described in Nishiuchi, R., Murayama, O., Fujiwara, H., Gu, J., Kawakami, T., Aimoto, S., Wada, Y., and Sekiguchi, K. (2003) J. Biochem. 134, 497-504.

### (9) Expression and Purification of GST-fused Polydom, Tenascin-C and Osteopontin Fragments

cDNAs encoding CCP21 or its deletion mutants were separately PCR-amplified and subcloned into pGEX4T-1 (GE Healthcare) at the EcoRI/SalI site. CCP21 and the same domain lacking Glu2628-Ser2664 (hereinafter referred to as "ΔD2628-S2664") were His-tagged at their C-termini. GST fusion proteins were induced in *E*. *coli* BL21 by incubation with 0.1 mM IPTG at 25°C for 2 hours. The cells were lysed by sonication and the supernatants were passed over a glutathione-Sepharose 4B (GE Healthcare) column. The bound proteins were eluted with 50 mM Tris-HCl (pH 8.0) containing 10 mM glutathione. The resulting GST fusion proteins of intact CCP21 or mutant CCP21 were further purified on an Ni-NTA-agarose column. The purified proteins were dialyzed against 20 mM HEPES buffer (pH 8.0) containing 130 mM NaCl and then quantified by the Bradford assay.

A cDNA encoding the third fibronectin type III domain of human tenascin-C (hereinafter referred to as "TNfn3") was obtained by RT-PCR using RNA extracted from HT1080 cells and subcloned into pBluescript KS(+). A cDNA encoding human osteopontin was purchased from the NIH Mammalian Gene Collection (Invitrogen). A cDNA encoding an osteopontin N-terminal fragment (hereinafter referred to as "OPN-Nhalf") was PCR-amplified. The RGD motifs within TNfn3 and OPN-Nhalf were then mutated to RAA for specific binding to integrin α9β1 (hereinafter referred to as "TNfn3-RAA" and OPN-Nhalf-RAA," respectively). After verification of the nucleotide sequence, the cDNA fragments were separately inserted into pGEX4T-1 at the EcoRI/SalI site. Both proteins were expressed and purified as described above.

### (10) Immunohistochemistry

Mouse embryos were embedded in O.C.T. compound (Sakura Finetek) for cryosectioning. The resulting sections were fixed with 3.7% formaldehyde (single staining for polydom) or ice-cold acetone (double staining for polydom and integrin α9). The fixed sections were subjected to pretreatment with chondroitinase ABC (5 U/ml, Seikagaku Corp.) and hyaluronidase (1 U/ml, Sigma) in PBS at 37°C for 30 minutes, followed by endogenous peroxidase inactivation with 0.3% H₂O₂ and blocking with PBS containing 1% BSA. The sections were labeled with antibodies at 4°C overnight and washed in PBS. The bound antibodies were visualized with HRP polymer-conjugated anti-rabbit IgG antibody and DAB (Dako), or with Alexa Fluor™-conjugated secondary antibodies. Finally, the sections were counterstained with Mayer' s hematoxylin (for DAB staining) or Hoechst 33342 (for immunofluorescence staining), and then mounted with Mount-Quick (Daido Sangyo) or Fluorescent Mounting Medium (Dako).

### (11) In Situ Integrin Binding Assay

Cryosections of mouse embryos were fixed with ice-cold acetone for 15 minutes, washed with TBS, and blocked with 1% BSA in TBS. After three washes with TBS, the sections were incubated with recombinant integrin α9β1 (3 µg/ml) in TBS containing 1% BSA and 1 mM MnCl₂ at 4°C overnight. The sections were then washed with TBS containing 1 mM MnCl₂ (TBS/Mn) and incubated with an anti-Velcro antibody (0.5 µg/ml) in TBS/Mn containing 1% BSA (TBS/Mn/BSA) at room temperature for 2 hours. After washing with TBS/Mn, the sections were incubated with Alexa Fluor™ 488-conjugated goat anti-rabbit IgG antibody in TBS/Mn/BSA at room temperature for 2 hours. After washing, the sections were incubated with 100 µg/ml normal rabbit IgG (Dako) in TBS/Mn/BSA at room temperature for 1 hour to block secondary antibody unbound sites. After washing with 20 mM HEPES buffer (pH 7.5) containing 130 mM NaCl and 1 mM MnCl₂, the sections were refixed with 3.7% formaldehyde in 20 mM HEPES buffer (pH 7.5) containing 130 mM NaCl and 1 mM MnCl₂ for 10 minutes. The sections were then washed with TBS and incubated with an Alexa Fluor™ 555-conjugated anti-polydom antibody in TBS containing 1% BSA at 4°C overnight. The sections were washed with TBS and mounted with Fluorescent Mounting Medium (Dako).

### (12) Antibodies Used

A mouse monoclonal antibody (mAb) against human integrin α3 (3G8) and a mouse mAb against human integrin α3 (8F1) were raised in the inventors' laboratory as described in Kikkawa, Y., Sanzen, N., Fujiwara, H., Sonnenberg, A., and Sekiguchi, K. (2000) J. Cell Sci. 113, 869-876; and Manabe, R., Ohe, N., Maeda, T., Fukuda, T., and Sekiguchi, K. (1997) J. Cell Biol. 139, 295-307, respectively. An mAb against integrin α6 subunit (AMCI7-4) was provided by Dr. Masahiko Katayama (Tsukuba Research Laboratories, Eisai Co.) (Katayama, M., Sanzen, N., Funakoshi, A., and Sekiguchi, K. (2003) Cancer Res. 63, 222-229). A mouse mAb against human integrin β1 (AIIB2) was obtained from the Developmental Studies Hybridoma Bank (Iowa). A mouse mAb against human integrin α2 subunit (P1E6), a mouse mAb against human integrin α9β1 (Y9A2), and normal mouse IgG were purchased from Santa Cruz Biotechnology, Inc. A goat polyclonal antibody against mouse integrin α9 was obtained from R&D Systems. An HRP-conjugated anti-FLAG M2 antibody and an anti-penta-His antibody were obtained from Sigma and Qiagen, respectively. An anti-Velcro antibody was raised in rabbits by immunization with coiled-coil ACID and BASE peptides as described in Takagi, J. , Erickson, H. P., and Springer, T. A. (2001) Nat. Struct. Biol. 8, 412-416.

### Experimental Results

### (1) Confirmation of Recombinant Polydom

The spent culture medium of cells for the expression of a recombinant polydom having the signal sequence of the mouse immunoglobulin κ chain was affinity-purified using an anti-FLAG monoclonal antibody, and the resulting polydom was subjected to 6% SDS-PAGE under non-reducing or reducing conditions.

The results are shown in Fig. 2. As is clear from Fig. 2, two major bands of 220 kDa and 100 kDa were detected under non-reducing conditions (NR). A similar banding pattern was obtained under reducing conditions (R) except that the corresponding two major bands migrated more slowly (270 kDa and 115 kDa), suggesting that both were enriched in intrapeptide disulfide bonds. Determination of the N-terminal amino acid sequences of the two protein bands revealed that the 115 kDa band started with DAAQ, which is the putative N-terminal sequence lacking the signal sequence of the mouse immunoglobulin κ chain. It was also revealed that the 270 kDa band started with VAPG, which is identical to the sequence of residues 1177 to 1180 of SEQ ID NO: 2 located N-terminal to the first EGF domain. These findings indicate that polydom was proteolytically processed, before or after secretion, into two parts (i.e. the N-terminal 115-kDa fragment and the C-terminal 270-kDa fragment), which remained associated with each other after the proteolytic cleavage.

Next, a recombinant polydom having an N-terminal FLAG tag and a C-terminal His tag was expressed in 293-F cells and then precipitated from the spent medium and cell lysate with anti-FLAG antibody beads or Ni-NTA beads. The precipitates were subjected to 5% SDS-PAGE under reducing conditions and analyzed by immunoblotting using an anti-His antibody and an anti-FLAG antibody. Similar procedures were performed on untransfected 293-F cells as a negative control.

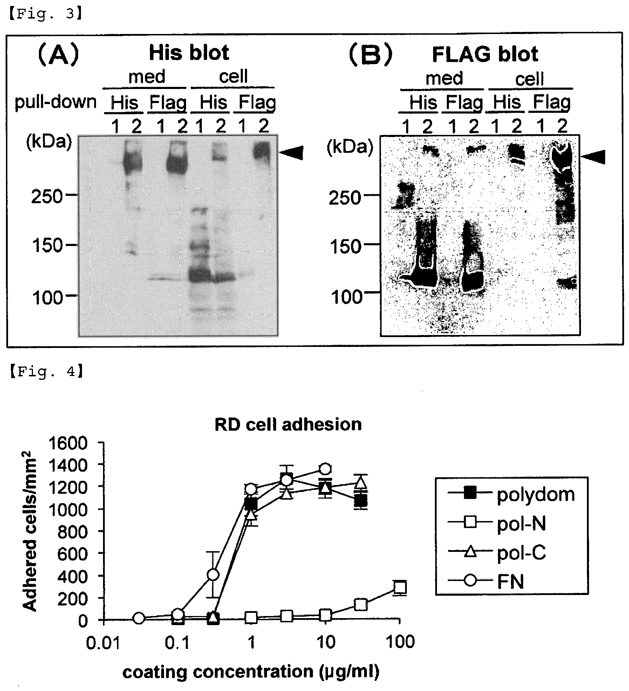

The results are shown in Figs. 3 (A) and (B). (A) shows the results of immunoblotting using an anti-His antibody, and (B) shows the results of immunoblotting using an anti-FLAG antibody. In Fig. 3, med represents medium, cell represents cell lysate, and His represents the precipitate obtained using Ni-NTA beads, and FLAG represents the precipitate obtained using anti-FLAG antibody beads. Lane 1 is for the negative control and lane 2 is for polydom. In the precipitate from the spent medium, both the 270-kDa and 115-kDa fragments were recovered irrespective of the type of bead used, thus confirming that these fragments remained associated with each other. Notably, a high molecular weight band migrating in the >300 kDa region was detected in the precipitate from the cell lysate irrespective of the type of bead used (arrowheads in Figs. 3 (A) and (B)). Therefore, it seems likely that the proteolytic processing of polydom into the N-terminal 115-kDa and C-terminal 270-kDa fragments occurs after secretion.

### (2) Polydom Mediates Integrin α9β1-dependent Cell Adhesion

Whether polydom is capable of promoting cell adhesion was examined with the use of the following three human cell lines: A549 human lung adenocarcinoma cells, HT1080 human fibrosarcoma cells, and RD human rhabdomyosarcoma cells. The cells were plated on 96-well plates coated with serial diluted concentrations of full-length polydom, pol-N, pol-C or plasma fibronectin (FN) and incubated at 37°C for 30 minutes. After washing out unattached cells, attached cells were fixed and stained with toluidine blue. The attached cells were counted under a microscope. The assay was performed in triplicate.

The results of RD cells are shown in Fig. 4. As is clear from Fig. 4, RD cells adhered to full-length polydom in a coating concentration-dependent manner, attaining the maximum adhesion at a coating concentration of 3 µg/ml. In the coating with pol-N or pol-C, only pol-C was capable of promoting cell adhesion, with a potency that was almost equivalent to that of full-length polydom. Although data are not shown, A549 and HT1080 cells did not adhere to polydom.

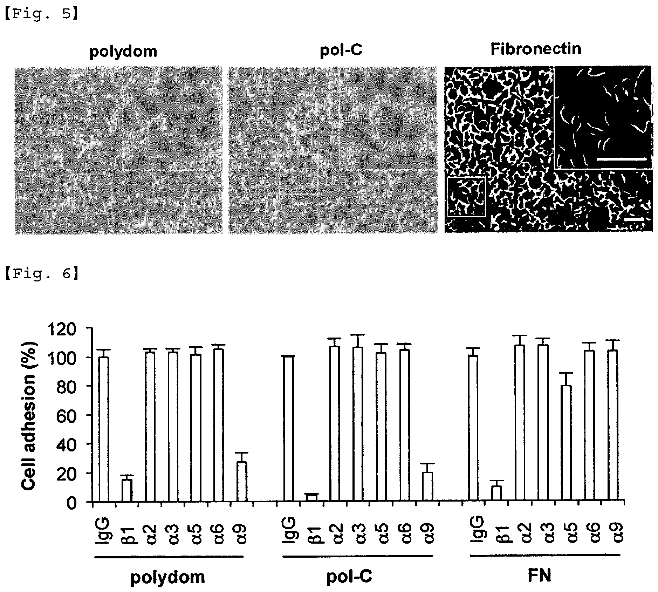

Fig. 5 shows the results of microscopic observation of adhesion of RD cells to full-length polydom, pol-C and fibronectin. As is understood from Fig. 5, the cells attached onto polydom or pol-C exhibited spread morphology, although the extent of the spreading was less pronounced than that of the cells attached onto fibronectin.

These findings indicate that polydom is capable of mediating cell adhesion and subsequent cell spreading, and that the cell adhesion-promoting activity resides within the C-terminal region composed of an array of CCP domains.

Next, it was explored whether the adhesion of RD cells to polydom is dependent on integrins, which are major cell surface receptors for ECM proteins. Ninety-six-well plates were coated with full-length polydom (3 µg/ml), pol-C (3 µg/ml) or plasma fibronectin (1 µg/ml). RD cells were preincubated with seven kinds of function-blocking mAbs (10 µg/ml) at room temperature for 10 minutes and then added to the precoated wells. The seven kinds of function-blocking mAbs were control mouse IgG (IgG), an anti-integrin β1 mAb AIIB2 (β1), an anti-integrin α2 mAb P1E6 (α2), an anti-integrin α3 mAb 3G8 (α3), an anti-integrin α5 mAb 8F1 (α5), an anti-integrin α6 mAb GoH3 (α6) and an anti-integrin α9β1 mAb Y9A2 (α9). After 30 minutes of incubation at 37°C, unattached cells were washed out. Then, attached cells were fixed and stained with toluidine blue.

The results are shown in Fig. 6. In the figure, the number of the attached cells is expressed as a percentage relative to the number of the attached cells in the treatment with control mouse IgG (100%). As is clear from Fig. 6, the mAb against integrin β1 subunit strongly inhibited the adhesion of RD cells to polydom. The mAbs against integrin α2, α3, α5 and α6 subunits did not inhibit the adhesion of RD cells to polydom, whereas the mAb against integrin α9β1 strongly inhibited the adhesion of RD cells to polydom. These findings are consistent with the finding that A549 and HT1080 cells did not adhere to polydom because integrin α9β1 was expressed on RD cells but not on A549 or HT1080 cells. Thus, it seems likely that the cell adhesion to polydom is primarily mediated by integrin α9β1.

### (3) Polydom Is a Preferred Ligand for Integrin α9β1

To corroborate the role of integrin α9β1 as an adhesion receptor for polydom, a direct integrin binding assay was performed using recombinant integrin α9β1. Plates were coated with full-length polydom, pol-N, pol-C, plasma fibronectin (pFN) or cellular fibronectin (cFN), and recombinant integrin α9β1 was added and allowed to bind thereto in the presence of 1 mM MnCl₂ or 10 mM EDTA. BSA was used as a negative control. The bound integrin was quantified using a biotinylated anti-Velcro antibody and HRP-conjugated streptavidin.

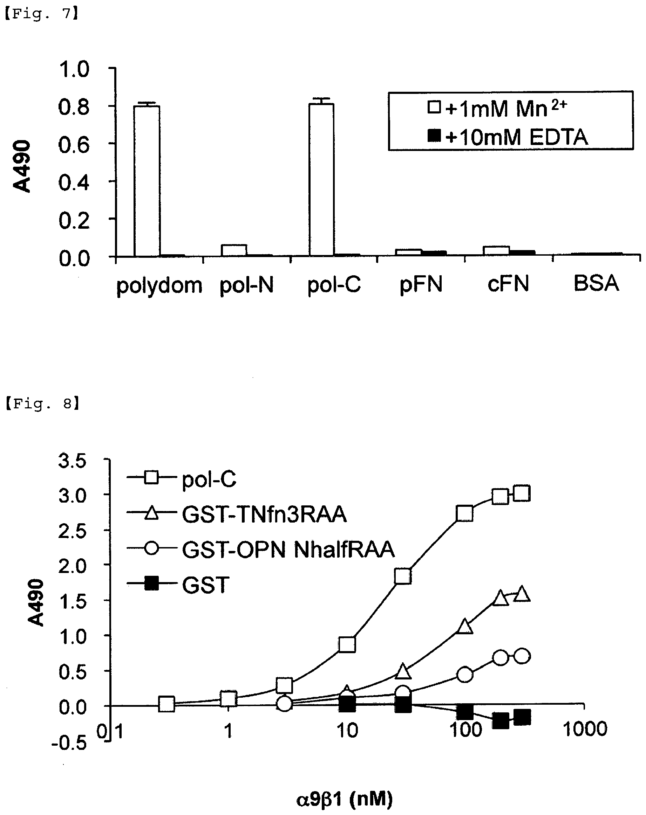

The results are shown in Fig. 7. As is clear from Fig. 7, the recombinant integrin α9β1 bound to full-length polydom and pol-C, but not to pol-N. The binding of the recombinant integrin α9β1 to polydom was completely abrogated in the presence of EDTA, thus confirming the specificity of the integrin binding assay. Although cellular fibronectin, which contains the EIIIA domain, is a putative ligand for integrin α9β1, the recombinant integrin α9β1 exhibited only marginal binding activity for cellular fibronectin. These findings are consistent with those of the cell adhesion assay and corroborate the notion that integrin α9β1 binds directly to polydom, which has the integrin-binding site in the C-terminal 270-kDa region.

Integrin α9β1 is known to bind to the third fibronectin type III domain of tenascin-C (TNfn3) and the osteopontin N-terminal half (OPN-Nhalf). These integrin α9β1 ligands were recombinantly expressed as GST fusion proteins and then purified. In the GST fusion proteins, the RGD cell-adhesive motifs were replaced with RAA to nullify their abilities to interact with RGD-binding integrins, such as those containing the αv subunit. Microtiter plates were coated with pol-C (10 nM), GST-TNfn3-RAA (100 nM) or GST-OPN-Nhalf-RAA (100 nM), and integrin α9β1 was added and allowed to bind thereto in the presence of 1 mM MnCl₂.

The results are shown in Fig. 8. As is clear from Fig. 8, TNfn3-RAA and OPN-Nhalf-RAA were capable of binding to integrin α9β1, but their affinities for integrin α9β1 were significantly lower than that of pol-C. The apparent dissociation constants for these integrin α9β1 ligands were unable to be determined due to incomplete saturation of binding at the highest integrin concentration, whereas the dissociation constant between integrin α9β1 and pol-C was estimated from three independent determinations to be 32.4±2.7 nM.

### (4) Integrin α9β1 Binds to CCP21

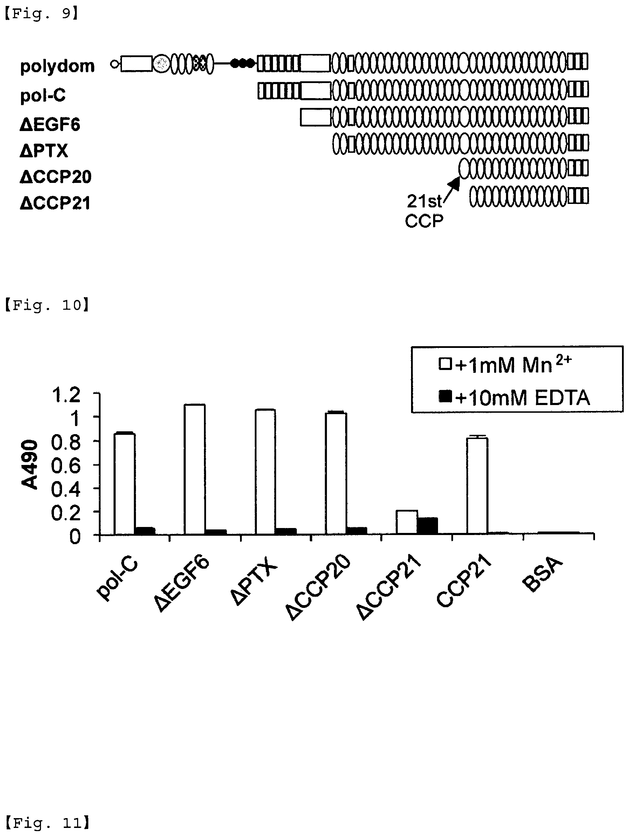

To locate the integrin α9β1-binding site within polydom, a series of five N-terminal deletion mutants of polydom (pol-C, ΔEGF6, ΔPTX, ΔCCP20 and ΔCCP21; see Fig. 9) were constructed. Their binding activities for integrin α9β1 were evaluated in the presence of 1 mM MnCl₂ or 10 mM EDTA using microtiter plates coated with these recombinant proteins (10 nM).

The results are shown in Fig. 10. As is clear from Fig. 10, although deletion of the region upstream of CCP21 did not compromise the integrin binding activity of pol-C, deletion of CCP21 resulted in a dramatic loss of the integrin binding activity, thus underscoring the critical role of CCP21 in the integrin binding activity. To explore whether CCP21 harbors the integrin α9β1-binding site, recombinant CCP21 as a GST fusion protein was produced and examined for the integrin α9β1-binding activity. As shown in Fig. 10, CCP21 alone was fully active in binding to integrin α9β1, thus confirming the critical role of CCP21 in the integrin binding activity.

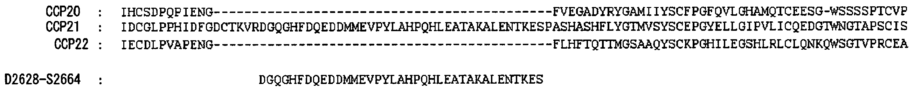

Fig. 11 shows the alignment of the amino acid sequences of the 20th, 21st and 22nd CCP domains by ClustalW. Among the 34 CCP domains within polydom, CCP21 is unique because it contains about 40 extra amino acids as compared with the other CCP domains. An assay to examine whether this extra region (hereinafter referred to as "extra segment") within CCP21 is involved in binding to integrin α9β1 was performed. Microtiter plates were coated with a GST-CCP21 fusion protein, a D2628-S2664 deletion mutant of the GST-CCP21 fusion protein (CCP21ΔD2628-S2664), D2628-S2664 alone (see Fig. 11), pol-C or GST alone, and integrin α9β1 was added and allowed to bind thereto in the presence of 1 mM MnCl₂ or 10 mM EDTA.

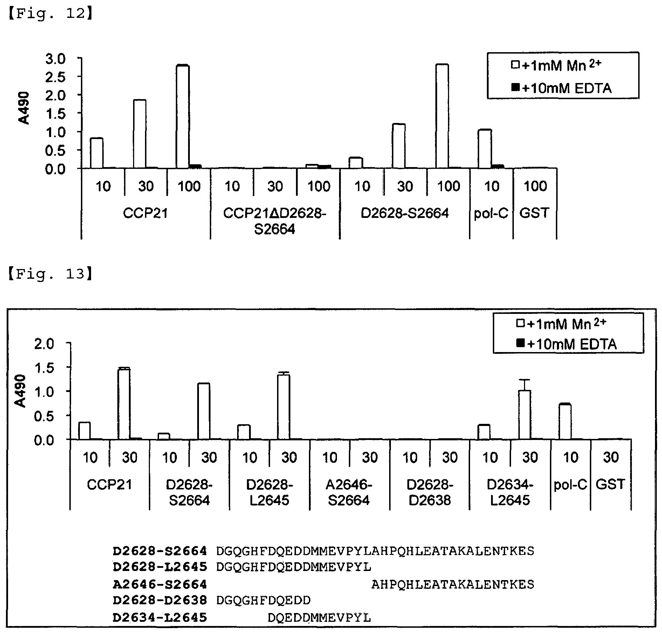

The results are shown in Fig. 12. As is clear from Fig. 12, CCP21ΔD2628-S2664 was unable to bind to integrin α9β1, whereas D2628-S2664 retained the binding activity for integrin α9β1. These findings show that the integrin α9β1-binding site of polydom is located within the extra 37-amino acid segment (D2628-S2664) of CCP21.

### (5) Integrin α9β1 Recognizes the Sequence EDDMMEVPY

To further narrow down the region responsible for binding to integrin α9β1, the extra 37-amino acid segment of CCP21 was divided into smaller segments, of which GST fusion proteins were produced. Microtiter plates were coated with eight kinds of segments including CCP21, pol-C and GST alone, and integrin α9β1 was added and allowed to bind thereto in the presence of 1 mM MnCl₂ or 10 mM EDTA.

The results are shown in Fig. 13. As is clear from Fig. 13, integrin α9β1 bound only to D2628-L2645 and D2634-L2645, which were within the N-terminal region of D2628-S2664. These findings show that the integrin α9β1-binding site can be mapped to the 12-amino acid segment (D2634-L2645) in the extra segment of CCP21.

To identify the residues involved in binding to integrin α9β1, alanine scanning mutants and N-terminal or C-terminal deletion mutants of D2634-L2645 were produced as GST fusion proteins. Microtiter plates were coated with the GST fusion proteins containing the mutated segments and integrin α9β1 was added and allowed to bind thereto.

The alanine scanning mutants are shown in Fig. 14. As is clear from Fig. 14, alanine substitution of Glu2641 (E2641A) almost completely abrogated the integrin binding activity, whereas alanine mutations at the residues from Glu2636 to Tyr2644 caused partial reductions in the integrin binding activity to variable extents. These findings indicate that integrin α9β1 recognizes the EDDMMEVPY sequence, within which Glu2641 is the critical acidic residue involved in ligand recognition of integrin α9β1.

To further corroborate the role of EDDMMEVPY as the integrin α9β1 recognition sequence, an integrin α9β1-pol-C binding inhibition assay was performed using synthetic peptides consisting of the EDDMMEVPY sequence or a part of the sequence. Integrin α9β1 (10 nM) was incubated on microtiter plates coated with pol-C (10 nM) in the presence of 1 mM MnCl₂ and various concentrations of the synthetic peptides. To prevent precipitation of the peptides, the assay was performed in the presence of 10% DMSO. Based on the amount of integrin α9β1 bound to pol-C set as 100%, the relative binding amount was calculated.

The results are shown in Fig. 15. As is clear from Fig. 15, the EDDMMEVPY peptide inhibited the binding of integrin α9β1 to pol-C in a dose-dependent manner and had an IC50 of 0.18 µM. A smaller peptide, DMMEVPY, was nearly as potent as EDDMMEVPY in inhibiting integrin α9β1 binding, and this finding is consistent with the relatively small contribution of the N-terminal Glu-Asp residues to the binding to integrin α9β1. Further, deletion of the C-terminal Tyr residue from the DMMEVPY peptide resulted in reduction in the inhibitory potency, thus supporting the involvement of Tyr2644 in the CCP21 recognition by integrin α9β1. Alanine substitution of Glu2641 in the DMMEVPY peptide resulted in a marked reduction in the inhibitory potency, and this finding is consistent with the importance of Glu2641 in the CCP21 recognition of integrin α9β1.

Further, the inhibitory activity of the EDDMMEVPY peptide was compared with those of the peptides AEIDGIEL and TYSSPEDGIHE, which represent the integrin α9β1 recognition sequences in tenascin-C and the fibronectin EIIIA domain, respectively. The results are shown in Fig. 15. As is clear from Fig. 15, the AEIDGIEL peptide was capable of inhibiting the binding of integrin α9β1 to pol-C and had an IC50 of 7.8 µM, although its potency was one order of magnitude less than that of the EDDMMEVPY peptide. The TYSSPEDGIHE peptide was barely inhibitory toward the binding of integrin α9β1 to pol-C. These findings support the conclusion that integrin α9β1 binds to polydom with an affinity that is significantly higher than those to tenascin-C and other known integrin α9β1 ligands, and that EDDMMEVPY is a preferred sequence for ligand recognition of integrin α9β1.

### (6) Polydom Partially Colocalizes with Integrin α9 in Tissues

The relatively high binding affinity of polydom for integrin α9β1 suggests that polydom functions as a physiological ligand for integrin α9β1. To address this possibility, immunofluorescence staining of mouse embryonic tissues with antibodies against polydom and integrin α9 was performed.

The results are shown in Fig. 16. Polydom was colocalized with integrin α9 at the submucosal mesenchymes in the stomach and intestine (Fig. 16, A to F). Integrin α9 was expressed in the smooth muscle layers in the stomach and intestine as well, where polydom was barely expressed. Polydom and integrin α9 were also colocalized at the sinusoids in the liver (Fig. 16, G to I), and at Bowman's capsules and the mesenchyme between renal tubules in the kidney (Fig. 16, J to L). In the lung, polydom was detected in the mesenchyme, where integrin α9 was only partially colocalized with polydom (Fig. 16, M to O). Integrin α9 was strongly expressed in the smooth muscle layer of the lung, where polydom was barely detectable. These findings demonstrate that polydom was colocalized with integrin α9 in the embryonic mesenchymes of various organs, except for the smooth muscle layers, where integrin α9 was highly expressed, and are consistent with the possibility that polydom serves as one of the physiological ligands for integrin α9.

To further corroborate the role of polydom as a physiological ligand for integrin α9β1, an in situ integrin binding assay was performed for visualization of integrin α9β1 ligands in whole tissues.

The results are shown in Fig. 17. As shown in A, E and I of Fig. 17, in the cryosections of mouse embryos incubated with integrin α9β1 in the presence of Mn²⁺, its ligands were detected in the mesenchymes and smooth muscle layers of the stomach, intestine and lung. As shown in D, H and L of Fig. 17, no signals were detected when the assay was performed in the presence of EDTA, thus confirming the specificity of the in situ integrin binding assay. As shown in B, F and J of Fig. 17, polydom was detected by immunofluorescence staining using an Alexa Fluor™ 555-conjugated anti-polydom antibody, and as shown in C, G and K in Fig. 17, the signals for polydom overlapped with those for bound integrin α9β1 in the mesenchymal regions of the stomach, intestine and lung. These findings support the conclusion that polydom serves as a physiological ligand for integrin α9β1 of mesenchymal ECM in these organs.

### Example 2: Interaction of Novel Ligand for Integrin α9β1 with Hematopoietic Stem Cells

Bone marrow cells were flushed out from the femurs and tibias of three mice of 6 to 7 weeks of age (C57BL/6J) and dissociated with a 22G needle. The cells were reacted with a biotin-labeled anti-lineage antibody for 30 minutes and subsequently with an anti-biotin antibody-conjugated magnetic beads, and lineage-positive cells were removed by automated magnetic cell sorter autoMACS (Miltenyi Biotec). After staining with PE-CD3, PE-Mac1, PE-Gr1, PE-Ter119, PE-B220, FITC-Sca1 and APC-c-Kit, a lineage (-) Sca1(+) c-Kit (high) cell fraction was sorted out using FACSAria and used for the experiments described below.

### (1) Examination of Proliferation and Differentiation of Hematopoietic Stem Cells

The above-prepared cells were plated on a 48-well plate coated with a recombinant polydom protein or fibronectin. The same type of plate without coating was used as a control. The medium used was RPMI containing 10% fetal calf serum, thrombopoietin (30 ng/ml) and flt3 ligand (100 ng/ml). For the examination of cell proliferation, the cells were plated in duplicate wells for each coating at 1000 cells/well and started to be cultured, and the cell number was counted at the start of culture, and on day 2 and day 3. The remaining cells were divided into three aliquots, plated in wells for each coating, and subjected to FACS analysis on day 3 after the start of culture. For the FACS analysis, staining with an antibody cocktail containing PE-CD3, PE-Mac1, PE-Gr1, PE-Ter119, PE-B220, FITC-Sca1 and APC-c-Kit was performed and FACSCont was used.

The results of the examination of cell proliferation are shown in Fig. 18. As is clear from Fig. 18, the cells on the fibronectin-coated wells proliferated as with the control, whereas the cells on the polydom-coated wells were remarkably inhibited from proliferating.

The results of the FACS analysis are shown in Fig. 19. In Fig. 19, the vertical axis represents the expression amount of c-Kit, and the horizontal axis represents the expression amount of Sca1. As is clear from Fig. 19, the cells on the polydom-coated well maintained the expression of Sca1, but had reduced expression of c-Kit, a growth factor receptor. Meanwhile, the cells on the fibronectin-coated well had increased expression of c-Kit.

The above findings indicate that, by interaction with polydom, hematopoietic stem cells were inhibited from proliferating and maintained in their undifferentiated state.

### (2) Examination of Effects of EDDMMEVPY Peptide on Interaction of Hematopoietic Stem cells with Polydom Protein

The above-prepared cells were divided into three groups and plated on 48-well plates at 5000 cells/well. The medium used was RPMI containing 10% fetal calf serum, thrombopoietin (30 ng/ml) and flt3 ligand (100 ng/ml). For an EDDMMEVPY peptide treatment group, 2 µl of the peptide was added to each well at the final concentration of 50 µM, and for the other two groups, 2 µl of PBS was added to each well. Then, the culture was performed at 37°C for 2 hours. Subsequently, the cells were transferred to 48-well plates coated with a recombinant polydom protein or fibronectin. The cells cultured in the presence of the peptide were transferred to the recombinant polydom protein-coated plate, and the cells in one of the other two groups were transferred to the recombinant polydom protein-coated plate, and the cells in the remaining group were transferred to the fibronectin-coated plate. The culture was continued for 3 days and the cell numbers were counted on day 2, day 3 and day 4.

The results are shown in Fig. 20. As is the case with Fig. 18, the cells on the fibronectin-coated plate (in the figure, Fibronectin) proliferated, whereas the cells on the polydom-coated plate (in the figure, Polydom) were remarkably inhibited from proliferating. Meanwhile, the cells cultured on the polydom-coated plate after treatment with the EDDMMEVPY peptide (in the figure, Polydom + peptide 50 µM) were shown to proliferate albeit more slowly than the cells on the fibronectin-coated plate. This result is probably attributed to the inhibition of the interaction of integrin α9β1 on the hematopoietic stem cells with polydom on the plate by the binding of the EDDMMEVPY peptide to integrin α9β1 on the hematopoietic stem cells.

### Example 3: Interaction of Novel Ligand for Integrin α9β1 with Cardiac Stem Cells

### (1) Separation of Cardiac Stem Cells

Tissue stem cells are known to generally have a very prolonged cell cycle and thus allow labeling compounds incorporated into DNA and the cell interior to remain stable for a long period of time without decay along with cell division. This nature can be advantageously used to identify and separate tissue stem cells by short-term labeling of cells with a nucleotide analog such as bromodeoxyuridine (BrdU) or a fusion protein of green fluorescent protein (hereinafter referred to as "GFP") and a nucleoprotein, histone, followed by selection of cells retaining the label for a long period of time (label-retaining cells). In this experiment, with the use of a tetracycline-inducible expression system in combination with the ROSA26 promoter, a fusion protein of GFP and histone H2B (H2B-GFP) was expressed in the heart of a mouse for 2 weeks starting 1 week before birth, and 6 weeks later, cardiac stem cells were separated as GFP label-retaining cells from the heart.

### (2) Analysis of Integrin α9 Expression in GFP Label-Retaining Cardiac Cells

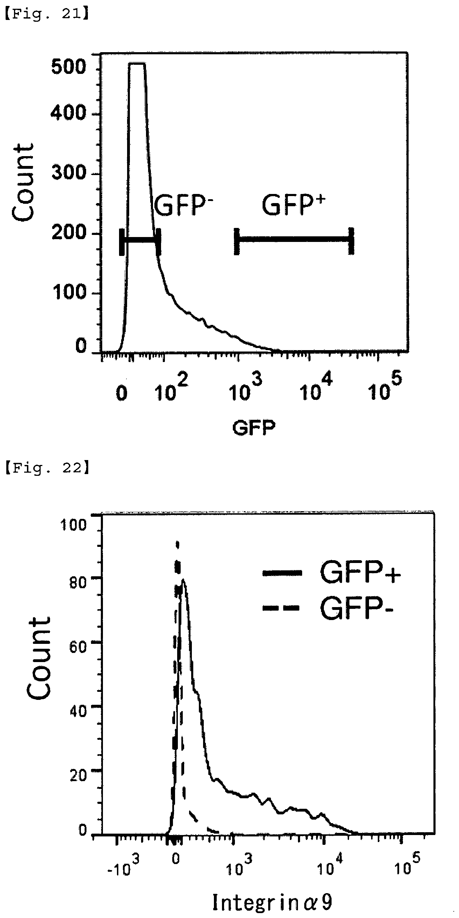

H2B-GFP was forcibly expressed in the heart of a mouse for 2 weeks around birth and then chased for 6 weeks, and GFP label-retaining cells were prepared from the heart. The cardiac cells were treated at 37°C for 30 minutes with Hank's Balanced Salt Solution (hereinafter referred to as "HBSS") containing 0.1% collagenase B (Roche) and 2.4 U/ml dispase (Life Technologies) for dissociation, and then passed through a BD Falcon cell strainer to give single cells. The resulting cells were fractionated based on the fluorescence of GFP using a cell sorter FACSAria (manufactured by BD) to separate GFP-positive cells (see Fig. 21). GFP-positive cells and GFP-non-positive cells were separately suspended at 10⁶ cells in 100 µl of HBSS containing 1 µg of an anti-mouse integrin α9 antibody (R&D) and 1% BSA, and reacted with the antibody on ice for 30 minutes. After washing with 500 µl of HBSS containing 1% BSA, the cells were suspended in 100 µl of HBSS containing an allophycocyanin-labeled anti-goat IgG antibody (R&D, 20-fold dilution) and 1% BSA, and reacted with the antibody on ice for 20 minutes to fluorescently label integrin α9 on the cells. After washing with 500 µl of HBSS containing 1% BSA, the cells were resuspended in 500 µl of HBSS containing 1% BSA and analyzed for the expression of integrin α9 using a cell sorter FACSAria. As shown in Fig. 22, the cells highly expressing integrin α9 were concentrated in the GFP-positive cell population.

### (3) Immunohistochemical Staining of Integrin α9

To confirm the high expression of integrin α9 on cardiac stem cells, GFP label-retaining cells and integrin α9 expressing cells were analyzed by double immunofluorescence staining. A mouse heart containing H2B-GFP-labeled cardiac stem cells was cryosectioned at a thickness of 8 µm and the resulting sections were fixed with 3.7% formalin for 10 minutes. After blocking with PBS containing 3% BSA at room temperature for 30 minutes, the sections were reacted with an anti-mouse integrin α9 antibody (5 µg/ml) at 4°C overnight and the bound antibody was then fluorescently labeled with an Alexa Fluor™ 546-labeled anti-goat IgG antibody. The fluorescently-labeled integrin α9 was observed with a confocal microscope. GFP label-retaining cells were identified as cardiac stem cells.

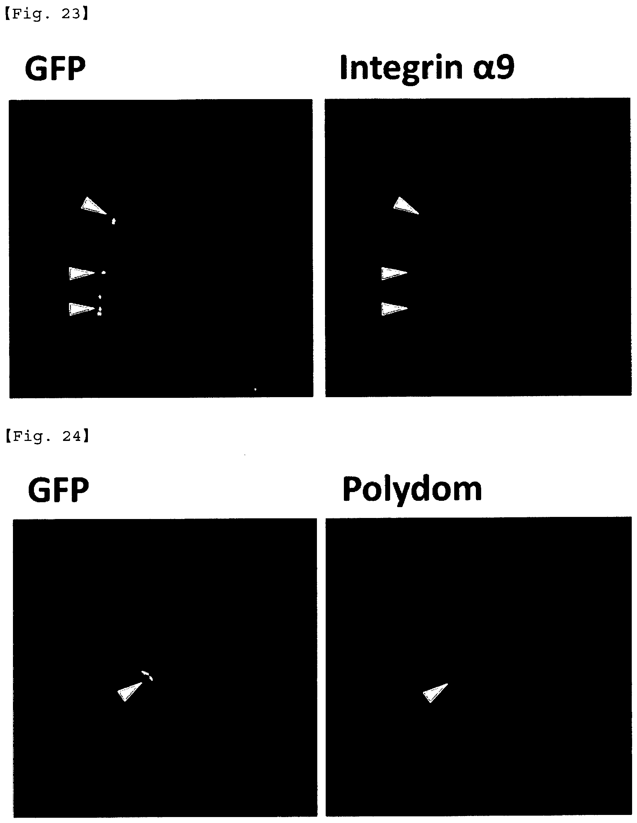

The results are shown in Fig. 23. The left panel is a GFP-stained image, the right panel is an integrin α9-stained image, and the arrowheads in the figure represent the locations of GFP label-retaining cells. Fig. 23 shows that the GFP label-retaining cells were mainly localized in the epicardium and colocalized with the integrin α9 expressing cells.

### (4) Immunohistochemical Staining of Polydom

To explore whether polydom, an integrin α9 ligand, is expressed around cardiac stem cells, double immunofluorescence staining was performed in the same manner as in the above (3). The concentration of the anti-polydom antibody used was 2 µg/ml, and the bound anti-polydom antibody was visualized with an Alexa Fluor™ 546-labeled anti-rabbit IgG antibody.

The results are shown in Fig. 24. The left panel is a GFP-stained image, the right panel is a polydom-stained image, and the arrowheads in the figure represent the locations of GFP label-retaining cells. As shown in Fig. 24, the expression of polydom was localized around the GFP label-retaining cells in the epicardium. These findings support the possibility that polydom functions as a ligand for cardiac stem cell-expressed integrin α9β1.

### (5) Cell Adhesion Assay Using GFP Label-Retaining Cells

To explore whether polydom serves as a scaffold for integrin α9β1-expressing cardiac stem cells, cardiac stem cells were separated as GFP label-retaining cells and subjected to a cell adhesion assay. A 96-well immuno plate (Nunc Maxisorp) was coated with 50 µl/well of 100 nM polydom or fibronectin at 4°C overnight. GFP label-retaining cells were collected by FACSAria and suspended at 1×10⁶ cells/ml in serum-free Iscove's Modified Dulbecco Medium (hereinafter referred to as "IMDM"). The cell suspension was plated at 100 µl/well and cultured in a 5% CO₂ incubator at 37°C for 12 hours. After three washes with PBS, the cells were fixed with 100 µl/well of 3.7% formalin for 15 minutes. Then, the cells were incubated with 0.5% toluidine blue (150 µl/well) for 10 minutes and washed with MilliQ water. After drying, the number of attached cells was counted under a microscope.

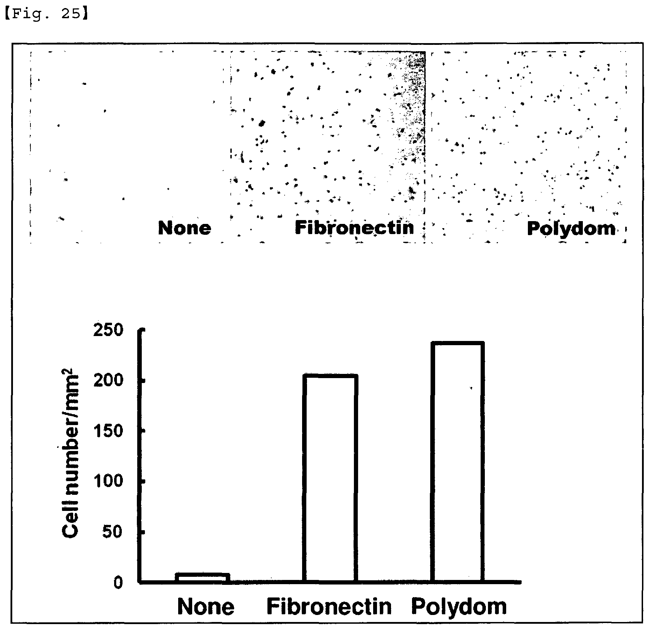

The results are shown in Fig. 25. The upper panel is a set of microscopic images showing attached cells, and the lower panel is a graph showing the cell adhesion activity (the number of attached cells per square millimeter). As is clear from Fig. 25, the cells hardly adhered onto the uncoated plate, whereas a large number of the cells adhered onto the fibronectin-coated plate and the polydom-coated plate. It was found that polydom is equivalent or superior to fibronectin in cell adhesion activity for cardiac stem cells.

### (6) Measurement of Colony Forming Ability Using GFP Label-Retaining Cells

To explore whether cardiac stem cells proliferate on a polydom-coated plate, colony formation was measured as an indicator of proliferation. A Falcon 6-well plate was coated with 800 µl/well of 100 nM polydom or fibronectin at 4°C overnight. GFP label-retaining cells were collected by FACSAria and suspended in IMDM containing 10% fetal calf serum. The cells were plated at 1×10⁴ cells/well and cultured in a 5% CO₂ incubator at 37°C. The culture was continued for 2 weeks with medium replacement every three days. After washing with HBSS containing 1 mM CaCl₂, Giemsa stain (Merck) was added at 1 ml/well, and 30 minutes later, washing with tap water was performed. After drying, the number of colonies was counted under a microscope.

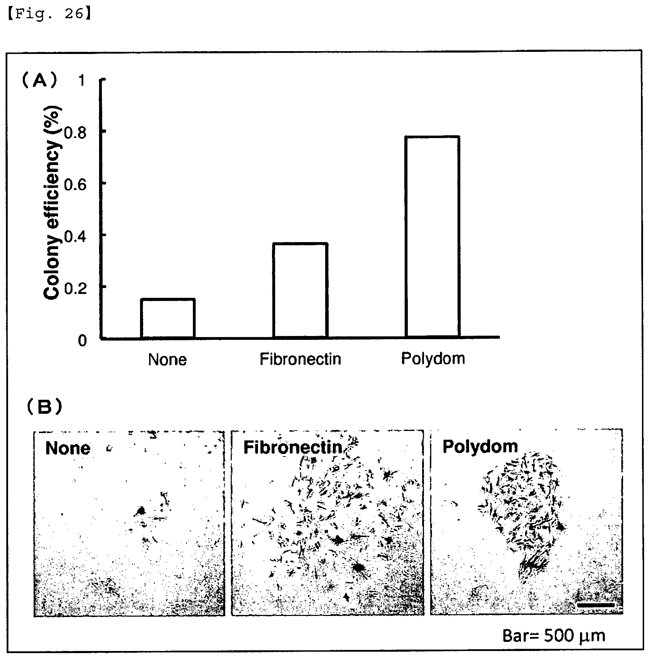

The results are shown in Fig. 26. (A) is a graph showing the colony forming activity and (B) is a set of images showing typical colony morphologies. The colony forming ability was expressed as the percentage of the colony count relative to the number of the cells plated. As is clear from Fig. 26, the colony forming activity of the cells on the uncoated plate was 0.2% or less, whereas the colony forming activity of the cells on the polydom-coated plate was about 0.8%. The colony forming activity of the cells on the fibronectin-coated plate was about 0.4%. That is, it was shown that cardiac stem cells on polydom are more capable of forming colonies than those on fibronectin.

The present invention is not limited to particular embodiments and examples described above, and various modifications can be made within the scope of the appended claims. Other embodiments provided by suitably combining technical means disclosed in separate embodiments of the present invention are also anticipated.

## Claims

1. A ligand for integrin α9β1, consisting of an active fragment of SVEP1, the active fragment having the following amino acid sequence:
(A) EDDMMEVPY (SEQ ID NO: 1) or
(B) an amino acid sequence the same as the amino acid sequence represented by SEQ ID NO: 1 except for having deletion, substitution or addition of 1 to 3 amino acids at residues other than E at residue 6.

2. The ligand for integrin α9β1 according to claim 1, wherein the active fragment has 2500 amino acid residues or less.

3. The ligand for integrin α9β1 according to claim 2, wherein the active fragment consists of the following peptide:
(a) a peptide consisting of EDDMMEVPY (SEQ ID NO: 1),
(b) a peptide the same as peptide (a) except that E at residue 1; E at residue 1 and D at residue 2; Y at residue 9; E at residue 1 and Y at residue 9; or E at residue 1, D at residue 2 and Y at residue 9 are deleted or substituted by an amino acid(s); or
(c) a peptide the same as peptide (a) or (b) except that M at residue 4, V at residue 7, or P at residue 8 is substituted by an amino acid.

4. A polynucleotide encoding a peptide constituting the ligand for integrin α9β1 according to any one of claims 1 to 3.

5. An expression vector containing the polynucleotide according to claim 4.

6. An antibody against the ligand for integrin α9β1 according to any one of claims 1 to 3.

7. A method for screening for substances capable of inhibiting interaction of SVEP1 with integrin α9β1, the method using the ligand for integrin α9β1 according to any one of claims 1 to 3.

8. A method for culturing integrin α9β1-expressing cells, the method using the ligand for integrin α9β1 according to any one of claims 1 to 3 as a substrate.

9. The method for culturing integrin α9β1-expressing cells according to claim 8, wherein the method inhibits tissue stem cells from proliferating with maintenance of their undifferentiated state.

10. The method for culturing integrin α9β1-expressing cells according to claim 8, wherein the method selectively separates integrin α9β1-expressing cells capable of binding to the substrate.

11. The method for culturing integrin α9β1-expressing cells according to claim 8, wherein the method comprises a step of bringing tissue stem cells in a resting phase or an antiproliferative state into contact with the substrate and a step of inducing the stem cells into a proliferative state.

12. A drug delivery system for delivering a drug to integrin α9β1-expressing cells, the system comprising the ligand for integrin α9β1 according to any one of claims 1 to 3, and the drug.

## Patentansprüche

1. Ligand für Integrin α9β1, bestehend aus einem aktiven Fragment von SVEP1, wobei das aktive Fragment die folgende Aminosäuresequenz aufweist:
(A) EDDMMEVPY (SEQ ID NR.: 1) oder
(B) eine Aminosäuresequenz, die dieselbe wie die durch SEQ ID NR.: 1 dargestellte Aminosäuresequenz ist, außer dass sie eine Deletion, Substitution oder Addition von 1 bis 3 Aminosäuren an Resten, die an Rest 6 nicht E sind, aufweist.

2. Ligand für Integrin α9β1 nach Anspruch 1, wobei das aktive Fragment 2500 Aminosäurereste oder weniger aufweist.

3. Ligand für Integrin α9β1 nach Anspruch 2, wobei das aktive Fragment aus dem folgenden Peptid besteht:
(a) einem Peptid, bestehend aus EDDMMEVPY (SEQ ID NR.: 1),
(b) einem Peptid, das dasselbe wie Peptid (a) ist, außer dass E an Rest 1; E an Rest 1 und D an Rest 2; Y an Rest 9; E an Rest 1 und Y an Rest 9; oder E an Rest 1, D an Rest 2 und Y an Rest 9 durch (eine) Aminosäure(n) deletiert oder substituiert sind; oder
(c) einem Peptid, das dasselbe wie Peptid (a) oder (b) ist, außer dass M an Rest 4, V an Rest 7 oder P an Rest 8 durch eine Aminosäure substituiert ist.

4. Polynucleotid, das ein Peptid kodiert, das den Liganden für Integrin α9β1 nach einem der Ansprüche 1 bis 3 bildet.

5. Expressionsvektor, enthaltend das Polynucleotid nach Anspruch 4.

6. Antikörper gegen den Liganden für Integrin α9β1 nach einem der Ansprüche 1 bis 3.

7. Verfahren zum Screenen nach Substanzen, die die Wechselwirkung von SVEP1 mit Integrin α9β1 inhibieren können, wobei das Verfahren den Liganden für Integrin α9β1 nach einem der Ansprüche 1 bis 3 nutzt.

8. Verfahren zum Kultivieren von Integrin α9β1-exprimierenden Zellen, wobei das Verfahren den Liganden für Integrin α9β1 nach einem der Ansprüche 1 bis 3 als ein Substrat nutzt.

9. Verfahren zum Kultivieren von Integrin α9β1-exprimierenden Zellen nach Anspruch 8, wobei das Verfahren verhindert, dass Gewebestammzellen proliferieren, wobei ihr undifferenzierter Zustand aufrechterhalten wird.

10. Verfahren zum Kultivieren von Integrin α9β1-exprimierenden Zellen nach Anspruch 8, wobei das Verfahren selektiv Integrin α9β1-exprimierende Zellen, die an das Substrat binden können, trennt.

11. Verfahren zum Kultivieren von Integrin α9β1-exprimierenden Zellen nach Anspruch 8, wobei das Verfahren einen Schritt des Inkontaktbringens von Gewebestammzellen in einer Ruhephase oder einem antiproliferativen Zustand mit dem Substrat und einen Schritt des Induzierens eines proliferativen Zustands der Stammzellen umfasst.

12. Arzneimittelabgabesystem zur Abgabe eines Arzneimittels an Integrin a9β1-exprimierende Zellen, wobei das System den Liganden für Integrin α9β1 nach einem der Ansprüche 1 bis 3 und das Arzneimittel umfasst.

## Revendications

1. Ligand pour l'intégrine α9β1, consistant en un fragment actif de SVEP1, le fragment actif ayant la séquence d'acides aminés suivante :
(A) EDDMMEVPY (SEQ ID N° 1) ou
(B) une séquence d'acides aminés identique à la séquence d'acides aminés représentée par SEQ ID N° 1 à l'exception d'une délétion, d'une substitution ou d'une addition de 1 à 3 acides aminés au niveau de résidus autres que E au résidu 6.

2. Ligand pour l'intégrine α9β1 selon la revendication 1, dans lequel le fragment actif comporte 2500 résidus d'acide aminé ou moins.

3. Ligand pour l'intégrine α9β1 selon la revendication 2, dans lequel le fragment actif consiste en le peptide suivant :
(a) un peptide consistant en EDDMMEVPY (SEQ ID N° 1),
(b) un peptide identique au peptide (a) à l'exception que E au niveau du résidu 1 ; E au niveau du résidu 1 et D au niveau du résidu 2 ; Y au niveau du résidu 9 ; E au niveau du résidu 1 et Y au niveau du résidu 9 ; ou E au niveau du résidu 1, D au niveau du résidu 2 et Y au niveau du résidu 9 sont délétés ou substitués par un ou des acides aminés ; ou
(c) un peptide identique au peptide (a) ou (b) à l'exception que M au niveau du résidu 4, V au niveau du résidu 7, ou P au niveau du résidu 8 est substitué par un acide aminé.

4. Polynucléotide codant un peptide constituant le ligand pour l'intégrine α9β1 selon l'une quelconque des revendications 1 à 3.

5. Vecteur d'expression contenant le polynucléotide selon la revendication 4.

6. Anticorps contre le ligand pour l'intégrine α9β1 selon l'une quelconque des revendications 1 à 3.

7. Procédé de criblage de substances capable d'inhiber l'interaction de SVEP1 avec l'intégrine α9β1, le procédé utilisant le ligand pour l'intégrine α9β1 selon l'une quelconque des revendications 1 à 3.

8. Procédé de culture de cellules exprimant l'intégrine α9β1, le procédé utilisant le ligand pour l'intégrine α9β1 selon l'une quelconque des revendications 1 à 3 comme substrat.

9. Procédé de culture de cellules exprimant l'intégrine α9β1 selon la revendication 8, dans lequel le procédé inhibe la prolifération de cellules souches tissulaires avec maintien de leur état indifférencié.

10. Procédé de culture de cellules exprimant l'intégrine α9β1 selon la revendication 8, dans lequel le procédé sépare sélectivement des cellules exprimant α9β1 d'intégrine capables de se lier au substrat.

11. Procédé de culture de cellules exprimant l'intégrine α9β1 selon la revendication 8, dans lequel le procédé comprend une étape consistant à amener des cellules souches tissulaires dans une phase de repos ou un état antiprolifératif en contact avec le substrat et une étape d'induction des cellules souches dans un état prolifératif.

12. Système de délivrance de médicament consistant à délivrer un médicament à des cellules exprimant l'intégrine α9β1, le système comprenant le ligand pour l'intégrine α9β1 selon l'une quelconque des revendications 1 à 3, et le médicament.
